# EUROPEAN PATENT SPECIFICATION

(11) **EP 3 883 600 B1**
(45) Date of publication and mention of the grant of the patent: **12.03.2025**
(21) Application number: 19812730.0
(22) Date of filing: 22.11.2019
(51) Int. Cl.: A61K 39/00, A61K 39/245, A61K 39/39, C07K 14/33, C07K 16/12, C07K 16/28

(54) **BI-SPECIFIC CONJUGATES**
BISPEZIFISCHE KONJUGATE
CONJUGUÉS BI-SPÉCIFIQUES

(30) Priority: 23.11.2018 GB 201819124; 15.02.2019 GB 201902159
(43) Date of publication of application: 29.09.2021
(73) Proprietor: Strike Pharma AB, 756 51 Uppsala (SE)
(72) Inventor: MANGSBO, Sara, 756 52 Uppsala (SE); PERSSON LOTSHOLM, Helena, 175 64 Järfälla (SE)
(86) International application number: PCT/EP2019/082322
(87) International publication number: WO 2020/104690

(56) References cited:
- EP-A1- 2 335 730
- WO-A1-2011/115483
- WO-A1-2012/123755
- WO-A1-2018/011421
- WO-A1-2018/017020
- WO-A2-2017/184619
- PEREBOEV A V ET AL: "Coxsackievirus-adenovirus receptor genetically fused to anti-human CD40 scFv enhances adenoviral transduction of dendritic cells", GENE THERAPY, NATURE PUBLISHING GROUP, LONDON, GB, vol. 9, no. 17, 1 September 2002 (2002-09-01), pages 1189 - 1193, XP002303755, ISSN: 0969-7128, DOI: 10.1038/SJ.GT.3301767
- TILLMAN B W ET AL: "Adenoviral vectors targeted to CD40 enhance the efficacy of dendritic cell-based vaccination against human papillomavirus 16-induced tumor cells in a murine model", CANCER RESEARCH, AMERICAN ASSOCIATION FOR CANCER RESEARCH, US, vol. 60, no. 19, 1 October 2000 (2000-10-01), pages 5456 - 5463, XP002973706, ISSN: 0008-5472
- OLIVER MANZKE ET AL: "CD3? ANTI-NITROPHENYL BISPECIFIC DIABODIES: UNIVERSAL IMMUNOTHERAPEUTIC TOOLS FOR RETARGETING T CELLS TO TUMORS", INTERNATIONAL JOURNAL OF CANCER, vol. 82, no. 5, 27 August 1999 (1999-08-27), pages 700 - 708, XP055671014

## Description

### Field of the Invention

The present invention relates to novel conjugates comprising a first binding molecule capable of binding specifically to CD40 conjugated, via a covalent linkage, including particularly a peptide bond, to a second binding molecule capable of binding to a tag moiety to form a conjugate-tag complex. The tag moiety may be provided as part of a tag construct further comprising an antigen. The invention further relates to a complex of the conjugate with such a tag-antigen construct, and the use of such a complex in therapy, particularly for the treatment of cancer.

### Background to the Invention

Monoclonal antibodies (mAbs) which modulate immune responses are proving highly effective in cancer treatment, with increasing evidence that such responses can be harnessed to provide durable eradication of tumours. Various antibodies against different targets have been developed, e.g. targeting the immune checkpoints CTLA-4 and PD-1, which support the view that T-cell immunity can provide an effective treatment for cancer. Promising clinical data have also been obtained with immunostimulatory mAbs that bind agonistically to the co-stimulatory receptor CD40 on antigen-presenting cells (APCs).

CD40 is a member of the TNF receptor superfamily (it is alternatively known as TNF receptor superfamily member 5; TNFRSF5) and is expressed on antigen-presenting cells (APC) such as B-cells, dendritic cells (DC), macrophages and monocytes, as well as epithelial and endothelial cells and certain tumour cells. When activated by its ligand (CD40 ligand; CD40L, also known as CD154), which is mainly expressed on mature T-cells, CD40 activates APC and induces both innate and adaptive immune responses. Agonistic CD40 agents, such as anti-CD40 antibodies, can be used to induce the immune system to prevent proliferation of and/or kill cancer cells and thus provide an effective therapeutic treatment for cancer. In particular, APC activated by an agonistic CD40 agent may stimulate T-cells, including effector T-cells, particularly CD8+ cytotoxic T-cells, leading to T-cell mediated destruction of cancer cells (e.g. tumours). There may also be direct cancer cell killing if the APC is a macrophage, and direct anti-tumour mechanisms may be observed for CD40-positive tumours, where the binding of anti-CD40 antibody to the tumour cell may induce apoptosis.

Various anti-CD40 antibodies are in pre-clinical or clinical development, including antibodies ADC-1013, CP-870,893, Chi Lob 7/4, SEA-CD40 and APX005M. Antibody ADC-1013 is described in WO 2016/023960, and various other anti-CD40 antibodies are proposed for anti-cancer use in WO 2015/091853 and US 2017/0342159. ChiLob 7/4 is described in US 2009/0074711, SEA-CD40 is described in US 2017/0137528, CP-870,893 is described in US 2017/0342159 and APX005M (sometimes alternatively referred to simply as APX005) is described in WO 2014/070934.

Effective stimulation (or priming) of T-cells requires not only a CD40 stimulus, but also the presentation of antigen (in the context of MHC, for binding to the T-cell receptor (TCR)). Thus, it is advantageous for the APC to cross-present antigen to the T-cell in the context of T-cell stimulation, in other words to take up, process and present antigen (of extracellular origin) to the T-cells. However, antigenic material may not always be present (for example if a tumour has been resected), and CD40 agonists may have poor efficacy in leading to effective T-cell stimulation in such a situation. CD40 stimulation may also be insufficient for T-cell activation (for example if there is a dose-limiting toxicity of the CD40 agonist as an infusion product). Accordingly, it would be of benefit to provide both agonistic activation of CD40 on the APC and, at the same time, delivery of antigen to the APC. The document WO2017/184619 describes a molecular conjugate comprising an agonist anti-CD40 antibody linked to an antigen. The present invention also addresses this need.

### Summary of the Invention

The invention is as defined in the claims

In particular, bi-specific conjugates are provided which are specific both for CD40 and for, ultimately, an antigenic component, and which may be used in the activation of antigen-presenting cells, particularly DC, and particularly in the context of therapy. Depending on the antigen, the therapy may be the prophylactic or therapeutic treatment of cancer, or the prophylactic or therapeutic treatment of an infection (activated T-cells may target and kill infected cells).

The bi-specific conjugate comprises two separate specific binding moieties covalently coupled together. The first binding moiety is specific for CD40. However, rather than providing a second specific binding moiety which is directly specific for an antigen, the conjugate of the invention comprises a second binding moiety which is instead specific for a tag. The tag may be provided as part of a construct in which the tag is covalently coupled to an antigen. Hence, by binding to the tag, the second binding moiety (and hence the conjugate) may be bound indirectly to the antigen, providing a flexible approach by which the antigen can be varied, since there is no chemical linkage between the antigen and the antibody. In other words, a complex may be formed between the conjugate and the tag construct, which complex provides both a CD40 agonist (i.e. for activation of an APC) and an antigen (i.e. for presentation by the APC). This advantageously allows for flexibility in preparing conjugates and complexes for use in personalised medicine. Rather than preparing conjugates comprising an antigen directly fused to a CD40 agonist (e.g. CD40 binder), or an antigen-specific binder fused to the agonist, which would require the laborious synthesis and production of a separate conjugate for each patient, the present proposal allows for a universal agent to be produced, i.e. the bi-specific (CD40- and tag-specific) conjugate, which can be tailored for individual, personalised use by binding to different tag constructs containing different antigens but the same tag, according to the need of a particular, individual, patient. In this way, only separate tag constructs need to be prepared, providing a benefit in the ease and costs of preparing patient-specific therapeutic agents. It is further believed that the non-covalent binding of antigen to the CD40 binder may be advantageous for the efficacy of the complex, as compared to antigen fused directly and covalently to the CD40 binder, or as compared to providing the CD40 binder and antigen separately.

Accordingly, in a first aspect the present invention provides a conjugate comprising:
i) a first specific binding molecule which binds CD40, wherein said first specific binding molecule is an agonist of CD40, and wherein said first specific binding molecule induces CD40 internalisation upon binding to CD40; and
ii) a second specific binding molecule which binds a tag moiety, wherein said tag moiety is a peptide and not a cancer antigen, wherein said peptide consists of the amino acid sequence set forth in any one of SEQ ID NOs: 6 or 10-14, or a fragment of any one of SEQ ID NOs: 6 or 10-14 of at least 5 amino acids;
wherein said first specific binding molecule and second specific binding molecule are antigen-binding proteins comprising an antigen-binding domain of an antibody and are covalently linked.

In particular the first specific binding molecule may bind to CD40 when localised on the surface of a cell, more particularly an antigen-presenting cell. The CD40 may be human CD40.

As noted above, the specific binding molecules are antigen-binding proteins, that is antibody-based molecules, as described further below. In a particular embodiment the first specific binding molecule is an antibody. In another specific embodiment the second specific binding molecule is a single chain antibody construct.

The conjugate comprises at least one first specific binding molecule and at least one second specific binding molecule. Accordingly, it may comprise two or more first and/or second specific binding molecules. In an embodiment the conjugate may comprise one first specific binding molecule and two or more, e.g. 2-4, second specific binding molecules. In such an embodiment the first specific binding molecule may be bivalent, or it may have a valency of 2 or more. Alternatively, or additionally, in such an embodiment the second specific binding molecule may be monovalent.

The tag moiety to which the conjugate binds may take the form of a tag construct comprising a tag moiety covalently linked to an antigen. The tag moiety is not a cancer antigen and is a peptide which consists of the amino acid sequence set forth in any one of SEQ ID NOs: 6 or 10-14, or a fragment of any one of SEQ ID NOs: 6 or 10-14 of at least 5 amino acids.

In one embodiment the tag moiety may be or may comprise a known natural B-cell epitope. In another embodiment the tag moiety comprises no known B-cell epitopes. In an embodiment the tag moiety comprises no known human B-cell epitopes. In another embodiment the tag moiety comprises no natural B-cell epitopes, and in particular no epitopes which are natural B-cell epitopes in a human.

The tag moiety is a peptide. Conveniently, the tag moiety may be a synthetically produced peptide. In another embodiment the antigen is an antigenic peptide. Thus, the tag construct may be a polypeptide comprising a first sequence (or domain) which is a tag sequence (or domain) and a second sequence (or domain) which is an antigen sequence (or domain). In other words, the tag construct may be a fusion polypeptide (alternatively referred to as a fusion peptide or a fusion protein) comprising a tag peptide and an antigen peptide.

Notably, in the case that the tag construct comprises a peptide tag moiety and a peptide antigen, the tag moiety and the peptide antigen are derived from different sources. For instance, the tag moiety may be a peptide with an amino acid sequence from a first protein, and the antigen a peptide with an amino acid sequence from a second protein. Preferably the sequence of the tag moiety is derived from a different species to the sequence of the antigen. Thus the tag construct is not a sequence found in nature, one part of which functions as the tag moiety and the other of which functions as the antigen. Rather, where the tag construct comprises a peptide tag moiety and a peptide antigen, the tag construct has an artificial sequence.

The conjugate comprises at least one first specific binding molecule which binds CD40. By this is meant that the first specific binding molecule is capable of binding CD40, more particularly specifically binding to CD40. Similarly, the at least one second specific binding molecule binds a tag moiety, by which is meant that the second specific binding molecule is capable of binding a tag moiety, more particularly, specifically binding to a tag moiety.

In a further aspect the disclosure provides a complex comprising (or between) a conjugate as defined herein and a tag construct, said tag construct comprising a tag moiety covalently attached to an antigen, wherein the tag moiety of said tag construct is non-covalently bound to the second specific binding molecule of the conjugate.

Depending on the second specific binding molecule and/or the number of second specific binding molecules in the conjugate, the complex may comprise one or more, e.g. two or more tag constructs.

The complex thus comprises a specific binding molecule directed against CD40, and an antigen. The antigen may be a cancer antigen or an antigen of a pathogen. The antigen may be recognised by a T-cell (more particularly the antigen, when presented in an MHC molecule, may be recognised by the TCR of a T-cell), and the T-cell may be activated, or stimulated, by the complex. The T-cell may particularly be an effector T-cell and more particularly a CD4+ or a CD8+ T-cell.

Also provided is a pharmaceutical composition comprising:
i) a conjugate as defined herein; or
iii) a complex as defined herein, together with at least one pharmaceutically-acceptable carrier or excipient.

Further provided is a complex as defined herein for use in therapy. Similarly, a product is provided, the product comprising a conjugate as defined herein and a tag construct as a combined preparation for simultaneous or sequential use in therapy, wherein the tag construct comprises
i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
ii) an antigen, being a cancer antigen or an antigen derived from a pathogen;
wherein said antigen is a polypeptide and said tag moiety is covalently linked to said antigen. It will be understood in this regard that the term "product" refers broadly to any combination of the conjugate and tag construct, which may be provided together for therapeutic use, for example for administration in separate formulations, for administration at the same time, or at different times, via the same or different administration routes, or together in the same formulation. Accordingly, in this aspect of the invention reference may alternatively be made to a pharmaceutical combination comprising the conjugate and tag construct, or to the conjugate for use in combination with the tag construct in therapy, or vice versa.

Particularly, the complex and product are for use in the treatment or prevention of cancer, or for use in the treatment or prevention of an infection (i.e. an infection with a pathogen). It will be understood in this respect that the cancer may be cancer the cells of which express the antigen, or the infection may be with a pathogen which expresses the antigen.
The references to the methods of preventive or therapeutic treatment by therapy described below are to be interpreted as references to compounds, pharmaceutical compositions, and medicaments of the present invention for use in those methods.

In a further aspect also provided is a method of treating or preventing cancer, which method comprises administering to a subject in need thereof:
i) a complex as defined herein, or a pharmaceutical composition comprising said complex; or
ii) a conjugate as defined herein (or a pharmaceutical composition comprising said conjugate) and a tag construct (or a pharmaceutical composition comprising said tag construct), wherein the tag construct comprises
   i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
   ii) an antigen, being a cancer antigen or an antigen derived from a pathogen;
wherein said antigen is a polypeptide and said tag moiety is covalently linked to said antigen.

Also provided is a method of treating or preventing an infection in a subject, which method comprises administering to said subject:
i) a complex as defined herein, or a pharmaceutical composition comprising said complex; or
ii) a conjugate as defined herein (or a pharmaceutical composition comprising said conjugate) and a tag construct (or a pharmaceutical composition comprising said tag construct), wherein the tag construct comprises
   i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
   ii) an antigen, being a cancer antigen or an antigen derived from a pathogen;
wherein said antigen is a polypeptide and said tag moiety is covalently linked to said antigen.

Particularly, an effective amount of the complex, or conjugate and tag (or pharmaceutical composition) is administered.

The disclosure further provides use of a complex as defined herein in the manufacture of a medicament for use in treating or preventing cancer, or for use in treating or preventing an infection in a subject. Similarly, the disclosure provides use of a conjugate as defined herein and a tag construct as defined herein in the manufacture of a medicament for use in treating or preventing cancer, or for use in treating or preventing an infection in a subject.

In a still further aspect the present invention provides a kit comprising a conjugate as herein defined and a tag construct comprising:
i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
ii) an antigen, being a cancer antigen or an antigen derived from a pathogen;
wherein said antigen is a polypeptide and said tag moiety is covalently linked to said antigen,

Such a kit may be provided for therapeutic use. Thus, the kit may be used to prepare a complex for use in therapy as defined and described herein. The kit may comprise one or more tag constructs, particularly different species of tag construct. Thus, two or more different tag constructs may be provided, each containing a different antigen with the same tag bound to each individual antigen. In an embodiment, the kit may comprise a library of tag constructs.

In another aspect the invention provides an *in vitro* or *ex vivo* method of activating a T-cell expressing a TCR which recognises an antigen, said method comprising contacting an antigen presenting cell (APC) with:
a) a conjugate as defined herein and a tag construct as defined herein, wherein said tag construct comprises the antigen recognised by said TCR and wherein the tag construct comprises
   i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
   ii) an antigen, being a cancer antigen or an antigen derived from a pathogen; wherein said antigen is a polypeptide and said tag moiety is covalently linked to said antigen
      ; or
b) a complex as defined herein wherein the tag construct of said complex comprises the antigen recognised by said TCR.

In particular, such a method comprises contacting said APC with said T-cell.

### Brief Description of the Sequence Listing

| **SEQ ID NO:** | **Description** | **Type** |
|---|---|---|
| 1 | OVA₂₅₇₋₂₆₄ | Protein |
| 2 | gp100₂₅₋₃₃ | Protein |
| 3 | UU-05 | Protein |
| 4 | UU-10 | Protein |
| 5 | UU-30 | Protein |
| 6 | MTTE | Protein |
| 7 | UU-05 OVA fragment | Protein |
| 8 | UU-05 C-terminal fragment | Protein |
| 9 | UU-30 gp100 fragment | Protein |
| 10 | SM083P001 | Protein |
| 11 | SM083P002 | Protein |
| 12 | SM083P003 | Protein |
| 13 | SM083P004 | Protein |
| 14 | SM083P005 | Protein |
| 15 | IgG2 C127S | Protein |
| 16 | TTx | Protein |
| 17-22 | ABS-1150 CDRs | Protein |
| 23 | ABS-1150 Heavy Chain Variable Domain | Protein |
| 24 | ABS-1150 Light Chain Variable Domain | Protein |
| 25 | ABS-1150 Heavy Chain Variable Domain | DNA |
| 26 | ABS-1150 Light Chain Variable Domain | DNA |
| 27 | CP-870,893 Heavy Chain Variable Domain | Protein |
| 28 | CP-870,893 Light Chain Variable Domain | Protein |
| 29 | CP-870,893 Heavy Chain Variable Domain | DNA |
| 30 | CP-870,893 Light Chain Variable Domain | DNA |
| 31-36 | ADC-1013 CDRs (IMGT) | Protein |
| 37 | ADC-1013 Heavy Chain Variable Domain | Protein |
| 38 | ADC-1013 Light Chain Variable Domain | Protein |
| 39 | ADC-1013 Heavy Chain Variable Domain | DNA |
| 40 | ADC-1013 Light Chain Variable Domain | DNA |
| 41 | Human IgG1 Constant Domain | Protein |
| 42 | Human IgG2 Constant Domain | Protein |
| 43 | Human IgG3 Constant Domain | Protein |
| 44 | Human IgG4 Constant Domain | Protein |
| 45 | Human κ Constant Domain | Protein |
| 46 | Human λ Constant Domain | Protein |
| 47 | 14GIIICII-b scFv | Protein |
| 48 | IBIIICI-b scFv | Protein |
| 49 | GGGGS₂ Linker | Protein |
| 50 | hCD40 | Protein |
| 51 | GGGGS linker module | Protein |
| 52 | ChiLob 7/4 Heavy Chain Variable Domain | Protein |
| 53 | ChiLob 7/4 Light Chain Variable Domain | Protein |
| 54 | SEA-CD40 Heavy Chain Variable Domain | Protein |
| 55 | SEA-CD40 Light Chain Variable Domain | Protein |
| 56 | APX-005M Heavy Chain Variable Domain | Protein |
| 57 | APX-005M Light Chain Variable Domain | Protein |
| 58-63 | APX-005M CDRs (IMGT) | Protein |
| 64-69 | CP-870,893 CDRs | Protein |
| 70 | FITC-8 scFv | Protein |
| 71 | UU-44 | Protein |
| 72 | UU-44 CMV Epitope | Protein |
| 73-74 | scFv Epitope Mapping Regions | Protein |
| 75-120 | Peptides used in scFv Epitope Mapping | Protein |
| 121 | SM083P003 Epitope | Protein |
| 122-125 | SM083P004 Epitopes | Protein |
| 126-131 | 14GIIICII-b CDRs | Protein |
| 132-137 | IBIIICI-b CDRs | Protein |
| 138-143 | ADC-1013 CDRs (Kabat) | Protein |
| 144-149 | APX-005M CDRs (Kabat) | Protein |
| 150 | scFv Y-SM083-p03-C06 | Protein |
| 151 | scFv Y-SM083-p04-C04 | Protein |
| 152 | scFv Y-SM083-p04-D04 | Protein |
| 153 | scFv Y-SM083-p04-F04 | Protein |
| 154 | scFv Y-SM083-p04-G04 | Protein |
| 155 | scFv Y-SM083-p04-H04 | Protein |

### Brief Description of the Figures

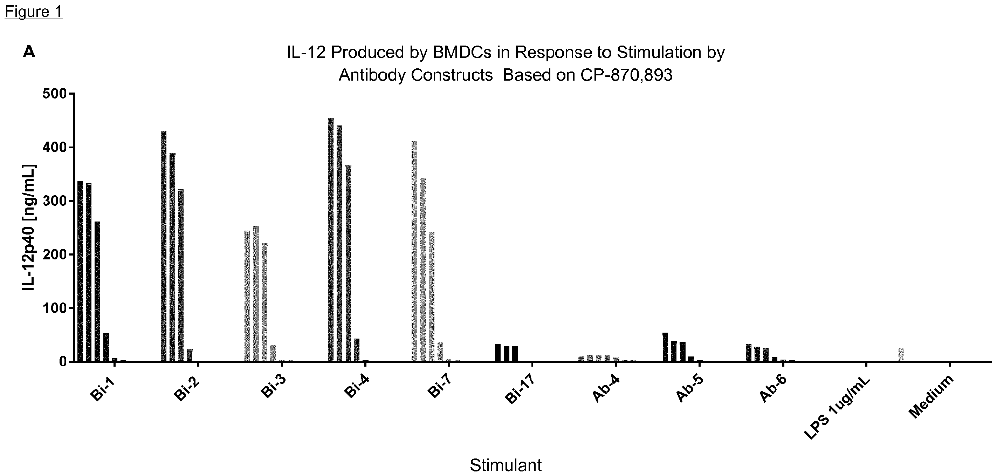

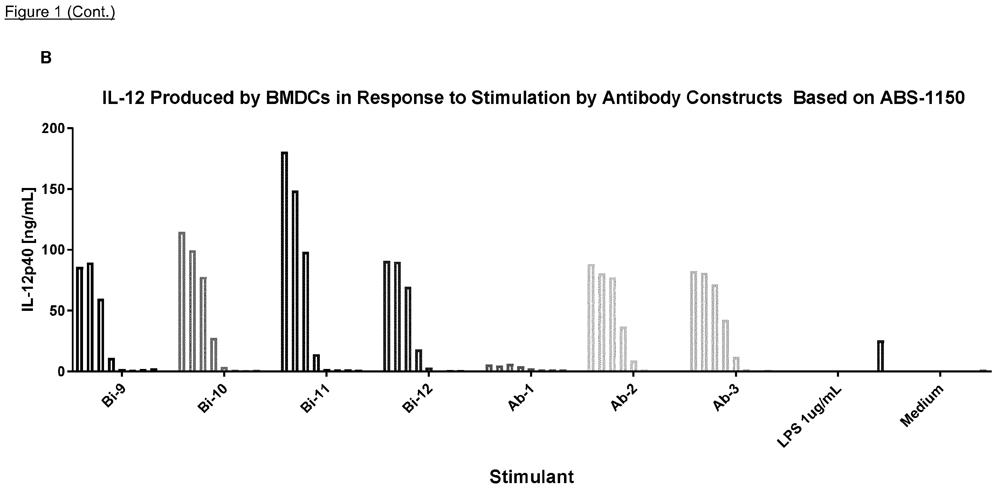

Figure 1 shows the results of IL-12 ELISAs performed on the supernatants of BMDCs cultured in the presence of various stimulants (bispecific antibodies, their parent, monoclonal antibodies and controls). In all cases human CD40-expressing BMDCs were used. Each antibody is referred to by an abbreviation of the name provided in Table 1.
   A shows the results from BMDCs cultured in the presence of antibodies based on CP-870,893; B shows the results from BMDCs cultured in the presence of antibodies based on ABS-1150. Supernatant from BMDCs cultured in the presence of 1 µg/ml LPS was used as a positive control and medium only as a negative control. The mean value of duplicates is presented of each tested concentration. A titration of the antibodies ranging from 0.0064-500 nM was assessed.
   As described below, each antibody was tested at 8 different concentrations. For each antibody, the results from using all 8 concentrations are shown, with the results obtained from use of the highest concentration on the left and the lowest concentration on the right. For some antibodies, the level of IL-12 produced at the lowest antibody concentrations is too low to be visible on the graph. Thus, for instance, if only four results for a particular antibody are shown, these correspond to the results obtained when the four highest concentrations of that antibody were used. LPS is presented as the mean value of duplicates for one concentration and medium represents background activation status.

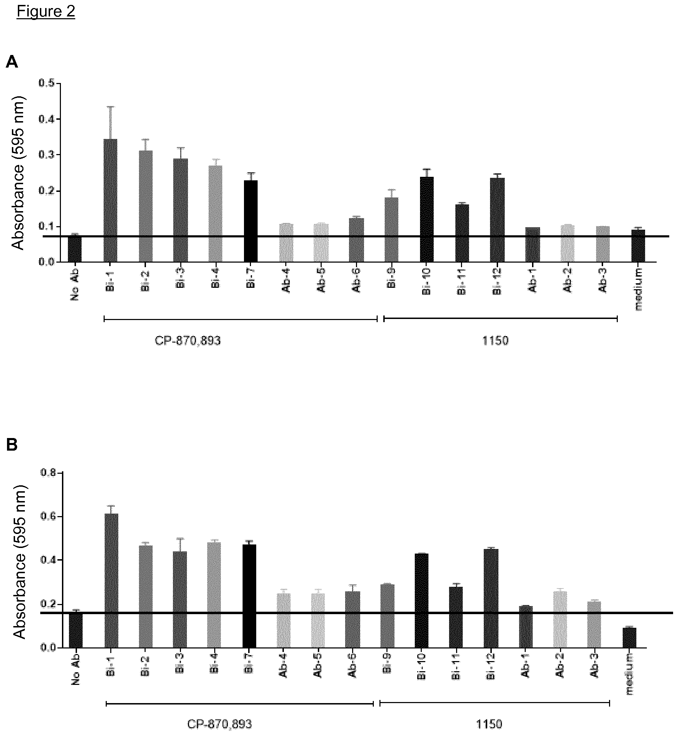

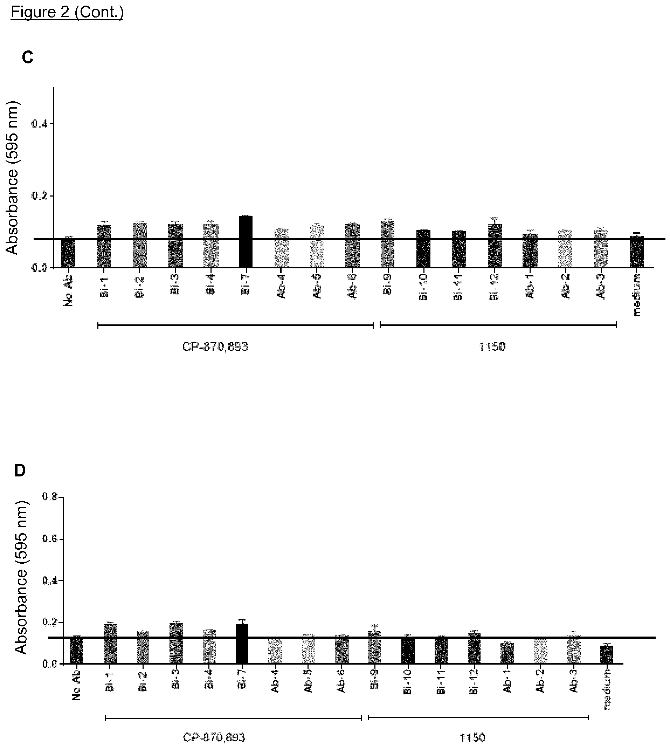

Figure 2 shows the level of T-cell activity (as measured by spectrophotometry based on CPRG hydrolysis) following BMDC/T-cell co-culture experiments. BMDCs were incubated with the antibody constructs shown at a concentration of 10 nM (as indicated results from constructs based on CP-870,893 are shown on the left and constructs based on ABS-1150 on the right; the antibodies are referred to by abbreviations of the names provided in Table 1) and either the SLP UU-05 at a concentration of 125 nM (A) or 250 nM (B), or the SLP UU-10 at a concentration of 125 nM (C) or 250 nM (D). The horizontal line in each figure indicates the basal level of T-cell activation obtained in a no-antibody control. Data is presented as mean values +SEM from duplicate values.

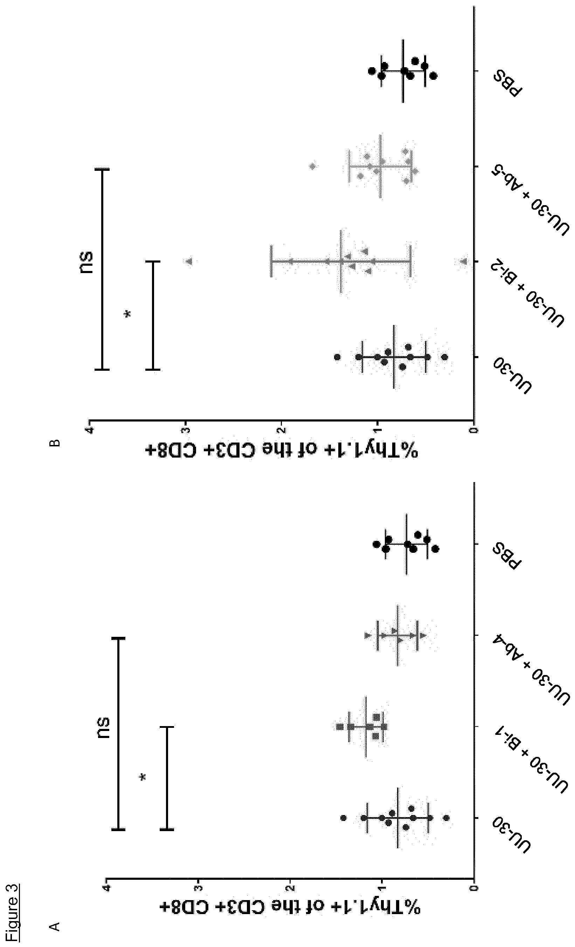

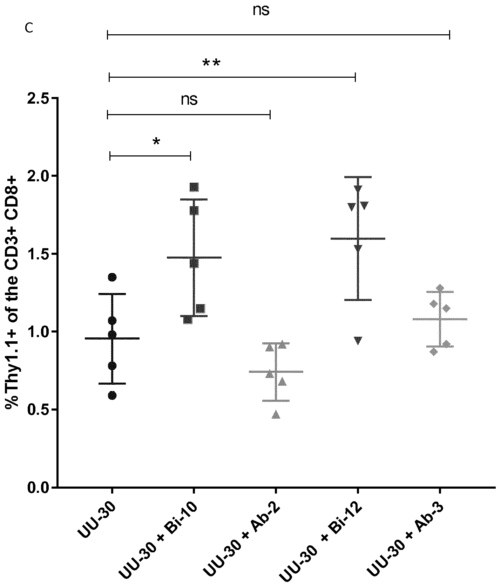

Figure 3 shows the results of *in vivo* experiments in which wild type C57BI/6 mice were injected with BMDCs expressing human CD40 and transgenic T-cells expressing Thy1.1 and a TCR recognising gp100 in MHC class I. The results show the level of proliferating T-cells which recognise an epitope present in the SLP UU-30 for each individual mouse in the group (and the mean+/-SD) in mice injected with that peptide. The left-hand panel (A) shows results obtained in mice administered UU-30 alone (left), UU-30 with the Bi-1 bispecific antibody, UU-30 and the monoclonal parental antibody CP-870,893 of the IgG1 subclass; and a PBS control (right). The right-hand panel (B) shows the same, except the bispecific antibody is the Bi-2 bispecific antibody and the monoclonal parental antibody is CP-870,893 of the IgG2 subclass. In both panels, a statistically significant increase in specific T-cell proliferation is shown in mice injected with UU-30 in combination with the bispecific antibody relative to mice injected with UU-30 alone (significant values were determined by 1way ANOVA with Dunnett's multiple comparisons test, with * representing an adjusted p value of <0.05). Also in both panels, a non-significant (ns) difference in specific T-cell proliferation is shown in mice injected with UU-30 in combination with a parental monoclonal antibody relative to mice injected with UU-30 alone.
Figure 3C shows the results of assessment of ABS-1150/1151 in the same way *in vivo,* with the UU-30 peptide alone or in combination with the various bispecific or parental antibodies of ABS-1150/1151. A significant difference in T-cell proliferation was established when comparing UU-30 with the respective combination of the peptide and the Bi-10 and Bi-12 bispecific antibodies, however when combining UU-30 with the parental antibody there was no significant difference in T-cell proliferation. Statistical analysis was performed using 1way ANOVA with Dunnett's multiple comparisons test, ** indicates p<0.01 and * p<0.05.

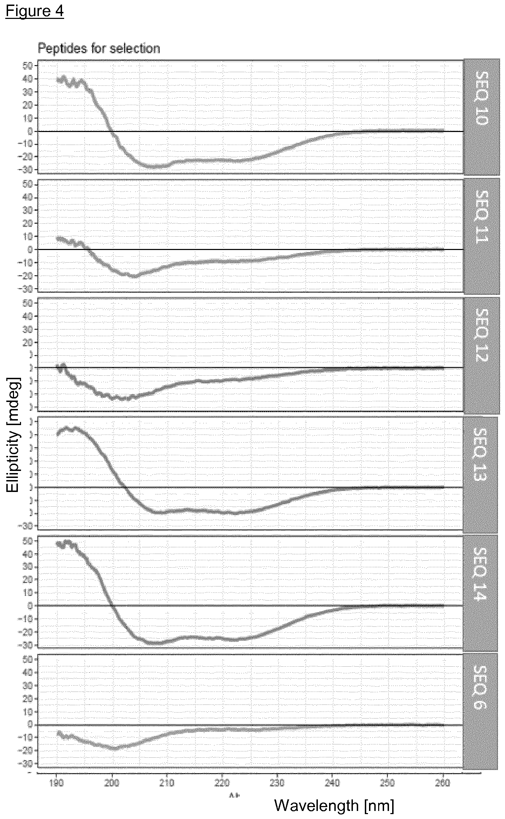

Figure 4 shows the results of circular dichroism spectroscopy of various putative TTx-derived tag peptides as a demonstration of their secondary structure.

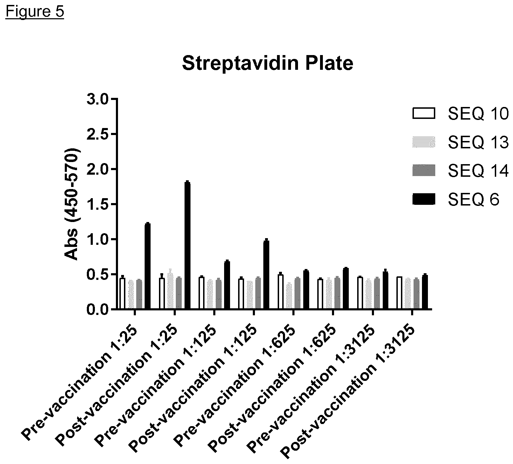

Figure 5 shows the results of ELISA experiments to determine binding of antibodies in serum obtained from individuals taken either just before or just after receipt of a DTP vaccine booster, to putative tag peptides. The level of antibody binding to four putative tag peptides (including the known MTTE peptide) is shown, at four different concentrations of serum. As shown, antibody binding to the putative tags of SEQ ID NOs: 10, 13 and 14 is minimal at all serum concentrations. As expected, significant antibody binding to the MTTE is seen at the lower two concentrations, with higher levels of antibody binding seen from serum taken post the DTP booster vaccine than prior to it.

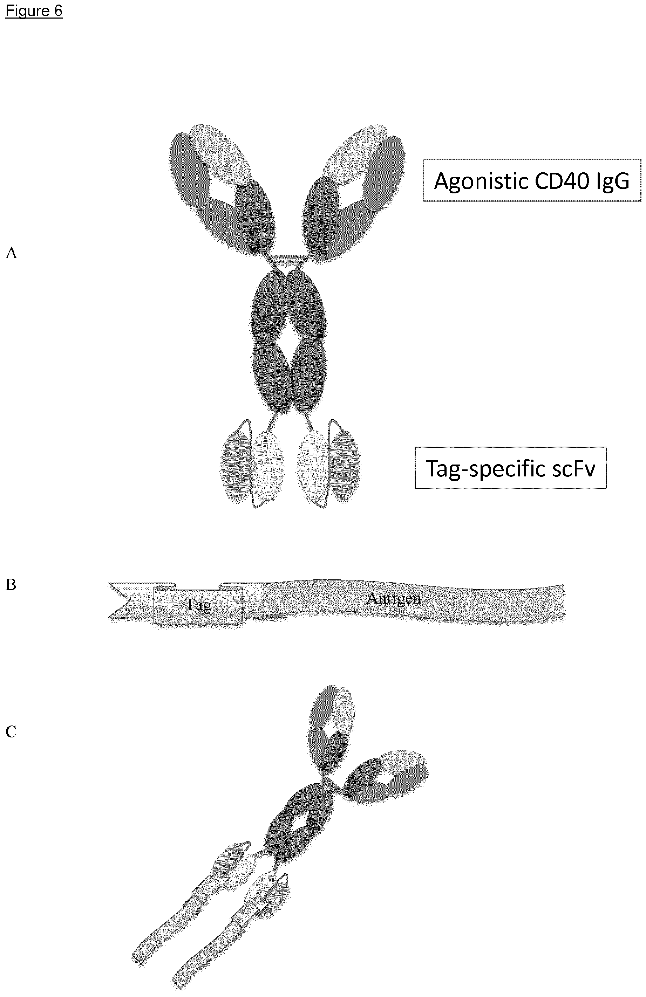

Figure 6 depicts a representative (A) conjugate according to the invention, (B) tag construct, and (C) complex according to the invention. Figure 6A shows a conjugate comprising an agonistic anti-CD40 IgG antibody as the first specific binding molecule, and two scFv specific for a tag moiety (e.g. a tag peptide) as second specific binding molecules, each linked to a a separate CH3 domain of the antibody, thereby providing a tetravalent conjugate (two valencies for CD40 and two for the tag peptide. Figure 6B shows a tag construct comprising a tag moiety (e.g. a tag peptide) linked to an antigenic peptide. Figure 6B shows a complex comprising a tag construct bound, via the tag moiety (e.g. tag peptide), to each of the two scFv of the conjugate.

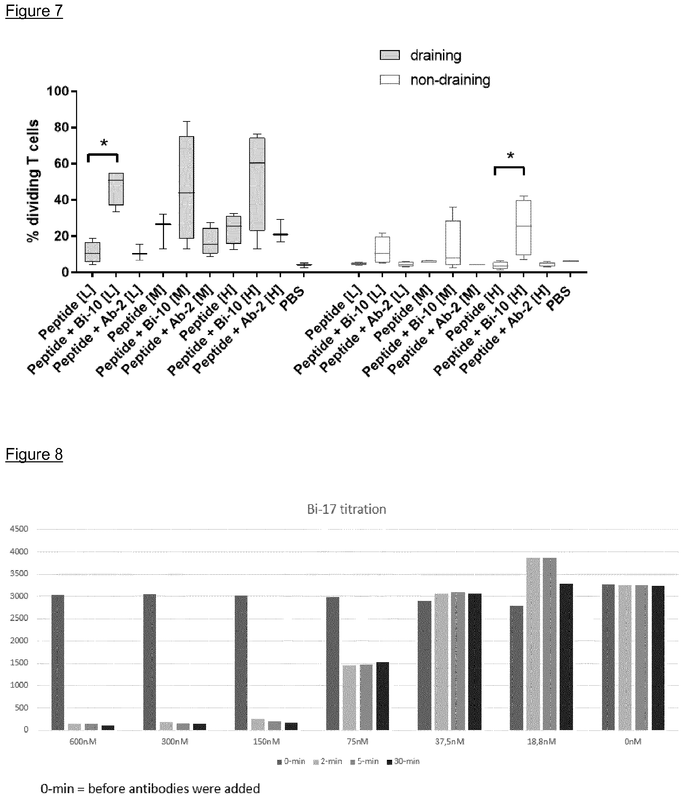

Figure 7 shows the percentage of proliferating T-cells in the draining (left-hand graph) and non-draining (right-hand graph) lymph nodes in mice. As shown, co-administration of a bispecific conjugate according to the invention and tag construct stimulates an antigen-specific T-cell response much more efficiently than does administration of a bispecific conjugate alone or a tag construct alone. The conjugates and constructs were administered in low (L), medium (M) or high (H) amounts, as described below. Asterisks indicate statistical significance (*p<0.05).
Figure 8 shows the effect of incubating a FITC labelled peptide with Bi-17, a bispecific conjugate containing the anti-FITC scFv FITC-8. The scale on the left indicates fluorescence intensity. As shown, before addition of Bi-17 high fluorescence signals are detected (at 0 minutes). Addition of a high concentration of Bi-17 completely quenches fluorescence. As the concentraton Bi-17 is reduced, such that an excess of FITC-labelled peptide is present, quenching is reduced.

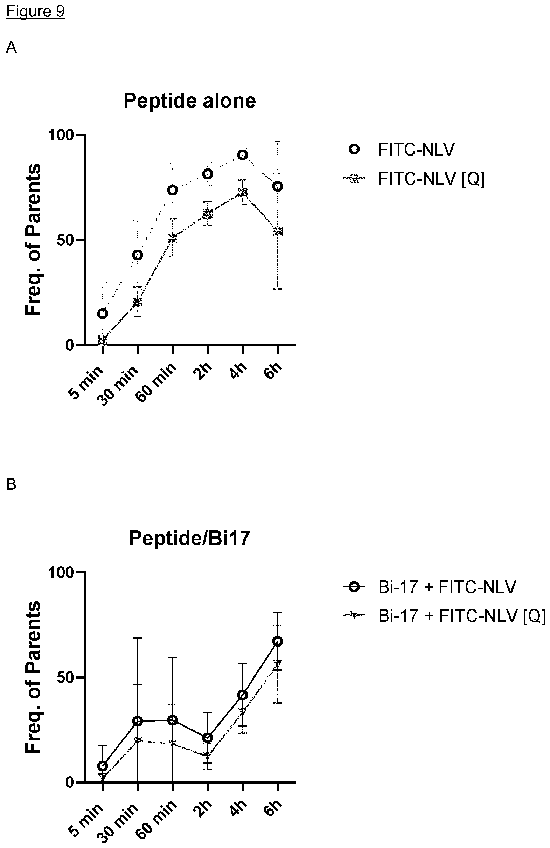

Figure 9 shows uptake of FITC-labelled peptide by dendritic cells, as measured by cellular fluorescence. "FITC-NLV" indicates all fluorescent cells, "FITC-NLV [Q]" indicates cells with intracellular fluorescence (obtained by quenching external fluorescent signal with trypan blue). **A** displays uptake of naked peptide by dendritic cells; **B** displays of uptake of peptide when the peptide was applied to dendritic cells as a complex with a bispecific antibody.

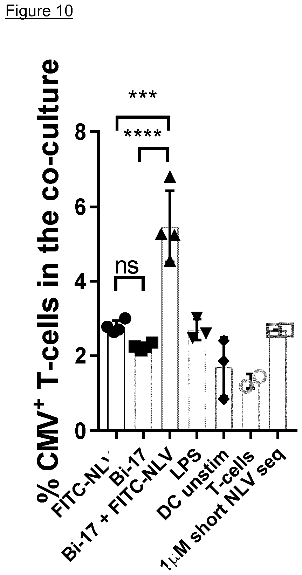

Figure 10 shows expansion of T-cells specific for a CMV antigen (as measured by the proportion of CMV-specific T-cells in the culture) when contacted with a bispecific conjugate according to the invention and tag construct (containing a CMV antigen). As shown, significantly more specific T-cell expansion was seen when the cells were contacted with a bispecific antigen and a tag construct than the bispecific antigen alone or the tag construct alone. ***p<0.001, ****p<0.0001, NS = not significant.

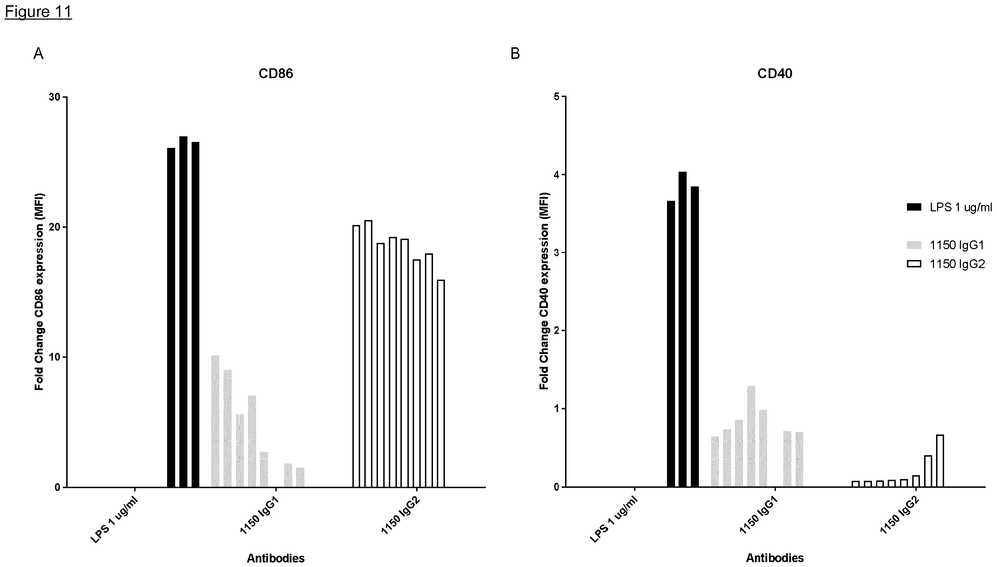

Figure 11 shows the effect of the anti-CD40 antibody ABS-1150/1151 on cell surface expression of CD86 (**A**) and CD40 (**B**) by dendritic cells. The effects of an antibody of the IgG1 isotype (X-SM083-ab-1) are compared to the effects of an antibody of the IgG2 isotype (X-SM083-ab-2). The effect on protein expression is shown in terms of fold-change relative to unstimulated cells. For each antibody, the multiple bars represent different concentrations in the titration range (left to right, high concentration to low concentration). As shown, the IgG2 isotype has greater effect in terms of increasing CD86 cell surface expression and reducing CD40 cell surface expression than does the IgG1 isotype.

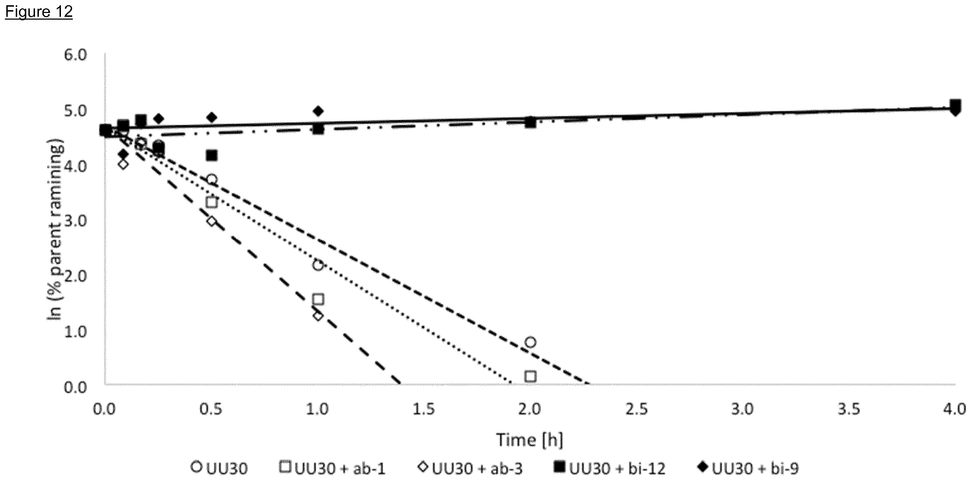

Figure 12 shows stability of the UU-30 peptide in mouse plasma as measured by mass spectrometry. The peptide was incubated in plasma alone, in combination with two anti-CD40 monoclonal antibodies and in complex with two bispecific antibodies according to the invention. Complexing of the UU-30 peptide with a bispecific antibody of the invention vastly increased its stability in mouse plasma.

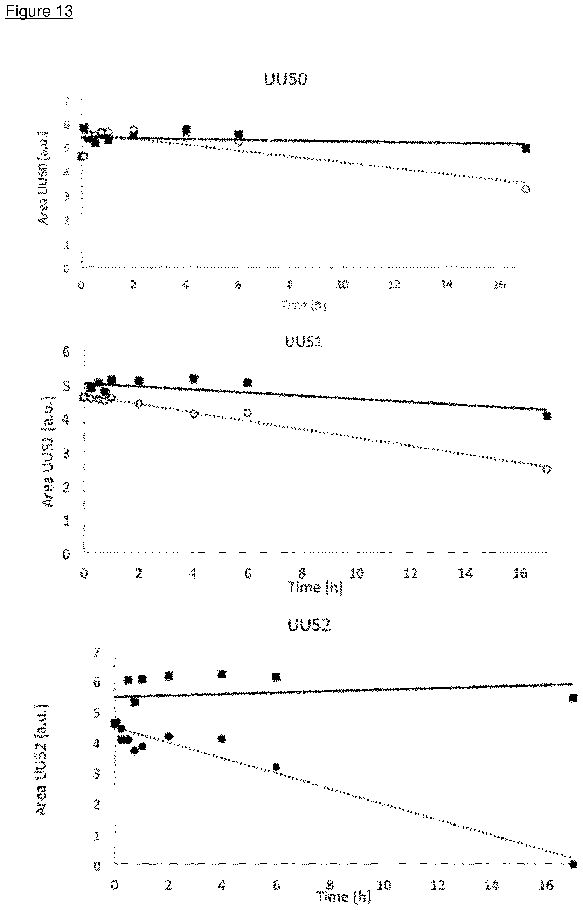

Figure 13 shows stability of 3 putative peptide tag moieties in mouse plasma (circles) and human plasma (squares) as measured by mass spectrometry. The tested peptides are UU-50 (SEQ ID NO: 10), UU-51 (SEQ ID NO: 12) and UU-52 (SEQ ID NO: 13).

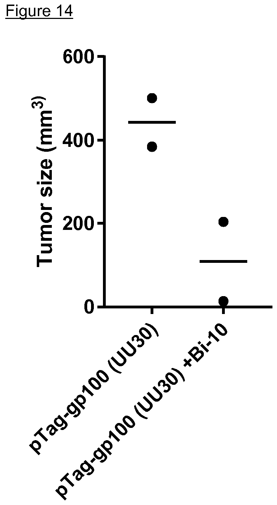

Figure 14 shows tumour load in a mouse model of melanoma. Tumour volume 20 days post tumour inoculations is compared between mice administered the tag construct UU-30 only (UU-30 contains an epitope from the melanoma antigen gp100, as detailed below), and mice administed the tag construct UU-30 in combination with the bispecific antibody Bi-10, which binds the UU-30 tag moiety. As shown, administration of UU-30 in combination with a bispecific antibody of the invention results in lower tumour growth than administration of UU-30 alone.

### Detailed Description of the Invention

The invention is as defined in the claims.

The invention relates to bi-specific conjugates comprising at least two specific binding molecules covalently linked together, a first specific for CD40 and a second specific for a tag moiety.

A specific binding molecule can be any binding partner (e.g. affinity binding partner) for the target molecule in question, i.e. for the CD40 or the tag moiety. That is, it can be any molecule that binds to the CD40 or tag moiety (i.e. any molecule capable of binding to CD40 or the tag moiety). According to the invention, the specific binding molecule is a specific binding protein comprising an antigen-binding domain of an antibody. By "specific" it is meant that the molecule binds to its target in a manner that can be distinguished from binding to non-target molecules, more particularly with greater binding affinity. That is, the binding molecule does not bind to other, non-target, molecules, or does not do so to an appreciable or significant degree, or binds with lower affinity to such other molecules. "Specific for" may alternatively be expressed as "directed against".

The term "CD40" refers to CD40 from any species. Thus, it may be human CD40 or its equivalent or corresponding molecule in other species, most notably other mammals. Preferably it is human CD40. The amino acid sequence of human CD40 is shown in SEQ ID NO: 50. In particular, the first specific binding molecule binds to CD40 in its native state, and more particularly CD40 when localised on the surface of a cell, or more particularly when expressed on the surface of cell.

The first specific binding molecule is capable of agonising CD40. That is, the molecule is capable of activating CD40. In other words, the first specific binding molecule is capable of enhancing CD40 signalling. The first specific binding molecule may bind to CD40 in the absence of CD40L and enhance the signalling of CD40. Alternatively, it may bind to a CD40:CD40L complex and enhance CD40 signalling. A first specific binding molecule may completely block binding of CD40L to CD40, or it may reduce binding of CD40L to CD40, or it may neither reduce nor block binding of CD40L to CD40. Thus, it may bind to an epitope close to the CD40L binding site on CD40, or to an overlapping epitope, or to a separate epitope distinct from the CD40L binding site It may fully or partially affect the CD40L binding site by inducing a conformational change. The first specific binding molecule can increase the activity of (i.e. activate or stimulate) a cell expressing CD40, notably an APC expressing CD40, such as a dendritic cell, or a B-cell, macrophage, monocyte or any myeloid cell. The cell may be CD11b-positive or CD11c-positive. In a particular embodiment, the first specific binding molecule is capable of activating DC.

Professional APCs, such as DC, are activated when signalling via CD40 occurs, which triggers several biological events, including immune cell activation, proliferation, and production of cytokines and chemokines. Methods for determining DC, or other APC, activation by CD40 are known in the art (see for example Schonbeck et al. 2001, Cell Mol Life Sci., 58:40-43, and WO 2015/091853 and US 2017/0342159) and are described in the Example below.

It is also known and reported in the art how to determine the ability of a specific anti-CD40 binding molecule to modulate (or increase) the activity of an APC such as a DC, for example by measuring the level of cell-surface markers such as CD86 and CD80, measuring cytokine release (e.g. IL-12 release by DC), and/or by measuring anti-CD40 binding molecule-induced T-cell activity (e.g. secretion of IFN-γ). An *in vitro* T-cell activation assay is described in Example 1 below.

In some embodiments the first specific binding molecule may increase the activation of DC with a potency (measured as an EC50, e.g. as described in Example 3 of WO 2015/091853) of 5 µg/ml or lower, e.g. 4, 3, 2.5, 2, 1.5, 1, 0.5, 0.4, 0.3 or 0.2 µg/ml or lower, typically between 0.1 µg/ml and of the upper limits specified above.

As is well known in the art, CD40 is a cell surface protein. Upon engagement by a ligand or agonist, CD40 may be internalised. TNFR internalisation can be a way of regulating immune activation. Antibodies to CD40 may have different internalisation rates. Without being bound by theory, it is possible that antibody internalisation is linked to agonistic capacity. Accordingly, the anti-CD40 antibody drives measurable internalisation of CD40. Internalisation of the antibody-CD40 complex results in internalisation by the target cell of the antigen in the tag construct bound to the second specific binding molecule, and thus may promote antigen cross-presentation. Thus the first specific binding molecule induces CD40 internalisation upon binding to CD40. CD40 internalisation may be experimentally measured as described in the Examples below.

The tag moiety is a peptide and not a cancer antigen, and consists of the amino acid sequence set forth in any one of SEQ ID NOs: 6 or 10-14, or a fragment of any one of SEQ ID NOs: 6 or 10-14 of at least 5 amino acids. It is a binding partner for the second specific binding molecule, and may be used to allow the conjugate to bind to a tag construct containing the tag moiety. Thus, the tag moiety is a member of an affinity binding pair. The term "peptide" is used broadly herein to include polypeptides and proteins, and implies no restriction on the number of amino acid residues, but rather indicates the proteinaceous nature of the molecule. The term "peptide" is thus synonymous or interchangeable with "polypeptide". A peptide may be a compound comprising two or more subunit amino acids, amino acid analogues or peptidomimetics. An amino acid be natural or synthetic and be a D or L optical isomer. The tag moiety may be a naturally occurring molecule, or fragment or part thereof, or it may be an artificial or synthetic molecule. Tag moieties and tag peptides are described further below.

For use according to the present invention, the tag moiety is provided as part of a tag construct wherein the tag moiety is covalently attached to an antigen, as described elsewhere herein. Accordingly, the tag moiety is typically synthetically produced.

As detailed above, the tag moiety is not a cancer antigen, and thus the second specific binding molecule does not bind to a cancer antigen. In particular, the tag moiety is not a human cancer antigen. That the tag moiety is not a cancer antigen includes that the tag moiety is not a part of a cancer antigen, i.e. it is not an amino acid sequence from a cancer antigen. In particular, the tag moiety is not an epitope from a cancer antigen.

Cancer antigens (or tumour antigens) include, as described further below, both tumour-specific antigens (i.e. neoepitopes arising from mutations) and tumour-associated antigens. A cancer antigen, as referred to herein, is in particular a protein which may be targeted in cancer therapy, e.g. by a therapeutic antibody or other drug entity.

In an embodiment, the tag moiety does not comprise any B-cell epitopes. In a particular embodiment, the tag moiety does not comprise any human B-cell epitopes. The tag moiety may be designed or selected so that it does not interact, or does not interact to any significant or substantial degree, with any endogenous antibodies which may be present in a subject to whom the conjugate, or a complex comprising the conjugate, is administered. In one embodiment competition for binding of the tag moiety between the conjugate of the invention and endogenous antibodies in a subject to whom the conjugate and tag of the invention are administered may be avoided. Accordingly, in an embodiment the second specific binding molecule may out-compete any endogenous antibodies of the subject for binding to the tag moiety, e.g. at the time that the conjugate is first administered.

A tag moiety which does not comprise any B-cell epitopes (for example human B-cell epitopes) may be non-immunogenic, in order to avoid the generation of an immune response to the tag moiety (or to any part of the conjugate/complex, other than the antigen of the tag construct) when it is administered.

Alternatively, in an embodiment a tag moiety may not comprise any natural B-cell epitopes (for example the tag moiety may not comprise any natural human B-cell epitopes). By a "natural B-cell epitope" is meant an epitope against which a subject, for example a human (in particular a patient to whom the conjugate is to be administered), has no circulating antibodies prior to administration of the conjugate. In this embodiment it is possible that administration of the tag moiety to a subject would induce anti-tag antibodies over time.

It will be understood in this respect that whether or not a given amino acid sequence (or other molecule) in a tag moiety represents a B-cell epitope may depend on the species of the subject to which it is administered, the subject's MHC repertoire and what antigens that subject has previously been exposed to. Thus, a tag moiety derived from a peptide or protein to which human subjects are exposed may comprise a human B-cell epitope, but the epitope sequence in question may not act as B-cell epitope in other species or is not a universal B-cell epitope.

By "universal" B-cell epitope is meant a B-cell epitope bound by antibodies present in the majority of individuals in a population. For example, a universal human B-cell epitope is an epitope against which the majority of individuals in a given population have antibodies. Universal B-cell epitopes may be, but are not limited to, epitopes present in proteins or peptides commonly administered in vaccines. For instance, tetanus toxin (TTx) contains a number of universal B-cell epitopes. Without being bound by theory, it is believed that this is because tetanus toxoid (TTd), an inactivated form of TTx, is utilised in the tetanus vaccine administered to almost all people worldwide. In an embodiment, a universal human B-cell epitope is a B-cell epitope recognised by the endogenous antibodies of at least 75 % of individuals in a population. A universal B-cell epitope may alternatively be defined as a common B-cell epitope.

A B-cell epitope can either be linear (a continuous amino acid sequence) or conformational (a non-continuous series of amino acids from a protein, recognised by an antibody in the context of the protein's secondary or tertiary structure).

For example, a tag peptide based on a TTx peptide (as described further below) may contain certain sequences which are human B-cell epitopes (e.g. the MTTE epitope, which is a natural linear B-cell epitope as described below, and in the examples), since anti-TTx antibodies are frequently found in human subjects and may thus interact with certain TTx-derived peptides. However, mice generally do not have circulating antibodies to the MTTE as they have not been exposed to tetanus toxoid and thus have not developed antibodies that recognise it. However, not all TTx-derived peptides are (or contain) universal human B-cell epitopes, and this can be determined by screening as discussed further below.

In another embodiment the tag moiety may comprise a natural B-cell epitope (for example a natural human B-cell epitope). In this embodiment it may be advantageous if the tag moiety is bound less strongly (i.e. more weakly) by endogenous circulating antibodies than by the second specific binding molecule. It may also be advantageous if the tag peptide adopts a different conformation when a naked peptide relative to its conformation in the context of the protein from which it is derived (and against which the endogenous antibodies have been generated), since in this case the endogenous antibodies may not bind the naked peptide, or may bind it only weakly. In particular, the second specific binding molecule may bind the tag moiety more strongly than do endogenous circulating antibodies in the subject to which the conjugate and tag moiety of the invention are administered.

The strength of antibody/specific binding molecule binding to the tag moiety may be quantified in the form of the dissociation constant (K_{d}). The greater the K_{d} the weaker the interaction (and vice versa). The K_{d} of a binding interaction between the tag moiety and an antibody or specific binding molecule may be determined by a variety of methods known in the art, e.g. surface plasmon resonance (SPR). Thus in an embodiment, the tag moiety comprises a natural human B-cell epitope but is bound by the second specific binding molecule with a lower K_{d} than it is bound by human endogenous circulating antibodies. In particular, the tag moiety may comprise a natural human B-cell epitope but be bound by the second specific binding molecule with a lower K_{d} than it is bound by endogenous circulating antibodies of the subject to be treated. The K_{d} of the interaction between the tag moiety and the second specific human antibody may be e.g. about 2-fold, 3-fold, 4-fold, 5-fold, 10-fold, 50-fold, 100-fold or 1000-fold or more lower than the K_{d} of the interaction between the tag moiety and endogenous circulating antibodies in a subject to be treated.

The tag moiety is a non-mammalian peptide. That is to say, the tag moiety is a peptide as defined in the claims, which has (comprises or consists of) an amino acid sequence not found in a mammal. By an amino acid sequence not found in a mammal is meant an amino acid sequence which is not encoded by a mammal in its genome. Thus, for instance, a bacterial protein secreted by a gut bacterium resident in the gut of a mammal, and thus which may be isolated from a mammal, is not, for the purposes of the present application, considered to be "found in a mammal". Thus the peptide is not a mammalian protein, or a fragment of a mammalian protein. Thus in this embodiment the second specific binding molecule does not bind to a mammalian protein.

Alternatively, the tag moiety is a peptide as defined in the claims, which is not expressed on the surface of a mammalian cell. That is to say, the tag moiety is not a mammalian cell surface protein, or a peptide derived from a mammalian cell surface protein (e.g. a part or fragment of a mammalian surface protein). In this embodiment the second specific binding molecule does not bind to a mammalian cell surface protein.

A tag peptide may be selected or designed not to include any human sequences, that is the tag peptide may not include any amino acid sequence corresponding to (or obtained or derived from) an amino acid sequence of or in a polypeptide which occurs in a human. That is to say, the tag moiety (or tag peptide) may be a non-human peptide, i.e. the tag moiety is not a human protein or a part or fragment of a human protein. Equivalently to the above discussion of a mammalian peptide, a non-human peptide has (comprises or consists of) an amino acid sequence not found in a human. An amino acid sequence not found in a human is an amino acid sequence not encoded by the human genome, and thus not produced by a native human cell. Thus in this embodiment the second specific binding molecule does not bind to a human protein.

In another embodiment the tag peptide may be selected or designed not to include any canine sequence, such that the tag peptide does not include any amino acid sequence corresponding to (or obtained or derived from) an amino acid sequence of or in a polypeptide which occurs in a dog. Thus in this embodiment, the second specific binding molecule does not bind to a canine protein.

The tag peptide may be a natural peptide, i.e. a peptide with a sequence derived from a natural protein sequence. In this case, it is preferred that the peptide sequence is derived from a protein of non-mammalian or non-human origin, e.g. from a prokaryotic or microbial protein, such as a bacterial protein or a yeast protein etc. As in the examples shown below, the tag peptide may be derived from *Clostridium tetani.* Alternatively, the tag peptide may have an artifical sequence, i.e. an amino acid sequence not found in nature. Thus the second specific binding molecule may bind a prokaryotic or microbial protein, such as a bacterial protein. Alternatively the second specific binding molecule may bind only an artificial sequence, such that the second specific binding molecule does not bind any naturally-occurring protein.

In a particular embodiment the tag peptide has an α-helical structure. In another embodiment, the tag peptide is unstructured, i.e. it does not adopt a particular secondary structure. As detailed in the Example, peptide secondary structure can be predicted using publicly-available software (e.g. Jpred4 (http://www.compbio.dundee.ac.uk/jpred/, Drozdetskiy et al., Nucl. Acids Res. 43(W1): W389-W394, 2015) and PASTA 2.0 (http://protein.bio.unipd.it/pasta2/, Walsh et al., Nucl. Acids Res. 42(W): W301-W307, 2014). Peptide secondary structure can be experimentally determined by circular dichroism spectroscopy, as is well known in the art (see e.g. Greenfield, N., Nat Protoc. 1(6): 2876-2890, 2006).

In one embodiment a consideration in selecting a tag peptide is that the peptide should not have a variable structure. In other words, without being bound by theory, it may be desirable that the structure of the tag peptide does not vary, or alter, depending on the context. For instance, it may be desirable that the structure of the tag peptide is the same when it is "naked" in solution (i.e. unmodified), when it is bound to a support (i.e. immobilised) and when its N-terminus and/or C-terminus is modified, e.g. if the peptide is synthesised in the context of a fusion protein with an antigen, or chemically labelled, etc. If the peptide has a changeable structure it may not bind the second specific binding molecule with high affinity in some contexts. Circular dichroism spectroscopy, for example, may be used to determine whether the structure of a tag peptide changes between contexts.

In a particular embodiment, the tag peptide may comprise a CD4 epitope (i.e. an epitope recognised or recognisable by a CD4+ T- cell). The CD4 epitope is derived from tetanus toxin. The presence of such an epitope in the tag peptide could enable the tag itself to act as an adjuvant, by presenting the CD4 epitope on the APC to a T-helper cell, and thereby attracting help for a CD8+ T-cell that recognises a CD8+ epitope in the antigen of the tag construct.

The tag peptide is derived from tetanus toxin. Examples of suitable tag peptide sequences derived from tetanus toxin include the MTTE, described below, which comprises a known human CD4 epitope and has the amino acid sequence set forth in SEQ ID NO: 6. Other examples of suitable tag peptides derived from tetanus toxin include those of SEQ ID NOs: 10-14, which do not include any known universal human CD4 epitopes. Thus the second specific binding molecule may bind tetanus toxin, in particular at SEQ ID NO: 6 or any one of SEQ ID NOs: 10-14. In another embodiment the tag peptide is a part of the MTTE, of e.g. at least 5 amino acids. For instance the tag peptide may be a fragment of the MTTE of 5-16, 6-15, 7-14, 8-13 or 10-12 amino acids. The tag peptide may also be a part of one of the tetanus toxin-derived peptides of SEQ ID NOs: 10-14, for instance a fragment of any one of SEQ ID NOs: 10-14 of at least 5 amino acids (e.g. a fragment of 8-15, 10-15, 8-12 or 10-12 amino acids). The tag peptide may be the tetanus toxin-derived peptide of SEQ ID NO: 121 (which is a fragment of SEQ ID NO: 12) or any one of the tetanus toxin-derived peptides of SEQ ID NOs: 122, 123, 124 and 125 (which are fragments of SEQ ID NO: 13). Thus the second specific binding molecule may bind tetanus toxin at any one of SEQ ID NOs: 121-125.

The first and second specific binding molecules are covalently linked. This includes that the respective molecules are directly linked to one another by a covalent bond, or that they are covalently linked via a linker molecule or linker group (i.e. they may be directly or indirectly linked to one another by a covalent linkage). It is well known in the art and widely described in the literature how to link two molecules, and particularly two proteins or polypeptides, together, whether directly (e.g. by a peptide bond, or by activating chemical groups in the respective molecules, in a manner which allows them to interact (e.g. react) together), or indirectly by means of a linker. For example, a specific binding molecule may be reacted with a bifunctional agent capable of reacting with thiol groups present in another binding molecule (or indeed in linker molecule), for example the N-hydroxysuccinimide ester of iodoacetic acid (NHIA) or N-succinimidyl-3-(2-pyridyldithio)propionate (SPDP). Amide and thioether bonds may for example be achieved with m-maleimidobenzoyl-N-hydroxysuccinimide ester. Many linker molecules are known, and are standard in the art for linking the component parts of a conjugate together, including in the field of antibody constructs, and any such linker can be used.

The first and second binding molecules are polypeptides or proteins, and they may be linked directly to one another by a peptide bond, or by the intermediacy of a linker which conveniently takes the form of an amino acid sequence. Thus, the conjugate may take the form of, or may comprise, a fusion protein (or fusion polypeptide) wherein the first and second specific binding proteins (or constituent polypeptides e.g. chains, thereof) are linked directly together, or indirectly via a linker sequence. For example, typically linkers may take the form of GGGGSn, (SEQ ID NO: 51) wherein n may typically be 1-10, e.g. 2-6. A representative linker (where n=2) is shown in SEQ ID NO: 49. The linker plays no functional or effector role, other than to link the two binding molecules (or chains or parts thereof) together.

The first and second specific binding proteins, or constituent polypeptide (e.g. chains) thereof, may be linked in either order, e.g. with the first specific binding protein (or chain thereof) joined at an N- or C-terminal end thereof to an N- or C-terminal end of the second specific binding protein (or chain thereof).

As noted above, the specific binding molecules are antigen-binding proteins. The term "antigen-binding protein" is used herein to denote a binding protein comprising an antigen-binding domain obtained or derived from an antibody, or based on an antigen binding domain of an antibody. Thus, the antigen-binding protein may be an antibody-based, or antibody-like, molecule comprising the binding site of, or a binding site derived from, an antibody. It is thus an immunological binding agent. The antigen-binding protein may for example comprise light and/or heavy chain variable regions from an anti-CD40 antibody, or the CDRs from an anti-CD40 antibody. An antigen-binding protein may thus be a native antibody or a fragment thereof, or an artificial or synthetic antibody, or an antibody construct, or derivative (e.g. a single chain antibody, as discussed further below). For the avoidance of doubt, in accordance with the present invention the antigen binding domain is not from a T-cell receptor (TCR). Thus, for the avoidance of doubt, antigen binding proteins in accordance with the present invention do not include TCRs.

Preferably, the antigen binding protein is an antibody or an antigen binding fragment or derivative thereof.

The term "antibody" as used herein refers to an immunoglobulin molecule. An antibody thus comprises at least two heavy (H) chains and two light (L) chains inter-connected by disulphide bonds. Each heavy chain is comprised of a heavy chain variable region (VH) and a heavy chain constant region. Each light chain is comprised of a light chain variable region (VL) and a light chain constant region. The VH and VL contain the binding domain that interacts with an antigen. The VH and VL can be sub-divided into regions of hypervariability, termed complementarity determining regions (CDR), interspersed with regions that are more conserved, termed framework regions (FR). Each VH and VL typically has 3 CDRs (which may be used to define the antigen-binding domain/site), and 4 FRs. The constant regions of the antibody may mediate the binding of the antibody to various cells or factors. The Fc region of the antibody is composed of parts of the constant regions of the two heavy chains (which contribute two or three constant domains to the Fc, depending on the class of the antibody), and this region in particular may bind to the Fc receptor which is present on certain cells, including APC, and may play a role in the ability of the antibody to stimulate the cell.

An antibody may be a polyclonal or a monoclonal antibody. The antibody may be of any isotype (or class), which is determined by the type of constant domain in the heavy chains. Thus it may be an IgA, IgD, IgE, IgG, or IgM antibody. Preferably it is an IgG antibody. Several of these classes are further divided into subclasses, such as IgG1, IgG2, IgG3, IgG4, and the like, and the antibody may be of any subclass. The heavy-chain constant domains that correspond to the difference classes of immunoglobulins are termed α, δ, ε, γ and µ, respectively. The subunit structures and three-dimensional configurations of different classes of immunoglobulins are well known.#The present inventors have demonstrated that IgG antibodies of the subclass IgG2 (i.e. IgG2 antibodies) have a particularly strong internalisation effect on CD40. Binding of an agonistic IgG2 antibody to CD40 is particularly effective in driving internalisation of CD40. Importantly, agonistic IgG2 antibodies are more effective at driving CD40 internalisation than IgG1 antibodies. As demonstrated in the Examples below, an IgG2 antibody is more effective at driving CD40 internalisation than an IgG1 antibody with the same antigen-binding domain, demonstrating the importance of the antibody isotype in driving CD40 internalisation and thus internalisation of any antigen bound to the second specific binding molecule. Thus use of an antibody of IgG2 isotype may result in superior MHC class I or II antigen loading, and hence superior immune activation. Given its efficacy in driving CD40 internalisation, an IgG2 antibody which is an agonist of CD40 is particularly preferred for use as the first binding molecule in the conjugate of the present invention.

The "light chains" of mammalian antibodies are assigned to one of two clearly distinct types: kappa (κ) and lambda (λ), based on the amino acid sequences of their constant domains and some amino acids in the framework regions of their variable domains. In some embodiments, kappa (κ) light chains are preferred.

In some embodiments, the specific binding molecule, and particularly the first specific binding molecule (which binds CD40), is an antibody or a fragment thereof. In particular it is an intact or whole antibody (i.e. a "full length" antibody comprising a full complement of H and L chain constant regions). In other embodiments the specific binding molecule, and particularly the second specific binding molecule, is an engineered or synthetic antibody construct.

The specific binding molecule may be an antigen-binding fragment of an antibody, that is a fragment which retains the ability of the antibody to bind specifically to an antigen (e.g. CD40, or a tag moiety). Such fragments are well known and examples include Fab', Fab, F(ab')₂, Fv, Fd, or dAb fragments, which may be prepared according to techniques well known in the art.

Alternatively the specific binding molecule may be a synthetic or artificial construct, i.e. an antibody-like molecule which comprises an antigen-binding domain, but which is genetically engineered or artificially constructed. This includes chimeric or CDR-grafted antibodies, as well as single chain antibodies and other constructs, e.g. scFv, dsFv, ds-scFv, dimers, minibodies, diabodies, single domain antibodies (DABs), TandAbs dimers, heavy chain antibodies such as VHH and camel antibodies etc. A scFv typically comprises, N-terminal to C-terminal, a VH region linked to a VL region by a linker sequence. The linker may be an amino acid sequence as discussed above. An exemplary linker sequence is GGGGS₄. However, alternative formats for single chain antibodies are possible. The preparation of such molecules is well known in the art, and they may be screened for utility in the same manner as intact antibodies.

The first and second specific antigen-binding proteins may be human or humanised proteins. A human protein may comprise VH and VL regions in which both framework and CDR regions are derived from human germline immunoglobulin sequences, and also the constant region, if this is contained in the protein. Such proteins may however include amino acids not encoded by human germline Ig sequences, for example mutations introduced by random or site-specific mutagenesis). Humanised proteins may comprise CDR sequences derived from the germline of another species (e.g. a mouse) grafted onto human framework sequences, or human "scaffold" sequences, and/or wherein certain amino acids of non-human variable regions are changed to better correspond with the amino acids typically present in human antibodies. Humanised proteins are thus chimeric proteins comprising both human-derived and non-human derived sequences. The non-human sequences may be minimal.

In another embodiment, the conjugate may be designed for use in veterinary medicine, and in particular for use in dogs. In this embodiment the first specific binding molecule may be a canine specific binding molecule, e.g. a canine antibody. In this embodiment the first specific binding molecule is an agonist for canine CD40 and preferably has canine heavy chain constant regions. The antibody may be a canine IgG antibody. The canine subclass IgGB is to some degree the canine equivalent of human IgG2, and thus for the same reasons that the IgG2 subclass is preferred for use in humans the conjugate for use in dogs is preferred to utilise an IgGB antibody which is an agonist of canine CD40 as the first specific binding molecule.

The antigen-binding protein may thus be prepared, expressed, isolated or created by recombinant or synthetic means according to techniques and principles well known in the art.

It is preferred for the conjugate to have more than one binding domain for each of its two targets, i.e. for each of CD40 and the tag moiety. In other words it is preferred for the conjugate to have a valency of at least 2 for each target. In this context, valency can be seen as equivalent to the number of binding domains for a target. Thus a valency can be viewed as a target binding domain. Accordingly, the first and/or second specific binding molecules may each have more than one (antigen) binding domain and/or the conjugate may comprise more than one first and/or second specific binding molecule. A specific binding molecule may thus be monovalent, or it may have a valency of two or more, i.e. it may comprise one, or two or more binding domains, for example 2-6, or 2-4 binding domains. Furthermore, the conjugate may comprise one first or second specific binding molecule, or it may comprise two or more first and/or second specific binding molecules, e.g. 2-6, or 2-4 first and/or second specific binding molecules. In the case where a specific binding molecule has a valency of more than one, in an embodiment the conjugate may comprise one specific binding molecule, e.g. one first specific binding molecule with a valency of 2 or more, for example 2. In a case where a specific binding molecule has a valency of one, in an embodiment the conjugate may comprise two or more specific binding molecules, e.g. two or more monovalent second specific binding molecules, for example 2-4. It will be understood that in the case where there are multiple second specific binding partners they will be specific for the same target peptide. Where there are multiple first or second specific binding partners in the conjugate, each first specific binding molecule and each second specific binding molecule will be generally be the same, but can be different (e.g. each conjugate could contain a single, bivalent first specific binding molecule which binds CD40, and two, different, monovalent second specific binding molecules, each of which binds a different tag moiety, or each of which is directed towards the same tag moiety, but e.g. binds to a different site therein).

In one preferred embodiment the first specific binding molecule is bivalent. Further, in such an embodiment the second specific binding molecule may be monovalent and the conjugate may comprise two second specific binding molecules. Thus, in such an embodiment the conjugate is tetravalent. It will be appreciated from the discussion above that different forms of tetravalent conjugate are possible. It is possible for the valency of the conjugate to be increased, for example by conjugating more than one second specific binding molecule (e.g. 2, 3 or 4 or more) to one first specific binding molecule. Alternatively, or additionally, more than one first specific binding molecule may be conjugated.

The term "binding affinity" refers to the tendency of a binding molecule to bind, or not bind, its binding partner or target. Binding affinity may be quantified by determining the dissociation constant (Kd) for a binding partner. Similarly, the specificity of binding of a binding molecule to its target may be defined in terms of the comparative dissociation constants (Kd) of the binding molecule for its target compared to the dissociation constant with respect to the binding molecule and a non-target molecule. Typically the Kd of the specific binding molecule for its target will be at least 2-fold, preferably at least 5, 10, 15, 20, 30, 40, 50, 100 or 200-fold, less than its Kd with respect to another non-target molecule. Binding affinities and dissociation constants may readily be determined using well known methods. A number of such methods are described in WO 2015/091853. The first and second specific binding molecules may be capable of binding to their targets with an affinity that is at least 2, 5, 10, 50, 100 or 200-fold higher than their affinity for binding to another non-target molecule.

The first and second specific binding molecules may bind their respective targets with an affinity (K_{d}) of at least about 10, 5, 4, 3, 2 or 1 µM, or at least about 1000 nM (i.e. 10, 5, 4, 3, 2 or 1 µM, or 1000 nM or less), but may exhibit higher affinity, for example about 900, 800, 700, 600, 500, 400, 300, 200, 150, 100, 90, 80, 70, 60, 50, 40, 30, 25, 20, 15, 10, 7, 5, 4, 3, 2, 1, 0.5, 0.2, 0.1, 0.05. 0.01 nM or less. Affinity can be determined using for example ELISA, isothermal titration calorimetry (ITC), surface plasmon resonance (e.g. BIAcore) or fluorescent polarisation assay.

The strength of interaction between a specific binding molecule and its target may alternatively be quantified based on its off-rate (*k*_{off} or kd, dissociation rate constant, s⁻¹). Methods by which the off-rate of a receptor-ligand interaction may be calculated are well known in the art, e.g. surface plasmon resonance and stopped-flow analysis. The first and second specific binding molecules may bind their respective targets with an off-rate of 0.0015 s⁻¹ or less, e.g. 0.001 or 0.0005 s⁻¹ or less, or 10, 5×10⁻⁵ or 10⁻⁵ s⁻¹ or less. The off-rate of an interaction is directly related to its half-life (the half-life = 1/*k*_{off}). Thus for instance, an interaction with an off-rate of 0.0005 s⁻¹ has a half-life of 1/0.0005 s, i.e. 2000 seconds or 33 minutes.

The affinity constant (KD/Kd) for binding to CD40 may typically be in the range of 1-10nM. The association rate (ka) may typically be in the range of 0.4-3.4 x 10⁶ 1/M. The dissociation rate constant (kd) may typically be in the range of 1-10 x 10⁻³ 1/s.

The first specific binding molecule which binds CD40 may be, in one embodiment, an antigen-binding protein which comprises an antigen binding domain and a Fc region of an antibody. As noted above, it may in some cases, e.g. antigen-binding molecules comprising constant regions of a certain class or sub-type (e.g. IgG1), be beneficial for the binding molecule also to include a Fc region. This may, for example, enhance activation the CD40 positive cell, or it may be beneficial for stability or half-life of the molecule. However, this is not a necessary requirement.

In certain embodiments the first specific binding molecule is an intact antibody, and in particular a monoclonal antibody. In another embodiment it is a F(ab')₂ fragment of an antibody. As noted above, the antibody or fragment can be of any class or isotype. However, in a particular embodiment it is an IgG antibody, and more particularly an IgG1 or IgG2 antibody. In certain embodiments the IgG2 format is preferred. For example, it may be an intact IgG2 antibody or a F(ab')₂ fragment of an IgG2 antibody.

The first specific binding molecule may thus be an anti-CD40 antibody or antibody fragment or an antigen-binding protein derived or obtained therefrom. A wide range of anti CD40 antibodies have been described, and are available. As noted above anti-CD40 antibodies are described in WO 2016/023960, WO 2015/091853, US 2017/032159, US 2009/0074711, US 2017/0137528, US 2017/0342159 and WO 2014/070934, and any of these, or antibodies or antibody fragments or antibody constructs based or derived from these may be used. These documents present the CDR, and/or VH and VL sequences of various antibodies enabling the preparation of antigen binding proteins and conjugates comprising such sequence based on them. They also describe the preparation of various antibody variants, and human or humanised antibodies. Alternatively, an anti-CD40 antibody may be generated according to well-known methods.

As noted above, methods determining and screening for CD40 agonist activity of an anti-CD40 antibody are well known and available in the art, such as determining DC cell activation by determining the ability of an antibody to induce IL-12 production by bone marrow DC, e.g. by ELISA. In this way an agonistic first specific binding molecule may be identified and selected.

The antibody may be an antibody selected from CP-870,893, APX005M, ADC-1013, Chi Lob 7/4, SEA-CD40, ABS-1132/1133, ABS-1140/1135#and ABS-1150/1151. Any of these antibodies may be used as is, or they may be used as the basis for preparing antibody derivatives for use as the first specific binding molecule(s).

In particular, mention may be made of the following antibodies, which may be used as, or as the basis for (e.g. to derive fragments or other derivatives or constructs), the first specific binding molecule (unless otherwise indicated, CDR sequences presented here and throughout this document are as identified by IMGT):
(i) Antibody ABS-1150/1151 which is described in WO#2015/091853. The VH and VL sequences of this antibody are set out in SEQ ID NOs: 23 and 24 respectively (the corresponding DNA sequences are set out in SEQ ID NOs: 25 and 26 respectively). The sequences of VHCDRs 1, 2 and 3 respectively are set out in SEQ ID NOs: 17-19 and the sequences of VLCDRs 1, 2 and 3 respectively are set out in SEQ ID NOs: 20-22. ABS-1132/1133 and ABS-1140/1135 are both also described in WO#2015/091853, which provides the CDR sequences and complete VL and VH sequences of these antibodies.
(ii) Antibody CP-870,893 which is described in US 2017/0342159. The VH and VL sequences of this antibody are set out in SEQ ID NOs: 27 and 28 respectively (the corresponding DNA sequences are set out in SEQ ID NOs: 29 and 30 respectively). The sequences of VHCDRs 1, 2 and 3 respectively, as identified by IMGT, are set out in SEQ ID NOs: 64-66 and the sequences of VLCDRs 1, 2 and 3 respectively, as identified by IMGT, are set out in SEQ ID NOs: 67-69.
(iii) Antibody ADC-1013 which is described in WO#2016/023960. The VH and VL sequences of this antibody are set out in SEQ ID NOs: 37 and 38 respectively (the corresponding DNA sequences are set out in SEQ ID NOs: 39 and 40 respectively). The sequences of VHCDRs 1, 2 and 3 respectively (as identified by IMGT) are set out in SEQ ID NOs: 31-33 and the sequences of VLCDRs 1, 2 and 3 respectively (as identified by IMGT) are set out in SEQ ID NOs: 34-36. The sequences of VHCDRs 1, 2 and 3 respectively (as identified by Kabat) are set out in SEQ ID NOs: 138-140 and the sequences of VLCDRs 1, 2 and 3 respectively (as identified by Kabat) are set out in SEQ ID NOs: 141-143.
(iv) Antibody ChiLob 7/4 which is described in US 2009/0074711. The VH and VL amino acid sequences of this antibody are set out in SEQ ID NOs: 52 and 53, respectively.
(v) Antibody SEA-CD40 which is described in US 2017/0137528. The VH and VL amino acid sequences of this antibody are set out in SEQ ID NOs: 54 and 55, respectively.
(vi) Antibody APX005M which is described in WO 2014/070934. The VH and VL amino acid sequences of this antibody are set out in SEQ ID NOs: 56 and 57, respectively. The sequences of VHCDRs 1, 2 and 3 (as identified by IMGT) are set out in SEQ ID NOs: 58-60, respectively, and the sequences of VLCDRs 1, 2 and 3 (as identified by IMGT) are set out in SEQ ID NOs: 61-63, respectively. The sequences of VHCDRs 1, 2 and 3 (as identified by Kabat) are set out in SEQ ID NOs: 144-146, respectively, and the sequences of VLCDRs 1, 2 and 3 (as identified by IMGT) are set out in SEQ ID NOs: 147-149, respectively.

An antigen-binding protein may comprise one or more CDRs from the VH and/or VL of each respective antibody, or a variant thereof.

Thus, a first specific binding molecule of the conjugate may be an antigen-binding protein comprising at least one heavy chain variable region (VH) and at least one light chain variable region (VL), wherein:
(i)
   (a) the VH region comprises VHCDRs 1, 2 and 3 which comprise (or in an embodiment, consist of) the amino acid sequences set forth in SEQ ID NOs: 17, 18 and 19 respectively, and
   (b) the VL region comprises VLCDRs 1, 2 and 3 which comprise (or in an embodiment, consist of) the amino acid sequences set forth in SEQ ID NOs: 20, 21 and 22, respectively; or
(ii)
   (a) the VH region comprises VHCDRs 1, 2 and 3 which comprise (or in an embodiment, consist of) the amino acid sequences set forth in SEQ ID NOs: 31, 32 and 33, respectively; and
   (b) the VL region comprises VLCDRs 1, 2 and 3 which comprise (or in an embodiment, consist of) the amino acid sequences set forth in SEQ ID NOs: 34, 35 and 36, respectively; or
(iii)
   (a) the VH region comprises VHCDRs 1, 2 and 3 which comprise (or in an embodiment, consist of) the amino acid sequences set forth in SEQ ID NOs: 58, 59 and 60, respectively; and
   (b) the VL region comprises VLCDRs 1, 2 and 3 which comprise (or in an embodiment, consist of) the amino acid sequences set forth in SEQ ID NOs: 61, 62 and 63, respectively; or
(iv)
   (a) the VH region comprises VHCDRs 1, 2 and 3 which comprise (or in an embodiment, consist of) the amino acid sequences set forth in SEQ ID NOs: 64, 65 and 66, respectively, and
   (b) the VL region comprises VLCDRs 1, 2 and 3 which comprise (or in an embodiment, consist of) the amino acid sequences set forth in SEQ ID NOs: 67, 68 and 69, respectively; or
(v)
   (a) the VH region comprises VHCDRs 1, 2 and 3 which comprise (or in an embodiment, consist of) the amino acid sequences set forth in SEQ ID NOs: 138, 139 and 140, respectively, and
   (b) the VL region comprises VLCDRs 1, 2 and 3 which comprise (or in an embodiment, consist of) the amino acid sequences set forth in SEQ ID NOs: 141, 142 and 143, respectively; or
(vi)
   (a) the VH region comprises VHCDRs 1, 2 and 3 which comprise (or in an embodiment, consist of) the amino acid sequences set forth in SEQ ID NOs: 144, 145 and 146, respectively, and
   (b) the VL region comprises VLCDRs 1, 2 and 3 which comprise (or in an embodiment, consist of) the amino acid sequences set forth in SEQ ID NOs: 147, 148 and 149, respectively;
optionally wherein one or more of said CDR sequences other than VLCDR2 of SEQ ID NO.21 are modified by 1, 2, or 3 sequence modifications or wherein VLCDR2 of SEQ ID NO.21 is modified by 1 or 2 sequence modifications.

Thus, the CDR sequences may optionally be variant sequences comprising sequence modifications relative to the sequences set out in the SEQ ID NOs recited above. The sequence modification may be a substitution, addition or deletion of an amino acid, particularly a substitution or addition, and more particularly a substitution. The substitution may be a conservative substitution. In an embodiment the CDRs comprise 1 or 2, or 1 modification(s). Preferably, one or more of CDRs 1 and 2 in a given variable region are modified, and CDR3 is unmodified. In certain preferred embodiments the CDRs are not modified. The term "conservative amino acid substitution", as used herein, refers to an amino acid substitution in which one amino acid residue is replaced with another amino acid residue having a similar side chain. Amino acids with similar side chains tend to have similar properties, and thus a conservative substitution of an amino acid important for the structure or function of a polypeptide may be expected to affect polypeptide structure/function less than a non-conservative amino acid substitution at the same position. Families of amino acid residues having similar side chains have been defined in the art, including basic side chains (e.g. lysine, arginine, histidine), acidic side chains (e.g. aspartic acid, glutamic acid), uncharged polar side chains (e.g. asparagine, glutamine, serine, threonine, tyrosine), non-polar side chains (e.g. glycine, cysteine, alanine, valine, leucine, isoleucine, proline, phenylalanine, methionine, tryptophan) and aromatic side chains (e.g. tyrosine, phenylalanine, tryptophan, histidine). Thus a conservative amino acid substitution may be considered to be a substitution in which a particular amino acid residue is substituted for a different amino acid residue in the same family. However, a substitution of an amino acid residue in the CDR sequence may also be a non-conservative substitution, in which one amino acid residue is substituted for another with a side-chain belonging to a different family.

The variant CDRs are functionally equivalent to their respective corresponding native, unmodified CDRs. By functionally equivalent is meant that a protein or amino acid sequence (here the CDR) retains, or substantially retains, the function or activity of the protein or amino acid sequence (here the CDR) from which is it derived, or on which it is based (i.e. to which it corresponds). In particular, the functionally-equivalent variant may retain at least 70%, or more particularly at least 75, 80, 85, 90 or 95%, of the activity or function of the corresponding (unmodified) protein or amino acid sequence. In practice, this means that the variant CDR does not negatively affect, or does not substantially negatively affect, the function or activity, or properties of the antigen-binding protein in which it is present (compared to a native, or unmodified antigen binding protein (e.g. antibody), or compared to antigen binding protein in which the CDR regions are not modified). Principally, this means that the variant CDR does not affect the binding specificity of the antigen-binding protein, that is the protein retains the ability to bind specifically to CD40. Further, the binding affinity of the antigen binding protein is not substantially reduced compared to the native, or unmodified antigen binding protein, or a antigen binding protein with unmodified CDR regions. However, binding affinity of the antigen binding protein may be improved by modification of the CDR regions, particularly CDRs 1 and/or 2.

In another embodiment the antigen-binding protein comprises a VH region comprising an amino acid sequence as set out in any one of SEQ ID NOs: 23, 27, 37, 52, 54 or 56 or a sequence having at least 70 % sequence identity with SEQ ID NO: 23, 27, 37, 52, 54 or 56.

In another embodiment the antigen-binding protein comprises a VL region comprising an amino acid sequence as set out in any one of SEQ ID NOs: 24, 28, 38, 53, 55 or 57, or a sequence having at least 70 % sequence identity with SEQ ID NO: 24, 28, 38, 53, 55 or 57.

In another embodiment the antigen-binding protein comprises:
(a) a VH region comprising an amino acid sequence as set out in SEQ ID NO: 23 or a sequence having at least 70 % sequence identity with SEQ ID NO: 23, and a VL region comprising an amino acid sequence as set out in SEQ ID NO: 24 or a sequence having at least 70 % sequence identity with SEQ ID NO: 24; or#
(b) a VH region comprising an amino acid sequence as set out in SEQ ID NO: 27 or a sequence having at least 70% sequence identity with SEQ ID NO: 27, and a VL region comprising an amino acid sequence as set out in SEQ ID NO: 28, or a sequence having at least 70 % sequence identity with SEQ ID NO: 28; or#
(c) a VH region comprising an amino acid sequence as set out in SEQ ID NO: 37 or a sequence having at least 70 % sequence identity with SEQ ID NO: 37, and a VL region comprising an amino acid sequence as set out in SEQ ID NO: 38 or a sequence having at least 70 % sequence identity with SEQ ID NO: 38; or#
(d) a VH region comprising an amino acid sequence as set out in SEQ ID NO: 52 or a sequence having at least 70 % sequence identity with SEQ ID NO: 52, and a VL region comprising an amino acid sequence as set out in SEQ ID NO: 53 or a sequence having at least 70 % sequence identity with SEQ ID NO: 53; or
(e) a VH region comprising an amino acid sequence as set out in SEQ ID NO: 54 or a sequence having at least 70 % sequence identity with SEQ ID NO: 54, and a VL region comprising an amino acid sequence as set out in SEQ ID NO: 55 or a sequence having at least 70 % sequence identity with SEQ ID NO: 55; or
(f) a VH region comprising an amino acid sequence as set out in SEQ ID NO: 56 or a sequence having at least 70 % sequence identity with SEQ ID NO: 56, and a VL region comprising an amino acid sequence as set out in SEQ ID NO: 57 or a sequence having at least 70 % sequence identity with SEQ ID NO: 57.#

It will thus be seen that in such embodiments the antigen-binding protein may comprise VH and VL regions which comprise sequences which are variants of the VH and VL region sequences as set out in the SEQ ID NOs specified above, i.e. which comprise sequence modifications relative to the specified SEQ ID NOs, as long as they meet the requirement of at least 70 % sequence identity. Such sequence modifications may be one or more amino acid substitutions, additions or deletions. Sequence modifications are discussed above. In particular, amino acid substitutions may be conservative substitutions. The variant sequences may be functionally equivalent to their respective parent sequences, as defined and explained above. In certain embodiments, in such variant sequences, the CDR sequences are not modified. Thus, in certain embodiments the CDR sequences may be as set out in the CDR sequences given above.

The antigen-binding protein according to any embodiment described above may also contain constant regions. Thus the antigen-binding protein may contain human light chain regions comprising all or part of the κ and λ constant domain sequences as set out in SEQ ID NOs: 45 and 46, respectively, or a sequence having at least 70 % sequence identity therewith. More particularly the antigen binding protein may contain a Fc region. As noted above, the Fc region may be from an antibody of any class, and in particular it may be an IgG1, IgG2, IgG3 or IgG4 region.

Thus, the antigen binding protein may comprise a heavy chain constant region comprising a sequence as set forth in any one of SEQ ID NOs: 41, 42, 43, or 44, or a sequence having at least 70 % sequence identity therewith. In particular the first specific binding molecule may comprise a heavy chain constant region comprising a sequence as set forth in any one of SEQ ID NOs: 41 or 42 or a sequence having at least 70 % sequence identity therewith. The earlier discussions above regarding variant sequences, sequence modifications and functional equivalence apply also in this context. Thus, the constant region, or a domain thereof, may comprise one or more sequence modifications relative to a native sequence. Mutations are known, for example, which may alter or affect the properties of an antibody or antibody construct containing such a modified (mutated) constant region. One such mutant, IgG2 C127S, is described in Example 1 below. This mutant is locked in to the IgG2B conformation, which has been found to enhance the ability of IgG2 antibodies to initiate immune responses. The amino acid sequence of the IgG2 C127S variant is presented in SEQ ID NO: 15. As will be apparent from comparison of the sequences, the cysteine residue at position 14 of the native IgG2 constant sequence (set forth in SEQ ID NO: 42) is substituted for serine in the C127S variant (set forth in SEQ ID NO: 15).#

The antigen-binding proteins may thus comprise amino acid sequences which are variants of specified sequences having at least 70 % sequence identity. In particular embodiments the sequence identity can be at least 75, 80, 85, 90, 95, 96, 97, 98 or 99 %.

% sequence identity may be assessed by any convenient method. However, for determining the degree of identity between sequences, computer programs that make multiple alignments of sequences are useful, for instance Clustal W (Thompson, Higgins, Gibson, Nucleic Acids Res., 22:4673-4680, 1994). If desired, the Clustal W algorithm can be used together with BLOSUM 62 scoring matrix (Henikoff and Henikoff, Proc. Natl. Acad. Sci. USA, 89:10915-10919, 1992) and a gap opening penalty of 10 and gap extension penalty of 0.1, so that the highest order match is obtained between two sequences wherein at least 50 % of the total length of one of the sequences is involved in the alignment. Other methods that may be used to align sequences are the alignment method of Needleman and Wunsch (Needleman and Wunsch, J. Mol. Biol., 48:443, 1970) as revised by Smith and Waterman (Smith and Waterman, Adv. Appl. Math., 2:482, 1981) so that the highest order match is obtained between the two sequences and the number of identical amino acids is determined between the two sequences. Other methods to calculate the percentage identity between two amino acid sequences are generally art-recognised and include, for example, those described by Carillo and Lipton (Carillo and Lipton, SIAM J. Applied Math., 48:1073, 1988) and those described in Computational Molecular Biology, Lesk, e.d. Oxford University Press, New York, 1988, Biocomputing: Informatics and Genomics Projects.

Generally, computer programs will be employed for such calculations. Programs that compare and align pairs of sequences, like ALIGN (Myers and Miller, CABIOS, 4:11-17, 1988), FASTA (Pearson and Lipman, Proc. Natl. Acad. Sci. USA, 85:2444-2448, 1988; Pearson, Methods in Enzymology, 183:63-98, 1990) and gapped BLAST (Altschul et al., Nucleic Acids Res., 25:3389-3402, 1997), BLASTP, BLASTN, or GCG (Devereux, Haeberli, Smithies, Nucleic Acids Res., 12:387, 1984) are also useful for this purpose. Furthermore, the Dali server at the European Bioinformatics institute offers structure-based alignments of protein sequences (Holm, Trends in Biochemical Sciences, 20:478-480, 1995; Holm, J. Mol. Biol., 233:123-38, 1993; Holm, Nucleic Acid Res., 26:316-9, 1998).

Sequence identity is determined over the entire length of the unmodified (i.e. reference) amino acid (or nucleotide) sequence (i.e. a global sequence alignment is carried out).

By way of providing a reference point, sequences according to the present invention having 65 %, 70 %, 75 %, 80 %, 85 %, 90 %, 95 %, 96 %, 97 %, 98 % or 99 % sequence identity may be determined using the ALIGN program with default parameters (for instance available on Internet at the GENESTREAM network server, IGH, Montpellier, France).

The second specific binding molecule may be any type of antigen-binding protein set out above. However, in a certain particular embodiment it may be a synthetic antibody construct, and in particular a single chain antibody. In an embodiment the second specific binding molecule is a scFv.

The specificity of the second binding molecule will depend on the tag moiety, and, in accordance with the discussion above, the second binding molecule will have a binding affinity for the tag moiety. It is well within the routine skill of a person skilled in the art to prepare a binding molecule, including particularly an antigen-binding protein, which is specific for a given or selected tag moiety. Indeed, antibody-tag moiety pairs are known and available in the art, for example antibody-hapten pairs. Based on available antibody sequences (protein and DNA), the skilled person is readily able to prepare various antibody based constructs, including single chain antibodies, and scFv's. Further, once a desired tag moiety, e.g. tag peptide, has been identified and prepared it is a routine matter to generate a specific binder for it, or to screen libraries of binding molecules to identify an appropriate or suitable specific binding molecule for it. Techniques for generating and selecting antibodies are well known, as well as for determining their sequences and the sequences which encode them, and for preparing derivative antibody constructs based on antigen binding domains of antibodies. Example 1 below describes two scFv second binding molecules against peptide moieties (termed pTAG-scFv). Equivalent scFV second binding molecules directed against other tag peptides may be prepared..

Preferably, the second specific binding molecule binds to the tag moiety (and more particularly to the tag moiety when contained in a tag construct) with high affinity. It will be understood in this respect that the second binding molecule when present in the conjugate is able to bind the tag moiety to form a complex with a long half-life. By a long half-life is meant that the interaction has a *k*_{off} (kd) of 0.0015 s⁻¹ or less, preferably 0.0005 s⁻¹ or less (as detailed above).

In a particular embodiment the scFv binds the MTTE of SEQ ID NO: 6. Examples of scFvs which bind the MTTE include 14GIIICII-b (SEQ ID NO: 47) and 1BIIICI-b (SEQ ID NO: 48). The CDRs of the 14GIIICII-b scFv are set forth in SEQ ID NOs: 126-131. VHCDRs1, 2 and 3 have the amino acid sequences set forth in SEQ ID NOs: 128-128, respectively. VLCDRs1, 2 and 3 have the amino acid sequences set forth in SEQ ID NOs: 129-131, respectively. The CDRs of the 1BIIICI-b scFv are set forth in SEQ ID NOs: 132-137. VHCDRs1, 2 and 3 have the amino acid sequences set forth in SEQ ID NOs: 132-134, respectively. VLCDRs1, 2 and 3 have the amino acid sequences set forth in SEQ ID NOs: 135-137, respectively. In a particular embodiment the second specific binding molecule is an scFv comprising six CDRs having the amino acid sequences set forth in SEQ ID NOs: 126-131. In another embodiment, the second specific binding molecule is an scFv comprising six CDRs having the amino acid sequences set forth in SEQ ID NOs: 132-137. In yet another embodiment, the second specific binding molecule is an scFv comprising the amino acid sequence set forth in SEQ ID NO: 47 or the amino acid sequence set forth in SEQ ID NO: 48, or an amino acid sequence having at least 80, 90 or 95 % sequence identity thereto.

The second specific binding molecule may be covalently linked to a light or heavy chain of the first specific binding molecule. As noted above, the second specific binding molecule may be linked to the first specific binding molecule at either end of the molecule, or at either end of a constituent part (e.g. polypeptide or chain) thereof, for example at a C- or N-terminal end thereof. In a particular embodiment the second specific binding molecule is linked to the C-terminal end of a heavy or light chain of a first antigen binding protein. Accordingly, the second specific binding molecule may be linked to the constant region of the light chain (CL) or to a constant domain of the constant region of the heavy chain (a CH domain), for example the CH3 domain. However, it is possible for the second binding molecule (e.g. an scFv) to be attached to the N-terminal end of the heavy or light chain.

In an embodiment the first specific binding molecule may be an intact antibody, e.g. an IgG antibody, and a second specific binding molecule may be linked to a VH or VL, or more preferably to a CL or CH3 domain thereof. More particularly, a second specific binding molecule may be linked to the VH, VL, CL or CH3 domain of each chain. More particularly still, each of two second specific binding molecules may be linked to the same (in the sense of same type of) domain in each of the two chains. However, in other embodiments second specific binding molecules may be linked to different domains (i.e. different types of domain) in the two chains, e.g. one second binding molecule may be linked to a CL domain and one second binding molecule may be linked to CH3 domain, in the same or another chain. In an embodiment, a second binding molecule may be linked to the CL and CH3 domain of each chain (i.e. the conjugate may comprise 4 second binding molecules).

In one preferred embodiment, the conjugate comprises one first specific binding molecule, which is an antibody, and two second specific binding molecules, which are scFvs, wherein:
i) one scFv is conjugated to the C_{H}3 domain of each heavy chain of said antibody; or
ii) one scFv is conjugated to the C_{L} domain of each light chain of said antibody. A conjugate structure of (i) is depicted in Figure 6.

Disclosed herein is also a nucleic acid molecule that comprises a nucleotide sequence encoding a conjugate as defined herein comprising a first and a second specific binding molecule, wherein said first and second binding molecule are each specific binding proteins. More particularly, the first and second binding molecules are antigenic binding proteins, or a chain thereof, as disclosed herein. Thus, the nucleotide sequence may encode a polypeptide comprising a VH region or a VL region of a first antigen-binding protein (e.g. antibody) specific for CD40 and a scFv specific for a tag moiety. More particularly the polypeptide may comprise a heavy chain or a light chain of an anti-CD40 antibody and a scFv specific for a tag moiety. The scFv may be linked directly or indirectly (i.e. via a linker amino acid sequence) to the N- ,or more preferably C-, terminal of the light or heavy chain. The nucleic acid molecule may be DNA or RNA. In particular, the nucleic acid molecule may comprise a nucleotide sequence which encodes a polypeptide comprising a VH or VL region as defined herein, or more particularly a polypeptide comprising a heavy chain comprising a VH region as defined herein or a light chain comprising a VL region as defined herein. For example the nucleic acid molecule may comprise a nucleotide sequence as set forth in SEQ ID NOs: 25, 26, 29, 30, 39 or 40 or a sequence having at least 70 % sequence identity to any aforesaid sequence (which encode, respectively, the VH and VL of antibody ABS-1150, the VH and VL of antibody CP-870,893, and the VH and VL of antibody ADC-1013). The sequence identity may be at least 75, 80, 85, 90, 95, 96, 97, 98 or 99 %.

Thus the nucleotide sequence may be a variant of one of the specific nucleotide sequences. For example, a variant may be a substitution, deletion or addition variant of any of the above nucleic acid sequences. A variant polynucleotide may comprise 1, 2, 3, 4, 5, up to 10, up to 20, up to 30, up to 40, up to 50, up to 75 or more nucleotide substitutions and/or deletions from the sequences given in the SEQ ID NOs. A variant nucleotide sequence may be a degenerate sequence. Methods of determining nucleotide sequence identity are well known in the art. Such identity is determined over the entire length of the unmodified (i.e. reference) nucleotide sequence.

Algorithms for measuring nucleotide sequence identity are known in the art. For example, the UWGCG Package provides the BESTFIT program which can be used to calculate identity (e.g. used on its default settings) (Devereux et al (1984) Nucleic Acids Research 12, p387-395). The PILEUP and BLAST algorithms can also be used to calculate identity or line up sequences (typically on their default settings), for example as described in Altschul S.F. (1993) J Mol Evol 36:290-300; Altschul, S, F et al (1990) J Mol Biol 215:403-10. Software for performing BLAST analysis is publicly available through the National Centre for Biotechnology Information (http://www.ncbi.nlm.nih.gov/). The BLAST program uses as defaults a word length (W) of 11 , the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

A conjugate of the invention may thus be produced from or delivered in the form of a polynucleotide which encodes, and is capable of expressing, it. Where the conjugate comprises two or more chains, a nucleic acid molecule may encode one or more chains. For example, a polynucleotide may encode a light chain, a heavy chain or both. Two nucleic acid molecules may be provided, one of which encodes a light chain and the other of which encodes the corresponding heavy chain. Such a nucleic acid molecule or pair of nucleic acid molecules may be expressed together such that a conjugate is generated.

The nucleic acid molecules can be synthesised according to methods well known in the art, as described by way of example in Sambrook et al. (1989, Molecular Cloning - a laboratory manual; Cold Spring Harbor Press).

The nucleic acid molecules may be provided in the form of an expression cassette which includes control sequences operably linked to the encoding nucleotide sequence, thus allowing for expression of the conjugate in vivo. These expression cassettes, in turn, are typically provided within vectors (e.g., plasmids or recombinant viral vectors). A suitable vector may be any vector which is capable of carrying a sufficient amount of genetic information, and allowing expression of a conjugate or chain thereof.

The present disclosure thus also includes a recombinant construct comprising a nucleic acid molecule as defined herein linked to a heterologous nucleic acid sequence.#By "heterologous" as used herein is meant a nucleic acid sequence which is not natively linked to the nucleic acid molecule described herein, i.e. which is not linked to the nucleic acid molecule described herein in nature.#n the construct, the nucleic acid molecule described herein may be flanked by restriction sites (i.e. nucleotide sequences recognised by one or more restriction enzymes) to enable easy cloning of the nucleic acid molecule of the invention. A "recombinant" construct is a nucleic acid construct synthesised using recombinant techniques, e.g. molecular cloning.

The term "linked" as used herein with respect to the construct may simply mean that the nucleic acid molecule is directly joined to a heterologous nucleic acid sequence. In the recombinant construct, the nucleic acid molecule disclosed herein may be operably linked to a heterologous expression control sequence.

Thus, also disclosed is an expression cassette comprising a nucleic acid molecule as defined herein, or a vector that comprises a nucleic acid molecule or a recombinant construct as defined herein. Expression vectors are routinely constructed in the art of molecular biology and may for example involve the use of plasmid DNA and appropriate initiators, promoters, enhancers, terminators and other elements, such as for example polyadenylation signals which may be necessary, and which are positioned in the correct orientation, in order to allow for expression of conjugate, or chain thereof.

Other suitable vectors would be apparent to persons skilled in the art, and include e.g. cloning vectors.

The disclosure also includes cells that have been modified to express a conjugate as defined herein, or a constituent polypeptide (e.g. chain) thereof. Thus, also disclosed is a cell comprising a nucleic acid molecule, recombinant construct, or vector as defined herein, that is a cell into which such a molecule, construct or vector has been introduced. The cell may be defined as a host cell, or as a production host cell.

Such cells include transient, or preferably stable, higher eukaryotic cell lines, such as mammalian cells or insect cells, lower eukaryotic cells, such as yeast or prokaryotic cells such as bacterial cells. Particular examples of cells include mammalian HEK293T, CHO, HeLa, NSO and COS cells. Preferably the cell line selected will be one which is not only stable, but also allows for mature glycosylation and cell surface expression of a polypeptide. Such cells may be cultured using routine methods to produce the conjugate.

The conjugate is used to bind to a tag moiety. The tag moiety, as noted above, is a tag peptide which consists of the amino acid sequence set forth in any one of SEQ ID NOs: 6 or 10-14, or a fragment of any one of SEQ ID NOs: 6 or 10-14 of at least 5 amino acids, as this can readily be designed or selected to minimise immunogenicity etc. As discussed above, a tag peptide is preferably a non-human peptide.

As described in Example 1 below, a peptide library may be screened to identify possible tag peptides. For example, a library of peptides from, or derived from, a non-human protein sequence, for example a bacterial protein, e.g. tetanus toxin (TTx), may be obtained and screened to identify peptides with suitable characteristics. Such characteristics may include, for example, good aqueous solubility, an α-helical structure and non-immunogenicity, particularly that the peptide is not associated with any endogenous immunity in a subject, most notably a human subject (that is to say, that the peptide does not contain any B-cell epitopes, in particular any universal human B-cell epitopes). Peptide libraries may be generated in various ways, including preparation of synthetic peptides. Screening may take place in silico using various available software programs, and/or by structural or other analysis. Absence of B-cell epitopes may be determined by screening for binding to sera from subjects.

Screening of peptides from a TTx peptide fragment library identified peptides of SEQ ID NOs: 10-13. SEQ ID NO.14 is a derivative of SEQ ID NO. 10 having capped ends, as described in Example 1. Any of these may be used, particularly SEQ ID NOs: 10, 13, and 14. Other peptide libraries may be screened in an analogous manner to that described in Example 1 to identify other suitable peptide tags.

As noted above, it may be advantageous for the tag moiety to contain a CD4 epitope, and peptides may be designed to contain, or may be screened for containing, such epitopes. It has in particular been identified that certain peptides contain so-called universal T-helper epitopes, and such an epitope may be included within a tag peptide. Universal epitopes have been identified in certain TTx peptides, for example the well known P2 and P30 T-helper epitopes, and in one embodiment the tag peptide may include one or both of these epitopes. In another embodiment, the tag peptide may not include a CD4 epitope.

As described above, the tag moiety is provided as part of a tag construct comprising the tag moiety covalently linked to an antigen. The linkage may be direct or indirect, i.e. the tag moiety may be linked to the antigen via a linker. The linker may be as described above. The tag construct comprises a tag peptide linked to an antigenic peptide, i.e. as a fusion polypeptide. The fusion may be direct or indirect, e.g. via a linker peptide. The tag peptide and antigen peptide may be linked in either order, i.e. the tag peptide may be at the N- or C-terminal end of the antigen peptide.# In another embodiment the antigen (e.g. tumour antigen) is embedded in the tag construct, i.e. there is a tag moiety both N- and C-terminal to the antigen. Thus the tag construct may comprise an antigen flanked by tag moieties, such that tag moieties are located at the N- and C-termini of the construct.

The conjugate may thus be used to bind, non-covalently, a tag construct comprising the tag moiety. In this way a complex may be formed comprising an antigen, non-covalently linked to the conjugate, by means of the bound tag construct. The complex thus comprises an antigen and a specific binding molecule for CD40. The tag construct is, as noted above, a synthetic, or artificial construct, or a biological construct produced outside the human body, or more broadly the body of a subject to whom the conjugate or complex is administered. Accordingly, the complex herein does not include a complex between a conjugate and a protein in or on a cell, for example *in situ* in or on a cell in the body of a subject or in or on an isolated cell or cell culture etc. Nor does it include a complex between a conjugate and a mammalian protein which is a native protein which may occur or be found in a mammalian subject, e.g. a protein which may be expressed on the surface of a cell. The complex is provided as an isolated complex.

As explained above, such a complex may be used to activate an APC (by binding to CD40 on the cell surface), whilst simultaneously delivering an antigen to the APC. Thus, whilst being activated, the APC is simultaneously induced to take up, process and present the antigen on MHC on its cell surface. The complex thus allows (i.e. enables or facilitates) simultaneous activation of the APC and antigen presentation by the APC. The APC may be any APC, for example as listed above, including notably a DC.

The APC activation using the complex may take place *in vitro, ex vivo,* or *in vivo.* Thus, APC may be contacted with the complex *in vitro* or *ex vivo,* or the complex (or its constituent parts) may be administered to a subject to activate APC *in vivo.* The complex may therefore have medical and non-medical uses, and all such uses are encompassed herein. For example, isolated or cultured APC may be contacted with the complex, e.g. in a laboratory setting, for example for research, development or testing purposes. This may be achieved by pre-mixing the conjugate and tag-construct, to form the complex, and then applying the complex to the APC. Alternatively, the conjugate and tag construct can be individually applied to the APC, such that the complex forms within the APC culture.

Further, as noted above, the complex may be used to activate a T-cell, and in particular a T-cell which expresses a TCR which recognises the antigen. Specifically the T-cell recognises the antigen when presented by the APC (i.e. in the context of MHC). Thus, an APC activated by the complex, or for activation by the complex, may be contacted with the T-cell. Thus for example, APC may be cultured or incubated in the presence of the complex (or constituent parts thereof), following which the APC may be contacted with the T-cells, e.g. co-cultured, or further incubated in the presence of the T-cells. Alternatively, the complex (or constituent parts thereof), APC and T-cells may be incubated or co-cultured together.

For use in therapy (including both therapeutic and prophylactic treatment), the complex may be administered to a subject, as described in more detail below. Alternatively, the conjugate and tag construct may be separately administered to the subject, such that the complex forms *in vivo,* as also discussed below. APC activated by the complex and presenting the antigen may activate T-cells *in vivo* which express a TCR which recognises the presented antigen.

The use of a complex according to the present disclosure and invention thus allows T-cells to be activated. This includes particularly effector T-cells and in particular, CD8+ cytotoxic T-cells. Such cytotoxic T-cells (CTL) may thus be primed to attack and destroy cells which are recognised by the T-cell, i.e. which express the antigen. As noted above the tag moiety part (or constituent) of the complex may comprise a CD4 epitope, and this may therefore act to activate CD4+ T-cells. Furthermore the antigen may include CD4 and/or CD8 epitopes. Thus, CD4+ cells may be activated. CD4+ cells activated in this way may augment an immune response, e.g. an immune response mediated by an endogenously activated CD8+ cell. In an embodiment both CD4+ and CD8+ cells may be activated by the complex. The use of the complex may allow T-cell activation, and the T-cell response against its target cell, to be improved. In particular, T-cell activation and T-cell immune response may be improved as compared to use of a specific binding molecule for CD40 (i.e. the parental first specific binding molecule) alone (e.g. as compared to the same first specific binding molecule of the conjugate when used alone) or when used alone in combination with the antigen (i.e. as compared to using a mixture of the first parental specific binding molecule (as is used in the conjugate) together with the antigen which is used in the construct which is bound in the complex). Example 1 below demonstrates that the level of agonistic activity of a conjugate prepared according to the principles of the present invention may be increased compared to a parental antibody, and that at least the level of agonistic activity of a parental antibody can be retained by a conjugate. Agonistic activity in activating APC may be improved by altering the class of the antibody in the conjugate.

The antigen may be any antigen that it is desired to deliver to a CD40-bearing cell, and in particular to an APC, e.g. a DC. Thus the antigen may be any antigen that it is desired for an APC to present. This may thus be an antigen, which when presented by the APC, is recognised by a T-cell it is desired to activate. In the context of therapy it is desirable to activate T-cells which recognise an antigen associated with a disease or condition it is desired to treat or prevent. Thus, the antigen may be an antigen expressed by (e.g. on the surface of) a cell it is desired to ablate, or more particularly to target for ablation. The antigen may thus be a cancer antigen or an antigen associated with an infection, e.g. an antigen of, or derived from, a pathogen. The target cell for the T-cell may thus be a cancer cell, or a cell infected by a pathogen. The pathogen may be a virus, or an intracellular pathogen.

The antigen may accordingly be a peptide. Thus, the antigen may comprise, or consist of, a peptide containing one or more antigenic epitopes. The epitopes may be CD4+ and/or CD8+ T-cell epitopes. The antigen may be a protein or polypeptide expressed by a cancer cell or pathogen, or a part thereof. In particular, the antigen may be a neo-antigen expressed by a cancer cell, that is an antigen generated by a somatic mutation in a cancer cell, which is not expressed by non-cancer cell. Thus, an antigenic peptide may comprise one or more neo-epitopes. Such neo-epitopes may for example be generated by frameshift mutations. Such mutations may occur in cancers which display microsatellite instability (MSI). Various neo-antigens and neo-epitopes are known and are described in the literature, in the context of various different cancers. Alternatively or additionally, the antigenic peptide may be, or may comprise one or more epitopes from, a tumour-associated antigen or an antigen from an oncovirus. Again, tumour-associated antigens are well known in the art and widely described in the literature, including for example, cancer testis antigens and hTERT antigens. Viral antigens are also well known and described. A comprehensive list of known, validated cancer antigens (both tumour-specific neo-epitopes and tumour-associated antigens) is publicly available online: the "Cancer Antigenic Peptide Database" is accessible at https://caped.icp.ucl.ac.be/Peptide/list. An extensive list of tumour antigens is also provided in Wang & Wang, Cell Research 27: 11-37, 2017.

The antigen may be a naturally-occurring peptide molecule or a fragment or part of a naturally-occurring protein. It may alternatively be a synthetic peptide, for example a peptide designed and prepared to contain one or more different epitopes, for example epitopes which do not occur naturally together. Such a synthetic peptide may comprise two or more epitopes linked together directly, or indirectly by linker, or spacer, sequences. The synthesis of such synthetic epitope-containing peptides (e.g. synthetic long peptides, SLPs) is known in the art, and known SLP peptides may be used.

In another aspect, the present invention provides compositions and formulations comprising a conjugate or a complex as defined and described herein. In an embodiment, such a formulation or composition may comprise a conjugate together with a tag construct. The conjugate and tag construct may be formulated or provided separately, or together in a single composition. Thus, the invention provides a pharmaceutical composition comprising a complex as defined herein, together with a pharmaceutically-acceptable carrier or excipient. Similarly, the invention provides a pharmaceutical composition comprising a conjugate as defined herein and at least one pharmaceutically-acceptable carrier or excipient, and a pharmaceutical composition comprising a tag construct as defined herein and at least one pharmaceutically-acceptable carrier or excipient. Also provided are kits, or combination products, as defined above, comprising, separately, a conjugate and a tag construct as defined herein. In such kits and products the conjugate and tag construct may be separately provided in compositions containing a pharmaceutically acceptable carrier or excipient.

As used herein, "pharmaceutically acceptable carrier or excipient" includes any and all solvents, dispersion media, coatings, antibacterial and antifungal agents, isotonic and absorption delaying agents, and the like that are physiologically compatible.

Preferably, the carrier or excipient is suitable for parenteral, e.g. intradermal, intravenous, intramuscular or subcutaneous administration (e.g. by injection or infusion). Depending on the route of administration, the complex, or constituent component thereof, may be coated in a material to protect the complex or component from the action of acids and other natural conditions that may inactivate or denature it.

Preferred pharmaceutically-acceptable carriers comprise aqueous carriers or diluents. Examples of suitable aqueous carriers that may be employed in the pharmaceutical compositions, kits and products include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. In many cases, it will be preferable to include isotonic agents, for example, sugars, polyalcohols such as mannitol, sorbitol, or sodium chloride.

The pharmaceutical composition, product or kit also may include a pharmaceutically-acceptable anti-oxidant. They may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol sorbic acid, and the like. It may also be desirable to include isotonic agents, such as sugars, sodium chloride, and the like. In addition, prolonged absorption of the injectable pharmaceutical form may be brought about by the inclusion of agents which delay absorption such as aluminum monostearate and gelatin.

Therapeutic compositions typically must be sterile and stable under the conditions of manufacture and storage. The composition can be formulated as a solution, microemulsion, liposome, or other ordered structure suitable to high drug concentration.

Sterile injectable solutions can be prepared by incorporating the active agent (e.g. complex) in the required amount in an appropriate solvent with one or a combination of ingredients enumerated above, as required, followed by sterilization microfiltration. Generally, dispersions are prepared by incorporating the active agent into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active agent plus any additional desired ingredient from a previously sterile- filtered solution thereof.

Pharmaceutical compositions, products and kits may comprise additional active ingredients as well as a complex, or component thereof, for example comprise additional therapeutic or prophylactic agents. Thus, the complex may be used as a monotherapy or as part of a combination therapy, e.g. in the treatment of cancer.

A kit or combination product as described herein may additionally contain instructions for use.

The complex as defined herein, or a combination product comprising the conjugate and tag construct as defined herein may be used in therapy. The combination product of the invention comprises a conjugate as defined herein and a tag construct as defined herein as a combined preparation for simultaneous or sequential use in therapy. That is to say, when the combination product disclosed herein is used according to the invention, i.e. in therapy, the conjugate and tag construct are administered simultaneously or sequentially to the subject. By "simultaneous" administration, as used herein, means that the two components are administered to the subject at the same time, or at least substantially the same time, by the same administrative route and at substantially the same location. By "sequential" administration, as used herein, is meant that the two components are administered to the subject at different times. In particular, administration of the first component is completed before administration of the second component commences.

Due to the nature of the present invention, sequential administration of the two components requires both (i.e. the conjugate and the tag construct) to be administered by the same route and at substantially the same location. Furthermore, although the administration of the conjugate and tag construct may be temporally spaced, the interval between the administrations should be such as to allow a complex to be formed, when both components have been administered. Thus, for example, both components may be administered within 1 hour of each other, or more particularly within 40, 30, 20, 15, 10, 8, 7, 6 or 5, 4, 3, 2, 1 or 0 minutes of each other.

Mention of the complex of the invention being administered to a subject for therapeutic purposes is to be understood as referring to a pre-mixed composition (e.g. solution) comprising both components of the complex (i.e. a conjugate of the invention and a tag construct), which components may exist in a dynamic equilibirium comprising the complex and its two individual components.

The therapy may be therapeutic or prophylactic. The complex (and combination product) may thus be used as a therapeutic or prophylactic vaccine. In therapeutic applications, complexes or compositions are administered to a subject already suffering from a disorder or condition, in an amount sufficient to cure, alleviate or partially arrest the condition or one or more of its symptoms. Such therapeutic treatment may result in a decrease in severity of disease symptoms, or an increase in frequency or duration of symptom- free periods. An amount adequate to accomplish this is defined as "therapeutically effective amount". Effective amounts for a given purpose will depend on the severity of the disease or condition as well as the weight and general state of the subject.

Prophylactic treatment may include the prevention of a condition, or a delay in the development or onset of a condition. For example the complex may be used to prevent an infection, or to reduce the extent to which an infection may develop, or to prevent, delay or reduce the extent of a cancer developing, or recurring, or for example to prevent or reduce the extent of metastasis.

As used herein, the term "subject" includes any human or non-human animal subject. The subject may in particular be a mammalian subject. In particular, the subject may be a human, or a laboratory, livestock, domestic, sport or zoo animal etc. It may thus be a primate or rodent. In a preferred embodiment the subject is a human. As noted above, the present invention may be used in veterinary medicine, in which case the subject is a non-human mammal. In a particular embodiment the subject may be a dog, in which case a canine conjugate of the invention may be used, as described above.

In particular, the complex (and combination product) may be useful in the treatment or prevention of cancer. By cancer is meant any malignant or pre-malignant neoplastic condition. The cancer may be thus be any cancer, of any organ, tissue or cell type. Cancers which present as solid tumours, and which do not exhibit solid tumours are included. Accordingly, haemopoietic cancers are included.

The cancer may be prostate cancer, breast cancer, colorectal cancer, pancreatic cancer, ovarian cancer, lung cancer, cervical cancer, rhabdomyosarcoma, neuroblastoma, multiple myeloma, leukemia, acute lymphoblastic leukemia, melanoma, bladder cancer, head and neck cancer, lymphoma, glioblastoma, or skin cancer. It may also be adrenal cancer, bone cancer, brain cancer, oespophageal cancer, eye cancer, gastric cancer, oral cancer, penile cancer, testicular cancer, thyroid cancer, uterine cancer, and vaginal cancer. Mast cell tumours and hemangiosarcoma may also be treated according to the present invention. In the case of canine therapy, the invention may be used for treatment of canine transmissible venereal tumour (CTVT) or canine melanoma or other canine malignancies.

The cancer may be newly diagnosed and naïve to treatment or it may be relapsed or refractory, or relapsed and refractory, primary or metastatic.

As explained above, the CD40 binder which is included in the conjugate, and hence the complex, activates the immune system by agonising CD40. In particular T-cells may be activated. The subsequent immune response exerts an anti-cancer effect on neighbouring or accessible tumour cells, without regard to CD40 expression by the tumour. The complex may therefore be effective against both CD40-positive and CD40-negative cancers,
In addition to the agonistic immune activating effect provided by the CD40 binder, the complex also provides an antigen, which may be processed and presented to T-cells which are activated, and thus T-cells may be primed to target cancer cells which express the antigen.

This may be of particular benefit in circumstances where cancer antigen presence is low or reduced, for example where a tumour has been surgically removed, or in cases where an anti-CD40 therapeutic cannot be delivered intra-tumourally. The complex provides a means for providing cancer antigen in the vicinity of the activation agonistic signal.

The cancer antigen may be selected based on the subject and the particular cancer, thus allowing personalised medicine. For example, the cancer of the subject may be subjected to genetic profiling, allowing a suitable antigen to be selected. A bank or library of antigens, or tag constructs comprising antigens, may be provided from which a suitable tag construct may be prepared or selected depending on the cancer type of the subject.

The complex (and combination product) may also be useful in the treatment or prevention of an infection. The present invention may in particular be used in therapy for viral infections, such as infections caused by Epstein-Barr virus (EBV), cytomegalovirus (CMV), human herpes viruses (e.g. HHV-6), parvovirus B19, human papillomavirus (HPV) and Ross River virus, though any viral infection can, in principle, be treated according to the present invention. Intracellular bacterial infections may also be treated according to the present invention, e.g. Brucellosis (caused by bacterial species of the genus *Brucella*)*,* Q fever (caused by *Coxiella bumetii*), diseases caused by species of Chlamydiae, such as chlamydia (caused by *Chlamydia trachomatis*) and pneumonia (caused by *Chlamydia pneumoniae*)*,* leprosy (caused by *Mycobacterium leprae* and *Mycobacterium lepromatosis*) and tuberculosis, including disseminated tuberculosis (caused by *Mycobacterium tuberculosis*)*.* Intracellular fungal or protozoal infections may also be treated by the current invention, including leishmaniasis (caused by trypanosomes of the genus *Leishmania*) and toxoplasmosis (caused by the apicomplexan *Toxoplasma gondii*)*.*

The antigen (e.g. cancer antigen or pathogen-derived antigen) may be selected to be recognised by a particular subset of T-cells in the subject to be treated, the T-cells expressing a TCR known to recognise the chosen antigen. In particular, the antigen may be selected on the basis that it is recognised by T-cells used in adoptive cell therapy in the subject to be treated.

For instance, in adoptive cell therapy, T-cells may be obtained from the subject, and T-cells which recognise an antigen of interest isolated. The isolated T-cells may then be expanded and/or otherwise treated to stimulate their effector functionality, and then re-infused into the subject to be treated. In this context, the antigen recognised by the re-infused T-cells may be used in the tag construct. A complex of the invention may then be administered to the subject, so that the antigen activates the re-infused T-cells.

Alternatively, T-cells may be obtained from the subject to be treated or a donor, and genetically modified to express a TCR which recognises a target antigen. The genetically modified T-cells may then be expanded and/or otherwise treated to stimulate their effector functionality, and then infused (or re-infused) into the subject to be treated. In this context, the antigen recognised by the genetically modified T-cells may be used in the tag construct. A complex of the invention may then be administered to the subject, so that the antigen activates the infused T-cells. Methods in which administration of a complex of the invention is combined with adoptive cell therapy are particularly useful in the treatment of cancer, in which case the antigen used in the tag construct is a cancer antigen.

Accordingly, disclosed is a method of treating cancer in a subject, the method comprising:
(i) obtaining T-cells from the subject;
(ii) isolating T-cells which recognise a target cancer antigen, and optionally expanding the isolated T-cells;
(iii) re-infusing the isolated T-cells into the subject; and
(iv) administering to the subject a complex of the present invention, wherein the tag construct comprises the target cancer antigen. Equivalently, in step (iv) the subject could alternatively be separately administered a conjugate of the invention and a tag construct.

Also disclosed is a method of treating cancer in a subject, the method comprising:
(i) obtaining T-cells from the subject or a donor;
(ii) genetically modifying the T-cells to express a TCR which recognises a target cancer antigen, and optionally expanding the T-cells before or after genetic modification;
(iii) infusing the genetically modified T-cells into the subject; and
(iv) administering to the subject a complex of the present invention, wherein the tag construct comprises the target cancer antigen. Equivalently, in step (iv) the subject could alternatively be separately administered a conjugate of the invention and a tag construct.

As noted above, the tag moiety may comprise a human B-cell epitope (at least in certain individuals), or antibodies against the tag moiety may develop over time during exposure, but the tag moiety may be bound more strongly by the second specific binding molecule than endogenous circulating antibodies in the subject to be treated. In an embodiment, tag moieties bound by endogenous circulating antibodies in the subject to be treated may be analysed to compare the strength of their interactions with the endogenous circulating antibodies of the subject to be treated with their interactions with one or more second specific binding molecules according to the invention. A tag moiety which binds a second specific binding molecule according to the invention more strongly than the endogenous circulating antibodies is selected. The subject is then administered a combination of the selected tag moiety and a conjugate comprising the relevant second specific binding molecule which binds the tag moiety.

A tag moiety comprising a B-cell epitope may be administered to a subject to be treated in combination with a conjugate comprising a second specific binding molecule which binds the tag moiety with high affinity, i.e. a low K_{d}, which does not allow endogenous antibodies to outcompete the conjugate and tag interaction after administration of the combination. Use of a second specific binding molecule which binds the tag moiety with high affinity may avoid the second specific binding molecule being outcompeted for binding of the tag by the subject's endogenous antibodies, or exchanged for such an endogenous antibody.

Above, mention of therapy utilising a combination of a conjugate according to the invention and tag construct includes both co-administration of the components in the form of a complex according to the invention, and separate administration of the two components.

Thus, also disclosed is a method of treating or preventing cancer or an infection in a subject, the method comprising:
i) identifying a tag moiety and a second specific binding molecule which interact with high affinity;
ii) selecting a conjugate of the invention which comprises the second specific binding molecule of (i) and a tag construct which comprises the tag moiety of (i) and an antigen; and
iii) administering a combination of the tag construct of (ii) and the conjugate of (ii) to the subject.

This method can be seen as utilising a conjugate which comprises a second specific binding molecule which binds a tag moiety with higher affinity than the tag moiety is bound by the subject's endogenous antibodies.

As noted above, the complexes of the invention, or conjugates and tag constructs, may be used as a monotherapy, or in conjunction with other therapeutic agents. Thus, in the treatment of cancer the other therapeutic agent may be an anticancer agent, such as a chemotherapeutic agent, numerous classes of which are known in the art, or an immunological agent, including for example, interferons, immune checkpoint inhibitors (e.g. anti-PD-1, -PD-L1 or -CTLA4 antibodies) and other immune-enhancing agents (e.g. anti-OX40 agonistic antibodies). Other therapeutic agents may be beneficial in the treatment of cancer or indeed an infection, e.g. anti-proliferative or anti-inflammatory cytokines, and anti-proliferative, immunomodulatory or factors influencing blood clotting, or inhibitors of angiogenesis. For treatment of an infection, the other (or second) therapeutic agent may be an antimicrobial agent, e.g. an antibiotic, anti-fungal or anti-viral agent.

The complex, or a composition comprising the complex, or the conjugate and tag construct components thereof, may be administered via one or more routes of administration using one or more of a variety of methods known in the art. Similarly, the conjugate and tag construct may be individually administered by these same methods. As will be appreciated by the skilled artisan, the route and/or mode of administration will vary depending upon the desired results. Preferred routes of administration include intravenous, intramuscular, intradermal, intraperitoneal, subcutaneous, spinal or other parenteral routes of administration, for example by injection or infusion, e.g. directly to the site of a tumour. The phrase "parenteral administration" as used herein means modes of administration other than enteral and topical administration, usually by injection. Alternatively, a non-parenteral route may be used, such as a topical, epidermal or mucosal route of administration. Local administration is preferred, including peritumoral, juxtatumoral, intratumoral, intralesional, perilesional, intra cavity infusion, intravesicle administration, and inhalation. However, the complex or composition may also be administered systemically.

In embodiments where the conjugate and tag construct are administered individually, i.e. they are not first pre-mixed to form the complex, the conjugate and tag construct must be administered via the same route. Preferably, they are both administered locally, e.g. intradermally, at the same (or substantially the same) site, such that the two components mix, and thus combine to form the complex, rapidly after administration. In these embodiments, the two components must be administered to the subject either simultaneously or rapidly one after the other, avoiding delay between administration of the first component and administration of the second component. This ensures the second component is administered, and complex formation is enabled, before the first component degrades or becomes excessively disseminated from the administration site.

A suitable dosage of an antibody of the invention may be determined by a skilled medical practitioner. Actual dosage levels of the active ingredients in the pharmaceutical compositions and products of the present invention may be varied so as to obtain an amount of the active ingredient which is effective to achieve the desired therapeutic response for a particular subject, i.e. patient, composition, and mode of administration, without being toxic to the patient. The selected dosage level will depend upon a variety of pharmacokinetic factors including the activity of the particular complex/conjugate employed, the route of administration, the time of administration, the rate of excretion of the complex, the duration of the treatment, other drugs, compounds and/or materials used in combination with the particular compositions employed, the age, sex, weight, condition, general health and prior medical history of the patient being treated, and like factors well known in the medical arts.

A suitable dose of a complex (or conjugate) of the invention may be, for example, in the range of from about 0.1 µg/kg to about 100 mg/kg body weight of the patient to be treated. For example, a suitable dosage may be from about 0.1 µg/kg to about 10 mg/kg body weight per day or from about 10 µg/kg to about 5 mg/kg body weight per day.

Dosage regimens may be adjusted to provide the optimum desired response (e.g. a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

The complexes/conjugates and tag constructs may be administered in a single dose or in multiple doses. The multiple doses may be administered via the same or different routes and to the same or different locations. Alternatively, complexes can be administered as a sustained release formulation, in which case less frequent administration is required. Dosage and frequency may vary depending on the half-life of the antibody in the patient and the duration of treatment that is desired. The dosage and frequency of administration can also vary depending on whether the treatment is prophylactic or therapeutic. In prophylactic applications, a relatively low dosage may be administered at relatively infrequent intervals over a long period of time. In therapeutic applications, a relatively high dosage may be administered, for example until the patient shows partial or complete amelioration of symptoms of disease. In an exemplary dosage regime, the complex (or combination of the conjugate and tag construct) is administered to the subject once a week, once a fortnight or once every three weeks, in a cycle repeated from 2 to 10 times.

Combined administration of two or more agents may be achieved in a number of different ways. In one embodiment, the complex and the other agent may be administered together in a single composition. In another embodiment, the complex and the other agent may be administered in separate compositions as part of a combined therapy. For example, the complex may be administered before, after or concurrently with the other agent. The complex of the invention may be administered in combination with or sequentially to tumour targeting antibodies, target therapy, pathway inhibitors or other immunomodulatory antibodies targeting eg. PD-1, PD-LI, CD 137, GITR, OX40, CTLA-4, CD27, HVEM, LtBR, and LAG3. Further the complex may also be combined with local radiation. Similarly, such additional therapies may be co-administered when the conjugate and tag construct are individually administered to the subject.

The invention is further illustrated by the following example, which should not be construed as limiting.

### Example 1

### Materials and Methods

### Cells

B3Z (Karttunen et al., PNAS 89(13): 6020-6024, 1992), a murine T-cell hybridoma expressing a TCR which recognises the ovalbumin peptide OVA₂₅₇₋₂₆₄ (SIINFEKL, SEQ ID NO: 1) in the context of the murine Class I MHC H-2Kb, was used to assess peptide loading and subsequent antigen presentation. B3Z cells express β-galactosidase under the control of the IL-2 promoter and thus, upon T-cell activation and proliferation, the enzyme will be expressed. β-galactosidase is able to hydrolyse the substrate chlorophenol red-β-D-galactopyranoside (CPRG), which leads to a colour change with a magnitude corresponding to the level of B3Z T-cell activation. Hence B3Z activation may be detected and measured by spectrophotometry.

Pmel-1 mice (Jackson Laboratory (USA), mouse strain 005023, described further below) are transgenic mice with T-cells which express a TCR specific for the murine gp100₍₂₅₋₃₃₎ peptide (SEQ ID NO: 2, amino acids 25-33 of the melanoma antigen gp100) in the context of the murine Class I MHC H-2Db, and which also recognises the corresponding human gp100 sequence in the context of H-2Db. Pmel-1 T-cells were isolated by harvesting spleens and inguinal lymph nodes from adult Pmel-1 mice. These organs minced by grinding them against a 70 µm cell strainer, and subsequent cells were passed through the cell strainer to achieve a single cell suspension. T-cells were isolated, and the isolated T-cells used for adoptive T-cell transfer experiments.

### Antibodies and Peptides

Anti-CD40 antibodies were either manufactured by the Drug Discovery and Development (DDD) Platform, SciLifeLab (Sweden), or custom-purchased from Absolute Antibody (UK), based on the available light and heavy chain sequences of CP-870,893 and ABS-1150/1151 (sometimes alternatively referred to herein as ABS-1150). Nine bispecific anti-CD40 antibodies and 6 parental agonistic human anti-CD40 antibodies were tested. Table 1 describes the antibodies used. The antibodies were expressed in HEK293 cells, and purified by affinity chromatography using protein A followed by preparative size exclusion chromatography (SEC). Endotoxin levels were <1 EU/mg as determined by LAL chromogenic endotoxin assay. Synthetic long peptides (SLPs) were custom-purchased from Capra Science (Sweden). Table 2 provides the amino acid sequences of the SLPs used.

### Mice

Adult C57BL/6 mice and male Pmel-1 transgenic mice were maintained at the animal facility at Uppsala Biomedical Center (BMC, Uppsala, Sweden). All animal experiments were approved by the Uppsala regional animal ethics committee.

### Bone Marrow Cell Isolation and Bone Marrow Dendritic Cell (BMDC) Differentiation

Bone marrow cells were isolated from the femora and tibiae of adult female tghCD40 mice (Mangsbo et al., Clin Cancer Res; 21(5); 1115-1126, 2015) under sterile conditions. BMDCs from tghCD40 express human CD40. After removal of soft tissue, the bones were disinfected in 70 % ethanol and the bone marrow then exposed by cutting open the bone epiphysis. Bone marrow cells were flushed out of the bone using IMDM medium, until the core of the bone became white. Cell clumps were passed through a 70 µm cell strainer to obtain a single cell suspension. The cells were washed by centrifugation and then frozen in FBS supplemented with 10 % DMSO at -160°C until the time of usage.

For BMDC differentiation, bone marrow precursor cells (isolated as described above) were thawed and cultured for eight days in non-tissue-culture treated (TCT) plates at a concentration of 2.5 × 10⁵ cells/ml in complete IMDM medium (i.e. IMDM medium supplemented with 10 % FBS, 1 % penicillin/streptomycin, 1 % HEPES and 0.5 % 2-mercaptoethanol), in the presence of 20 ng/ml mGM-CSF. Half of the medium was exchanged at day 3 and day 6 and replaced with fresh complete IMDM with 20 ng/ml mGM-CSF. On day 8, cells were harvested by gentle washing of the plate with pre-warmed IMDM medium, and flow cytometry was performed to detect DC differentiation markers (CD11b and CD11c) and activation markers (CD86 and MHC-II).

### BMDC Maturation and Activation Protocol

After 8 days of differentiation of bone marrow precursor cells, 1 × 10⁵ immature BMDC were plated per well in a 96-well TCT plate. Anti-CD40 antibodies (see Table 1) were added to each well at concentrations ranging from 500-0.0064 nM. BMDC were cultured for 48 hours in IMDM medium supplemented with 20 ng/mL mGM-CSF before the supernatant was collected and ELISA against IL-12 was performed to assess the agonistic stimulative capacity of the different antibodies. As a positive control, an IL-12 ELISA was performed on the supernatant of BMDCs cultured as above in the presence of 1 µg/ml LPS (instead of an antibody); as a negative control the ELISA was performed on IMDM medium. Tests were run in duplicate.

**Table 1**

| **Bispecific Antibodies** | | | | |
|---|---|---|---|---|
| | **Anti-CD40 IgG** | **Subclass** | **scFv** | **scFv Appending Position** |
| **X-SM083-bi-1** | CP-870,893 | IgG1 | 14GIIICII-b | CH3 |
| **X-SM083-bi-2** | CP-870,893 | IgG2 | 14GIIICII-b | CH3 |
| **X-SM083-bi-3** | CP-870,893 | IgG1 | 14GIIICII-b | CL |
| **X-SM083-bi-4** | CP-870,893 | IgG2 C1275 | 14GIIICII-b | CH3 |
| **X-SM083-bi-7** | CP-870,893 | IgG1 | IBIIICI-b | CL |
| **X-SM083-bi-9** | ABS-1150 | IgG1 | 14GIIICII-b | CH3 |
| **X-SM083-bi-10** | ABS-1150 | IgG2 | 14GIIICII-b | CH3 |
| **X-SM083-bi-11** | ABS-1150 | IgG1 | 14GIIICII-b | CL |
| **X-SM083-bi-12** | ABS-1150 | IgG2 C1275 | 14GIIICII-b | CH3 |
| **X-SM083-bi-17** | CP-870,893 | IgG2 | FITC-8 | CH3 |
| | | | | |

| **Parental Antibodies** | | | | |
|---|---|---|---|---|
| | **CD40 IgG** | Subclass | | |
| **X-SM083-ab-4** | CP-870,893 | IgG1 | | |
| **X-SM083-ab-5** | CP-870,893 | IgG2 | | |
| **X-SM083-ab-6** | CP-870,893 | IgG2 C127S | | |
| **X-SM083-ab-1** | ABS-1150 | IgG1 | | |
| **X-SM083-ab-2** | ABS-1150 | IgG2 | | |
| **X-SM083-ab-3** | ABS-1150 | IgG2 C127S | | |

By "scFv Appending Position" is meant the location on the parental antibody to which the scFv is attached. The appending is done via a peptide linker (SEQ ID NO: 49). The C127S mutant of IgG2 is locked into the IgG2B conformation, and has been found to enhance the ability of IgG2 antibodies to initiate immune responses (White et al., Cancer Cell 27: 138-148, 2015).
The VH and VL sequences for antibodies ABS-1150 and CP-870,893 are shown in SEQ ID NOs: 23 and 24, and 27 and 28 respectively. Human IgG1 and IgG2 constant domain and human κ and λ constant domain sequences are shown in SEQ ID NOs: 41, 42, 45, and 46 respectively. The sequences of the scFvs 14GIIICII-b, IBIIICI-b and FITC-8 are shown in SEQ ID NOs: 47, 48 and 70 respectively.

### In Vitro T-Cell Activation Assay

After 8 days of differentiation of bone marrow precursor cells, 25,000 immature BMDC were plated per well in a 96-well TCT plate. Mixtures comprising UU-05 or UU-10 SLP (Table 2), at a concentration of either 125 nM or 250 nM, in combination with one of various anti-CD40 antibodies as described above, were added to the immature BMDC culture. After 24 hours the supernatant containing the peptide/antibody mix was harvested and the wells were washed twice by centrifugation with pre-warmed IMDM with minimal disturbance to the BMDCs.

B3Z T-cells were added to the BMDCs and the co-culture was incubated for a further 24 hours, before the cells were lysed by addition of the lysis buffer (100 mM β-mercaptoethanol, 0.125 % IGEPAL CA-630, 9 mM MgCl₂, and 1.8 µg/ml CPRG). Cells were lysed to allow β-galactosidase to access the CPRG. The colour change was determined by reading the absorbance of the culture medium at 595 nm after 6 hours incubation in the lysis buffer.

**Table 2**

| **Peptide** | **Sequence** | **SEQ ID NO** |
|---|---|---|
| UU-05 | FIGITELKKLESKINKVFLEQLESIINFEKLAAAAAK | 3 |
| UU-10 | LEQLESIINFEKLAAAAAK | 4 |
| UU-30 | FIGITELKKLESKINKVFAVGALKVPRNQDWLGVPRQL | 5 |

### In Vivo Experimental Setup

Adult female C57BL/6 mice (18-20 g weight) were used for the *in vivo* antigen uptake model. Groups of C57BL/6 mice (6-10 mice per group) received 11 × 10⁶ splenocytes from Pmel-1 mice (i.v.) via tail vein injection and 1 × 10⁵ tghCD40 immature BMDC (right side footpad injection) on day 0. On day 1, different combinations of anti-CD40 antibodies together with the UU-30 peptide (containing the gp100₍₂₅₋₃₃₎ sequence, Table 2) were injected on the right side footpad to investigate the peptide uptake *in vivo.* After 72 hours, spleens and right side popliteal lymph nodes were harvested and passed through a 70 µm cell strainer to obtain single cell suspensions. Pmel-1 cell accumulation and activation was assessed in popliteal lymph nodes by flow cytometry staining for CD90.1 (a congenic marker not expressed on host T cells). Statistical analysis of results was performed using one-way ANOVA with Dunnett's multiple comparisons test.

### Flow Cytometry

The following fluorescent-labeled antibodies from Biolegend (USA) were used for flow cytometry (clone): CD11c (N418), CD11b (M1/70), CD90.1 (OX-7), ICOS (C398.4A), I-A/I-E (M5/114.15.2), CD86 (GL-1). The hCD40 (5C3) antibody from BD Biosciences (USA) was also used for flow cytometry. For flow cytometry staining, 1-5 × 10⁵ cells were pelleted in flow cytometry tubes/plates by centrifugation before the antibody staining cocktail was added to the cells. Cells were incubated at 4°C for 20 minutes then washed in PBS supplemented with 1 % BSA. Cells were run in a CytoFLEX Flow Cytometer (Beckman Coulter Life Science, USA).

### IL-12 ELISA

ELISA plates were coated with purified anti-mouse IL-12 antibody (clone C15.6, Biolegend) overnight at 4°C. Plates were blocked in PBS supplemented with 1 % BSA before being diluted to a suitable concentration for expected readout within the linear region. Culture supernatant was added to the ELISA plate. Thereafter, secondary biotinylated anti-mouse IL-12 (clone C17.8, Biolegend) was added, then Streptavidin/HRP (Code no. P0397, Dako/Agilent, USA) was added, and the plate was incubated for 1 hour at room temperature. Lastly, the ELISA reaction was developed by addition of TMB (34028, Sigma, USA) and the reaction then stopped with 1 M H₂SO₄. Absorbance was read at 450-570 nm.

### Data Analysis

Data from flow cytometry was analysed using FlowJo software (FlowJo, LLC, USA). GraphPad Prism 7 (GraphPad Software, USA) was used to plot the graphs and to perform statistical analysis.

### Tag Peptide Selection

The tetanus toxin peptide library (103 peptides, listed in Table 1 of WO 2011/115483) was used to screen for alpha helical peptides, with the following characteristics:
- Good solubility
- Preferably no endogenous antibody response to the tag sequence

To predict peptide structure, the following software was used: JPRED4 (http://www.compbio.dundee.ac.uk/jpred4; Drozdetskiy et al., Nucleic Acids Res 43(W1): W389-W394, 2015) and PASTA 2.0 (http://protein.bio.unipd.it/pasta2; Walsh et al., Nucleic Acids Res 42(W1): W301-W307, 2014). To predict solubility, the Innovagen peptide solubility calculator was used (https://pepcalc.com/peptide-solubility-calculator.php). The location of peptides of interest within the tetanus toxin protein was analysed using PDBsum (available through the European Bioinformatics Institute website (https://www.ebi.ac.uk/)), using the PDB ID 5n0b for the tetanus toxin crystal structure.

### Circular Dichroism (CD) Spectroscopy

To perform CD spectroscopy, peptides of interest were dissolved to a concentration of 0.2 mg/ml in 50 mM sodium phosphate, pH 6. A JASCO J-1500 CD spectrometer was used and before and between each analysis the cuvette was rinsed with water. Between each peptide Hellmanex 2 % was used to wash the cuvette. Buffer was used for baseline measurement.

### Tag Peptide ELISA

Biotinylated peptides were coated on streptavidin plates, and ELISA plates were then blocked with a BSA-containing solution. After blocking, the peptides were incubated with serum from a donor pre- and post-vaccination against diphtheria, tetanus and pertussis at different concentrations, and subsequently an anti-human IgG HRP antibody was used to detect human antibody binding to the peptide. The assay was developed using TMB, before H₂SO₄ was used to stop the reaction.

### Results

### In Vitro BMDC and T-Cell Activation

*In vitro* activation of BMDC was assessed using bispecific and parental monoclonal antibodies at the following range of concentrations: 0.0064 nM, 0.032 nM, 0.16 nM, 0.8 nM, 4 nM, 20 nM, 100 nM and 500 nM.

*In vitro* activation of BMDC and T-cells was tested using the 10 bispecific antibodies, and the corresponding 6 parental monoclonal antibodies, listed in Table 1. The results of this *in vitro* work are set forth in Table 3, below.

Transient production of the antibodies was performed in HEK293 cells and antibody preparations were confirmed endotoxin-low/free and with >95 % monomeric fractions. SPR and ELISA (single and double-target analysis) along with stability assessments were performed and all antibodies had affinities in the range of the parental antibodies and bound targets in accordance with these. The antibody constructs were functionally evaluated for agonistic properties and antigen loading capacity (as measured by T-cell activation), as described above. Agonistic properties were assessed by measuring the IL12p40 levels produced by BMDC of hCD40 transgenic mouse, by IL-12 ELISA, following incubation of the cells with the antibody constructs at the concentration ranges recited above. The amount of IL-12p40 produced by the BMDCs during incubation with each antibody construct is shown in **Figure 1****.** The level of agonistic activity displayed by each antibody construct (i.e. the level of DC activation caused by each antibody construct) is summarised in Table 3, based on the results presented in Figure 1.

**Table 3**

| | Agonism | T-Cell Activation |
|---|---|---|
| **X-SM083-bi-1** | ++++ | ++++ |
| **X-SM083-bi-2** | +++++ | +++ |
| **X-SM083-bi-3** | +++ | +++ |
| **X-SM083-bi-4** | +++++ | +++ |
| **X-SM083-bi-7** | ++++ | +++ |
| **X-SM083-bi-9** | ++ | ++ |
| **X-SM083-bi-10** | ++ | +++ |
| **X-SM083-bi-11** | +++ | ++ |
| **X-SM083-bi-12** | ++ | +++ |
| **X-SM083-bi-17** | ++ | not performed |
| **X-SM083-ab-4** | + | + |
| **X-SM083-ab-5** | ++ | + |
| **X-SM083-ab-6** | ++ | + |
| **X-SM083-ab-1** | -/+ | + |
| **X-SM083-ab-2** | ++ | + |
| **X-SM083-ab-3** | ++ | + |

As shown above, for bispecific constructs based on the CP-870,893 anti-CD40 antibody the level of agonistic activity displayed can depend on the attached scFv. Whereas certain scFvs were found to greatly enhance the agonistic capacity of the antibody constructs relative to the parental antibody (see e.g. Bi-1, Bi-2 and Bi-3, each of which comprise the 14GIIICII-b scFv, and Bi-7, which comprises the IBIIICI-b scFv), others mimic the activity of the parental antibody (see Bi-17, which comprises an anti-FITC scFv). The IgG2 parental antibody has a higher level of agonistic activity than the IgG1 parental antibody, and in the bispecific design the IgG2 isotype also displays higher activity than bispecific designs using the IgG1 isotype. Mutations which cause a rigid hinge in the IgG2 structure do not improve agonistic activity relative to the native IgG2 sequence (compare results with the native IgG2 isotype and the IgG2 C127S isotype). As also shown in Table 3, the effect of attaching an scFv to the ABS-1150 anti-CD40 antibody on its level of agonistic activity was much less pronounced.

In the T-cell activation assay, the BMDCs were incubated with a mixture containing the relevant antibody construct with the UU-05 peptide (SEQ ID NO: 3). UU-05 comprises at its N-terminus a minimal tetanus toxin epitope (MTTE) which functions as a peptide tag. MTTEs are short fragments of the tetanus toxin (TTx) α-chain, which comprise the minimum sequence necessary to include a β-cell epitope, and thus against which antibodies can be raised. The identification of MTTEs is described in WO 2011/115483. The MTTE used as a peptide tag in UU-05 has the amino acid sequence set forth in SEQ ID NO: 6: the MTTE of SEQ ID NO: 6 is a natural human B-cell epitope in humans (particularly humans vaccinated with TTd); notably, unless mice have been exposed to tetanus toxoid in a vaccination context, mice do not have antibodies against the MTTE of SEQ ID NO: 6 (i.e. in the present experimental setup the mice have no endogenous antibodies to MTTE as production of such antibodies was not induced). Immediately C-terminal to the MTTE is located an ovalbumin (OVA) fragment with the amino acid sequence set forth in SEQ ID NO: 7, which comprises the OVA epitope of SEQ ID NO: 1. The C-terminus of UU-05, immediately C-terminal to the ovalbumin fragment, has the amino acid sequence set forth in SEQ ID NO: 8.

After 24 hours the BMDCs were washed and B3Z T-cells added, as described above. As detailed above, the B3Z T-cells recognise the OVA peptide of SEQ ID NO: 1 when displayed in the H-2Kb murine MHC I. Upon recognition of their cognate peptide/MHC the T-cells are activated. Activation is quantified by spectrophotometry, as described above, based on the production by the cells of β-galactosidase, which is under the control of the IL-2 promotor (and thus is expressed when the cells are activated). As shown in Table 3, incubation of B3Z T-cells with BMDC previously incubated with antibody constructs and the antigenic peptide UU-05 resulted in T-cell activation. As controls, BMDCs were incubated with antibody mixtures without the antigenic peptide UU-05, but with UU-10 instead. Subsequent incubation of these BMDCs with B3Z T-cells did not give rise to T-cell activation.

To demonstrate that the tag peptide improves T-cell activation by improving loading of peptides, a BMDC/T-cell co-culture experiment was performed as above, in which BMDCs were incubated with the various antibody constructs (each at 10 nM) and either the UU-05 or UU-10 peptide, at a concentration of either 125 nM or 250 nM. The UU-10 peptide (SEQ ID NO: 4) comprises the same OVA fragment and C-terminus as UU-05, but lacks the N-terminal MTTE tag. This means that UU-05 is bound by the anti-MTTE scFvs in the bispecific constructs, but UU-10 is not, enabling assessment of the success of the inventors' "pull-in" strategy.

The results of the experiment (in terms of the levels of T-cell activation attained) are presented in **Figure 2****.** The figure illustrates that T-cell activation only takes place when the antigenic peptide comprises a tag that can interact with the scFv (i.e. is bound by the scFv) part of the bispecific conjugates, and not when the DCs are contacted with a simple mixture of antibody and antigen. Notably, all bispecific constructs, regardless of design, anti-CD40 antibody or scFv, display improved T-cell activation when applied to DCs with a tagged antigenic peptide, compared to application of the parental antibody version with the same antigenic peptide. The levels of T-cell activation set out in Table 3 are based on the data presented in Figure 2.

### In Vivo T-Cell Activation

Mice were administered Pmel-1 T-cells followed by anti-CD40 antibodies in combination with the antigenic peptide UU-30, as described above. UU-30 has the amino acid sequence set forth in SEQ ID NO: 5, and comprises at its N-terminus the MTTE sequence of SEQ ID NO: 6, immediately C-terminal of which is a fragment of human gp100 epitope with the amino acid sequence set forth in SEQ ID NO: 9. This gp100 fragment, in the context of the murine H-2Db Class I MHC, is recognised by Pmel-1 murine T-cells which express a TCR with specificity for the murine gp100(₂₅₋₃₃) fragment in the same MHC context. SEQ ID NO: 9 comprises the human gp100 epitope flanked by murine flanking peptides to make a synthetic long peptide that can be presented by murine APCs after processing.

*In vivo* experiments were performed using UU-30 in combination with X-SM083-bi-1 and X-SM083-bi-2 (defined in Table 1 above). Pmel-1 T-cells can be tracked in wild-type C57BL/6 mice, because they express the congenic marker Thy1.1 (also known as CD90.1). Following harvesting of the popliteal lymph node, and preparation of a single cell suspension (as described above), the suspension was stained and analysed by flow cytometry to measure proliferation of the Pmel-1 cells in the mice. Proliferation was determined as the accumulation of Thy1.1+ cells in the overall CD8+ T-cell population. The results are shown in **Figure 3****,** and demonstrate a statistically significant increase in Thy.1.1+ cells in mice administered both UU-30 and a bispecific antibody, relative to mice administered only the UU-30 peptide. No such increase was seen in mice administered a mixture of UU-30 and the corresponding parental antibody, demonstrating that attachment of an scFv to the parental antibody to allow binding of the antigenic peptide to the antibody enhances T-cell responses to the antigen.

### Tag Peptide Selection

Suitable tag peptides were identified by screening a library of fragments of the TTx protein to identify suitable tag sequences. The full-length TTx protein has the amino acid sequence set forth in SEQ ID NO: 16, UniProt entry P04958. Suitable tag peptides were desired to display the following characteristics: good solubility, an α-helical structure, and not to be associated with any endogenous immunity (i.e. the sequence should contain no B-cell epitopes). *In silico* analysis performed as described above identified four particular putative tag peptides: SM083P001 (SEQ ID NO: 10, corresponding to amino acids 609-630 of SEQ ID NO: 16); SM083P002 (SEQ ID NO: 11, corresponding to amino acids 81-101 of SEQ ID NO: 16); SM083P003 (SEQ ID NO: 12, corresponding to amino acids 285-309 of SEQ ID NO: 16); and SM083P004 (SEQ ID NO: 13, corresponding to amino acids 225-245 of SEQ ID NO: 16).

All four identified peptides are expected to have primarily α-helical secondary structures and to contain no β-sheets. A fifth putative tag peptide was also generated by capping the ends of SM083P001 with an Asp residue at the N-terminus and an Arg residue at the C-terminus, yielding the peptide SM083P005 (SEQ ID NO: 14). Capping of the ends was performed to attempt to improve stability of the peptide's α-helical structure. All putative tag peptides except SM083P004 were predicted to be highly soluble; SM083P004 was predicted to be relatively poorly soluble.

The peptides, with N-terminal biotinylation, were analysed by circular dichroism spectroscopy to check their secondary structure, the results of which are shown in **Figure 4****.** In addition to the 5 putative tag peptides described above, N-terminal biotinylated MTTE (SEQ ID NO: 6) was also analysed. Peptides SM083P004 and SM083P005 were found to display an α-helical secondary structure; the others were unstructured. The structures of the peptides with N-terminal PEGylation or biotinylation, or as naked (i.e. unmodified) pepides, were all investigated by CD analysis to evaluate if their conformations changed when modified at their N-terminus. This showed that SM083P001 changes conformation as a naked peptide compared to when it is N-terminally modified. SM083P003 and SM083P004 did not display a conformational change when modified at the N-terminus.

To confirm that these three peptides of interest do not contain any β-cell epitopes, the peptides were assessed for antibody binding from sera taken from a healthy volunteer pre- and post- a DTP vaccine booster, from whom we have established that antibodies to the known universal B-cell epitope (MTTE) increased post the DTP booster. The MTTE peptide was used as a positive control. As shown in **Figure 5****,** as expected (given that it is a known TTx B-cell epitope) the MTTE is bound by antibodies in the sera, whereas the other peptides are not. This demonstrates that the peptides of SEQ ID NOs: 10, 13 and 14 are particularly suitable for use as tag peptides according to the invention.

This experiment was repeated using serum from up to 8 donors who had recently received a vaccination containing tetanus toxoid. Serum taken pre and post vaccination was tested (for SM083P001, SM083P004, SM083P005 and the MTTE, serum for 4 donors pre-vaccination and 8 donors post-vaccination was tested; for SM083P002 and SM083P003 the numbers were 3 and 7, respectively). Only the MTTE peptide and peptide SM083P002 displayed binding to antibodies at titres above 400, and only the MTTE peptide was recognised by endogenous antibodies from all donors (Table 4).

**Table 4**

| | SM083P001 | SM083P002 | SM083P003 | SM083P004 | SM083P005 | MTTE |
|---|---|---|---|---|---|---|
| Pre | 0% | 33% | 0% | 0% | 0% | 100% |
| Post | 0% | 14% | 0% | 0% | 0% | 100% |

These results suggest that the selected tetanus-derived peptides are not universal B-cell epitopes and it is unlikely that there will be endogenous pre-existing antibodies to these peptides at the start of a vaccination scheme as a result of a pre-formed immunity against tetanus.

### Example 2 - Systemic Spread of T-Cell Activation Following Therapeutic Administration

### Materials and Methods

Adult female C57BL/6 mice (18-20 g weight) were administered CFSE (carboxyfluorescein succinimidyl ester)-labelled splenocytes from Pmel-1 mice and tghCD40 immature BMDC as described in Example 1 above (see *"In Vivo Experimental Setup").* On day 1, various combinations of antibodies and UU-30 peptide were administered as described above. The antibodies used in this example were Ab-2 and Bi-10, which were administered at doses of 7.5 pmol (low, L); 15 pmol (medium, M); and 22.5 pmol (high, H). The UU-30 peptide was administered at doses of 18.75 pmol (L), 37.5 pmol (M) and 56.25 pmol (H).

After 72 hours, the draining popliteal and non-draining inguinal lymph nodes were harvested and passed through a 70 µm cell strainer to obtain single cell suspensions. Pmel-1 cell accumulation and proliferation was assessed by flow cytometry. Pmel-1 T-cells were gated out based on expression of the congenic marker CD90.1, and the number of proliferating T-cells was measured based on the dilution of the CFSE membrane dye. Statistical analysis was performed using the Kruskal-Wallis test with Dunn's multiple comparisons test for each dose level, comparing the peptide group with each of the antibody/peptide groups.

### Results

The Bi-10 antibody is a bispecific antibody able to bind the peptide tag in UU30; the Ab-2 antibody is the parental anti-CD40 antibody, and thus cannot bind the UU30 peptide. Co-administration of Bi-10 and UU30 results in enhanced T-cell proliferation compared to administration of the peptide alone at all doses in both draining and non-draining lymph nodes, whereas co-administration of Ab-2 with UU30 does not cause any increase in T-cell proliferation **(****Figure 7****).** Statistically significant increases in T-cell proliferation were seen in draining lymph nodes, using low doses of peptide and Bi-10 antibody; and in non-draining lymph nodes, using high doses of peptide and Bi-10 antibody. Co-administration of a tag construct and bispecific antibody according to the invention thus drives proliferation of antigen-specific T-cells.

### Example 3 - Stability of the Interaction Between a Tag and an scFv on a Tetravalent Antibody

### Materials and Methods

The bispecific antibody Bi-17 contains the FITC-8 scFv, which recognises FITC. The scFv is located at the C-terminus of the heavy chain of the anti-CD40 antibody (i.e. it is encoded C-terminal to the IgG2 constant region). The amino acid sequence of the FITC-8 scFv is set forth in SEQ ID NO: 70.

The Bi-17 bispecific antibody was produced by Absolute Antibody (UK). The antibody was expressed in HEK293 cells, and purified by affinity chromatography using protein A followed by preparative size exclusion chromatography (SEC). The purity was determined by SDS-PAGE to be >98 % and monomeric content determined to be 94 % by analytical SEC. Endotoxin levels were <1 EU/mg as determined by LAL chromogenic endotoxin assay.

### CD14 Monocyte and PBMC Isolation.

Peripheral blood mononuclear cells (PBMCs) were isolated from Buffy Coats, donated by healthy volunteers, by Ficoll separation using SepMate (Stemcell Technologies) together with cell density gradient Ficoll^{®} Paque Premium (GE Healthcare) according to the manufacturer's protocol. Further CD14 monocyte separation was performed using the MACS human CD14 microbeads isolation kit (Miltenyi Biotec). Isolated CD14⁺ cells were cultured for 6 days in complete RPMI GlutaMAX medium supplemented with 10 % FBS, 1 % 100 IU/ml penicillin/streptomycin, 10 mM HEPES and 0.1 mM β-mercaptoethanol (Gibco), plus 150 ng/ml hGM-CSF and 50 ng/ml hIL-4 (Peprotech) to drive differentiation of the cells into immature dendritic cells. Half of the medium was replaced (with fresh medium) every 2-3 days.

### Intracellular Peptide Tracking

CD14⁺ cells were cultured for 6 days before the monocytic derived dendritic cells (MoDCs) were harvested and seeded 1 × 10⁵ cells/well in a 96-well flat-bottomed tissue culture plate (Sarstedt). Peptide UU-44 alone (900 nM), antibody Bi-17 alone (300 nM) or peptide UU-44 (900 nM) plus antibody Bi-17 (300 nM) was added to each well, and the cultures incubated. Peptide UU-44 has the amino acid sequence set forth in SEQ ID NO: 71. FITC is conjugated to the N-terminus of the peptide via an aminohexanoic acid linker. SEQ ID NO: 71 contains a human HLA-A2 epitope from the CMV protein pp65 (SEQ ID NO: 72).

Cells for each time point were treated with ice-cold PBS to terminate internalisation and then kept on ice until analysed by flow cytometry (based on detection of FITC). To detect intracellular peptide, trypan blue was used to quench extracellular signal.

### Quenching

UU-44 peptide was diluted in PBS + 1 % BSA in a black Nunc MaxiSorp plate to a concentration of 300 nM. The targeting antibody Bi-17 was serially diluted. Fluorescence was measured using a FLUOstar Omega with excitation at 485 nM and emission at 520 nM. Fluorescence of peptide alone was measured at 0-time point and then 2, 5 and 30 min after the addition of the antibody.

### Results

Bi-17 at 150-600 nM completely quenched the fluorescent signal of 300 nM peptide UU-44 **(****Figure 8****).** This indicates that both scFvs in Bi-17 are functional and bind up tag sufficiently that two UU-44 peptides are bound per bispecific antibody. The quenching is maintained over time (here measured up to 30 min). When an excess of free peptide is present, fluorescence intensity (y-axis) can be measured as expected. Thus the binding of the anti-FITC scFv of the Bi-17 antibody quenches all FITC fluorescent signal. This interaction is in the low nM range (Söderlind et al., Nature Biotechnology 18: 852-856, 2000).

Internalisation of peptide UU-44 by the MoDCs is shown in **Figure 9****.** The figure shows the proportion of MoDCs that are FITC positive (i.e. have internalised the UU-44 peptide). **Fig. 9A** shows internalisation of peptide UU-44 alone. Over time there is a gradual increase in peptide uptake (as expected, since the experiment was performed in a closed system).

**Fig. 9B** shows internalisation of peptide UU-44 when applied to the cells in combination with the bispecific antibody Bi-17. Initially, a low fluorescence signal was seen, since the peptide was bound by Bi-17 and the FITC signal quenched by the scFv. From 2 to 6 hours there was a gradual increase in intracellular FITC signal, indicating that the peptide is stably internalised by the cell, as the transporter antibody binds to CD40 and is itself internalised. Release of the peptide from the bispecific antibody takes place intracellularly and increases over time forming a depot reservoir of the peptide that is delivered for processing and antigen presentation over time. Delivery of the peptide via a bispecific antibody can thus provide antigen over a period of time for MHC class I presentation.

### Example 4 - Expansion of Infectious Disease-Specific T-Cells

### Materials and Methods

Monocytes and T-cells were obtained from a CMV-positive, HLA-A2-positive donor, and Ficoll separation was performed according to the manufacturer's protocol to isolate the PBMCs from the blood. For further separation, MACS beads were used for both positive selection of CD14+ monocytes and negative selection of T-cells, according to the manufacturer's protocol. CD14+ monocytes were cultured for 6 days in the presence of hGM-CSF and IL4 and cytokines were added on day 0, 3 and 5.

On day 6 the MoDCs were harvested, counted and plated, and antibodies and peptides were then added as follows: to one sample only 200 nM peptide UU-44 was added, to another sample only 100 nM bispecific antibody Bi-17 and to a third sample 200 nM UU-44 and 100 nM Bi-17. For the final sample, the Bi-17 antibody and UU-44 peptide were mixed and incubated for 30 minutes at 37°C prior to addition to the sample, to allow peptide-antibody complexes to form.

One day post maturation, T-cells from the same donor were added to the cultures at a ratio of T-cells:MoDCs of 5:1. The cultures were then incubated for 7 days and subsequently analysed by using tetramer staining to detect expansion of T cells specific for the pp65 epitope of UU-44. A one-way ANOVA with Tukey's multiple comparisons test was used to assess the statistical significance of differences between the UU44, Bi-17 and UU44/Bi-17 groups.

### Results

The proportion of T-cells in each culture specific for the CMV pp65 epitope in UU-44 is shown in **Figure 10****.** The data shows that incubation of the immune cells with peptide UU-44 alone or bispecific antibody Bi-17 alone did not result in any increase in pp65-specific T-cells relative to controls. Incubation of the immune cells with peptide UU-44 in combination with bispecific antibody Bi-17 resulted in a statistically significant increase in pp65-specific T-cells, demonstrating that use of the conjugate/peptide complexes of the invention drives expansion of antigen-specific T-cells.

### Example 5 - Target Protein Internalisation

### Materials and Methods

Human CD14⁺ cells were differentiated into immature DCs in 6 days, as described above, and seeded in a 96-well tissue-culture plate (Sarsedt) at a density of 1 × 10⁵ cells/well. The ABS-1150/1151 clones X-SM083-ab-1 (IgG1) and X-SM083-ab-2 (IgG2) were used in a titration range (3.9-500 nM) along with LPS and unstimulated cells to investigate agonistic properties of the antibodies and their effect on CD40 expression.

The test antibodies were added at different concentrations (in the titration range) to the dendritic cells, which were then incubated for 48 h at 37°C. LPS (1 ug/ml) was used as positive control. DC activation was assessed by FACS, using the following markers: CD14-APC-Cy7 (HCD14), HLA-DR PE-Cy5 (L243), CD86-BV421 (IT2.2), CD40-FITC (5C3), CD1a-PE (HI149) and CD83-APC (HB15e), and IL12 p40 sandwich ELISA (Biolegend ELISA MAX kit, cat. 430704).

The detection antibody used, CD40-FITC (5C3), can be blocked by ABS-1150/1151 (data not shown). However since 48 hours had passed between application of the test antibodies to the cells and the FACS analysis, it was deemed that this would not impact the results obtained.

### Results

Internalisation of CD40 upon its engagement by an antibody of the invention is required to ensure antigen uptake by the target cells. The ABS-1150/1151 antibody in either IgG1 or IgG2 format was used to stimulate human MoDCs, and to investigate the agonistic activity of the two antibodies, along with target protein surface expression. The IgG2 subtype of the ABS-1150/1151 antibody was superior to the IgG1 subtype in upregulating CD86 (see **Figure 11A****),** in terms of the fold change in CD86 expression relative to unstimulated cells. Similar results were obtained for expression of other activation/maturation markers and IL12 production (data not shown).

The IgG2 subtype antibody also caused a decrease in cell surface expression of CD40, which was not seen when the IgG1 subtype was used **(****Figure 11B****).** Thus antibodies of the IgG2 subtype are superior to those of the IgG1 subtype in promoting CD40 internalisation, which is advantageous in the invention. This is also supported by the data in Figure 2, which shows that ABS-1150/1151 in the IgG2 format improves antigen presentation relative to the IgG1 format.

### Example 6 - Increase in Plasma Stability of a Peptide Upon Binding to Bispecific Antibody

### Materials and Methods

Peptide UU-30 (SEQ ID NO: 5) was purchased from Capra Science (Sweden). Bispecific antibodies X-SM083-bi-9 and X-SM083-bi-12 (Bi-9 and Bi-12 for short) and their corresponding parental monoclonal antibody counterparts X-SM083-ab-1 and X-SM083-ab-3 (ab-1 and ab-3) were produced by the Drug Discovery and Development Platform, SciLifeLab (Sweden) (see Example 1 and Table 1). Mouse plasma (containing K₂EDTA as anticoagulant) was purchased from Innovative Research (USA). LC grade acetonitrile (ACN) and methanol (MeOH) were obtained from Millipore. MS grade formic acid (HCOOH) was obtained from VWR. Type 1 milliQ water (18.2 mΩ.cm at 25°C, Millipore) was used for preparation of mobile phases.

1 µM UU-30 peptide was mixed with binding (Bi-9 and Bi-12) and non-binding antibodies (Ab-1 and Ab-3) at a molar Ag:Ab ratio of 0.5:1 and 1:1, respectively, to achieve < 1 % free peptide. The complexes were mixed with mouse plasma pre-warmed to 37°C under agitation at 600 rpm. Reactions were stopped at time points by adding three volumes of ice-cold methanol. To zero-minute samples 100 % ice-cold methanol was added prior to addition of plasma to the peptides. The samples were vortexed and centrifuged for 20 minutes at 3220 × g and 4°C, followed by evaporation of supernatant and subsequent reconstitution of dried material in 100 µL 2 % ACN; 0.05 % HCOOH; 97.5 % H₂O prior to analysis by LC-MS/MS. For comparison, samples of UU-30 without addition of any antibodies were prepared analogously.

Analysis was based on monitoring peptide transitions by LC-MS/MS in positive electrospray mode on a QTrap6500 connected to an Acquity UHPLC system. 10 µL sample aliquots were injected into a BEH C₈ column (2.1 × 50 mm, 1.7 µm) set at 60°C, and eluted at 500 µL/min with linear gradient from 0 % to 100 % mobile phase B within 1.6 minutes. Composition of mobile phase A was 0.1 % HCOOH: 99.9 % H₂O and B was 0.1 % HCOOH: 99.9 % ACN. Monitored transitions in MRM mode for UU-30 were set to 862.4 > 1012.6 (DP, 120 V; CE, 38 V), 718.8 > 810.3 (DP, 120 V; CE 30 V) and 616.2 > 740.1 (DP, 100 V; CE, 18 V). Capillary voltage was set to 5.5 kV, temperature to 450 °C, gas 1 and 2 to 60 au and curtain gas to 40 au.

### Results

Association of a peptide with a larger protein could in theory improve the serum stability of the peptide. Thus a peptide tag construct
could have an improved half-life in the context of a complex with a bispecific peptide. Using mass spectrometry we assessed if the UU-30 peptide, comprising a T-cell epitope and MTTE tag, would have an improved half-life in plasma when complexed with a bispecific antibody compared to when the peptide is merely mixed together with a non-binding antibody.

It was found that complexing of UU30 with a bispecific antibody according to the invention resulted in a significant increase in half-life of the peptide, relative to when the peptide was free in plasma **(****Figure 12** and Table 5).

**Table 5**

| | t_{1/2} (min) | Average t_{1/2} (min) Non-Binding | Average t_{1/2} (min) Binding |
|---|---|---|---|
| UU-30 | 20 | 17 | - |
| UU-30 + Ab-1 | 19 | | |
| UU-30 + Ab-3 | 13 | | |
| UU-30 + bi-12 | 424 | - | > 300 |
| UU-30 + bi-9 | 1344 | | |

Improvement of the half-life of peptide constructs according to the invention provides a greater opportunity to generate an effective immune response against the T-cell epitope. Thus use of the bispecific antibodies of the invention increases the likelihood of an effective immune response against the target antigen being generated.

### Example 7 - Peptide Tag Stability in Plasma

The potential tag peptides SM083P001 (SEQ ID NO: 10), SM083P003 (SEQ ID NO: 12) and SM083P004 (SEQ ID NO: 13), identified in Example 1, were tested to determine their stability in plasma.

### Materials and Methods

The three peptides were purchased from Capra Science. Mouse plasma (containing K₂EDTA anticoagulant) was purchased from Innovative Research, while human plasma (containing sodium citrate anticoagulant) was obtained from Uppsala University Hospital. LC grade acetonitrile (ACN) and methanol (MeOH) were from Millipore. MS grade formic acid (HCOOH) was ordered from VWR. Type 1 milliQ water (18.2 mΩ.cm at 25°C, Millipore) was used for preparation of mobile phases.

10 µM of each peptide was incubated in human or mouse plasma for up to 17 hours at 37°C under agitation at 600 rpm. Incubations were stopped at time points by adding three volumes of ice-cold methanol. To zero-minute samples 100 % ice-cold methanol was added prior to addition of plasma to the peptides. The samples were vortexed and centrifuged for 20 minutes at 3220 × g and 4°C, followed by evaporation of supernatant and subsequent reconstitution of dried material in 100 µL 2 % ACN, 0.05 % HCOOH, 97.5 % H₂O prior to analysis by LC-MS/MS.

Analysis was based on monitoring peptide transitions with LC-MS/MS in positive electrospray mode on a TQ-S micro connected to an Acquity UHPLC system. 10 µL sample aliquots were injected into a BEH C₈ column (2.1 × 50 mm, 1.7 µm) set at 60°C and eluted at 500 µL/min with linear gradient from 0 % to 100 % mobile phase B within 1.6 minutes. Composition of mobile phase A was 0.1 % HCOOH, 99.9 % H₂O and B was 0.1 % HCOOH, 99.9 % ACN. Monitored transitions in MRM mode for SM083P001 were 875.54 > 1097.22 and 875.54 > 1170.51 (CE, 15 V; Cone, 27 V), for SM083P003 were 728.21 > 838.31 and 728.21 > 943.62 (CE, 25 V; Cone, 21 V) and for SM083P004 were 814.88 > 1078.33 and 814.88 > 1136.44 (CE, 20 V; Cone, 25 V). Source conditions were set to 2 kV for capillary voltage with gas temperature set at 600°C.

### Results

As shown in **Figure 13** and Table 6, all three peptides demonstrated good half-lives in both mouse and human plasma, supporting their use in the invention as tag peptides.

**Table 6**

| **Peptide Tag** | **t_{1/2} [h] in mouse plasma** | **t_{1/2} [h] in human plasma** |
|---|---|---|
| SM083P001 | 5.7 | 45 |
| SM083P003 | 5.6 | 14 |
| SM083P004 | 2.8 | 28 |

### Example 8 - Generation of Novel Anti-Tag Peptide scFvs

Phage display selections were performed to isolate scFvs which recognise the potential tag peptides SM083P001-5 (SEQ ID NOs: 10-14).

### Materials and Methods

### Phage Display Selections from Naive Libraries

For all five biotinylated peptide antigens (SM083P001-0005, see Example 1) phage display was performed using four rounds of enrichment employing two in-house constructed human synthetic scFv phage libraries, SciLifeLib1 and SciLifeLib2, similar in design and construction to previously reported (Säll, A. et al., Protein Eng Des Sel 29, 427-437, 2016). The selections were performed by immobilising the peptides on streptavidin-coated paramagnetic beads (Dynabeads M-280, ThermoFisher Scientific, #11206D), and most of the steps in the selection process were automated and performed with a Kingfisher Flex robot. Prior to the phage-antigen incubation step in rounds 1 and 2, the phage stocks were pre-selected on streptavidin-coated beads in order to remove non-specific or streptavidin binders. The selection pressure was increased between the different rounds by gradually decreasing the antigen amount (concentration range 200-50 nM) and by increasing the number and intensity of washes (5-10). Elution of phages was performed using trypsin. Recovered phages were propagated in Top10F' *E. coli,* either on agar plates at 37°C overnight (round 1) or in solution at 30°C overnight (rounds 2-4). Phage stocks were made by infecting with an excess of M13K07 helper phage (New England Biolabs, #N0315S) and scFv expression induced by the addition of IPTG. The overnight cultures were PEG/NaCI-precipitated, resuspended in selection buffer (2 % (w/v) BSA, 0.05 % (v/v) Tween-20 in PBS) and used for the next round of selection.

### Re-Cloning and Expression of Soluble scFvs

To allow production of soluble scFv, phagemid DNA from the third and fourth round of each selection track was isolated. In pools, the genes encoding the scFv fragments were sub-cloned into a screening vector, providing a signal for secretion of the scFv along with a triple-FLAG tag and a hexahistidine (His) tag at the C-terminus. The constructs were subsequently transformed into TOP10 *E.coli.* For each selection track, 188 colonies in total from rounds 3 and 4 were picked and cultured in 96-well plates and expressed scFv (supernatants) assessed for binding.

### Primary ELISA and Sequencing

Binding of the selected scFv was initially assessed by ELISA. Each clone was tested against its target peptide and streptavidin, all in duplicate. Streptavidin, diluted in PBS to 1 µg/ml, was coated onto a polystyrene 384-well plate (Corning, #3700) at 4°C overnight. The biotinylated peptides were added in PBT (0.5% (w/v) BSA, 0.05% (v/v) Tween-20 in PBS), followed by the addition of scFv (culture supernatant). Detection of binding was enabled by a horseradish peroxidase (HRP)-conjugated α-FLAG M2 antibody, followed by incubation with chromogenic substrate Ultra TMB-ELISA. Signal development was stopped by addition of 1 M sulphuric acid and absorbance was measured at 450 nm. Clones considered positive in ELISA were subjected to DNA sequencing (GATC Biotech, Cologne, Germany).

### Secondary ELISA

All sequence-unique clones identified for each selection track were further analysed in a secondary ELISA screen. Here, binding to N-terminal PEGylated peptide variants of SM083P001, SM083P003 and SM083P004 was assessed. In addition, on a sub-set of scFv, binding was analysed in a third ELISA where neutravidin was used as capture surface for the biotinylated peptide instead of streptavidin that was used in the phage selection and in the primary ELISA screen.

### Affinity Measurements Using Surface Plasmon Resonance (SPR)

Affinity measurements were performed on a Biacore T200 biosensor instrument (GE Healthcare) on a sub-set of the selected scFv. SM083P003 and SM083P004 peptides were captured on sensor chip SA with pre-immobilised streptavidin. One streptavidin surface was left unmodified and used as a reference surface. A 5-fold dilution series of each scFv clone and reference antibody, ranging between 0.16-100 nM, was prepared in running buffer and sequentially injected over the chip surfaces. Following a dissociation phase, the chip surfaces were regenerated with 10 mM glycine-HCI, pH 2.1. By subtracting the response curve of a reference surface, being a streptavidin immobilised surface, response curve sensorgrams for the scFv clones were obtained. Data was analysed using the Biacore T200 Evaluation 3.1 software and kinetic parameters were calculated assuming a 1:1 Langmuir binding model.

### Epitope Mapping

ScFv binders specific for SM083P003 (Y-SM083-p03-B06 and C06) and SM083P004 (Y-SM083-p04-C04, D04, F04, G04, and H04) were epitope mapped in detail using 15-mer peptides. SM083P003 has a length of 25 aa whereas SM083P004 is 21 aa long. However, in order to investigate whether future modifications can be allowed without affecting antibody binding, a set of 15-mer peptides spanning a 37 aa region was assessed, i.e. scFv specific for SM083P003 were analysed on peptides found in the following sequence DANLISIDIKNDLYEKTLNDYKAIANKLSQVTSCNDP (SEQ ID NO: 73), whereas anti-SM083P004 were assessed on peptides found in TIGKSKYFQDPALLLMHELIHVLHGLYGMQVSSHEII (SEQ ID NO: 74). The underlined regions correspond to SM083P003 and SM083P004, respectively, and the sequences surrounding these are those naturally found in tetanus toxin. In total, 46 15-mer peptides carrying an N-terminal biotin moiety, a glycine amide at the C-terminus and covering SM083P003 and SM083P004 with 1 amino acid shift were ordered from JPT Peptide Technologies GmbH (Germany) (Table 7).

384-well ELISA plates were coated with 2 µg/ml streptavidin in PBS at 4°C, overnight. Following washing and blocking (5 % BSA in PBS) peptides were allowed to bind for 1 hour. All scFv clones were diluted to 2 µg/ml in PBT (0.5 % (w/v) BSA, 0.05 % (v/v) Tween-20 in PBS) and allowed to bind for 1 h. Detection of binding was enabled by the use of an HRP-conjugated α-FLAG M2 antibody as described above. Each sample was assayed in duplicate from which a mean absorbance value was retrieved with blank well background subtraction. Absorbance values equal to or higher than 0.5 were considered as positive.

**Table 7**

| | **Peptides used for mapping anti-SM083P003 scFv** | | **Peptides used for mapping anti-SM083P004 scFv** | |
|---|---|---|---|---|
| **1** | DANLISIDIKNDLYE | (SEQ ID NO: 75) | TIGKSKYFQDPALLL | (SEQ ID NO: 98) |
| **2** | ANLISIDIKNDLYEK | (SEQ ID NO: 76) | IGKSKYFQDPALLLM | (SEQ ID NO: 99) |
| **3** | NLISIDIKNDLYEKT | (SEQ ID NO: 77) | GKSKYFQDPALLLMH | (SEQ ID NO: 100) |
| **4** | LISIDIKNDLYEKTL | (SEQ ID NO: 78) | KSKYFQDPALLLMHE | (SEQ ID NO: 101) |
| **5** | ISIDIKNDLYEKTLN | (SEQ ID NO: 79) | SKYFQDPALLLMHEL | (SEQ ID NO: 102) |
| **6** | SIDIKNDLYEKTLND | (SEQ ID NO: 80) | KYFQDPALLLMHELI | (SEQ ID NO: 103) |
| **7** | IDIKNDLYEKTLNDY | (SEQ ID NO: 81) | YFQDPALLLMHELIH | (SEQ ID NO: 104) |
| **8** | DIKNDLYEKTLNDYK | (SEQ ID NO: 82) | FQDPALLLMHELIHV | (SEQ ID NO: 105) |
| 9 | IKNDLYEKTLNDYKA | (SEQ ID NO: 83) | QOPALLLMHELlHVL | (SEQ ID NO: 106) |
| **10** | KNDLYEKTLNDYKAl | (SEQ ID NO: 84) | DPALLLMHELIHVLH | (SEQ ID NO: 107) |
| **11** | NDLYEKTLNDYKAIA | (SEQ ID NO: 85) | PALLLMHELIHVLHG | (SEQ ID NO: 108) |
| **12** | DLYEKTLNDYKAIAN | (SEQ ID NO: 86) | ALLLMHELIHVLHGL | (SEQ ID NO: 109) |
| **13** | LYEKTLNDYKAIANK | (SEQ ID NO: 87) | LLLMHELIHVLHGLY | (SEQ ID NO: 110) |
| **14** | YEKTLNDYKAIANKL | (SEQ ID NO: 88) | LLMHELIHVLHGLYG | (SEQ ID NO: 111) |
| **15** | EKTLNDYKAIANKLS | (SEQ ID NO: 89) | LMHELIHVLHGLYGM | (SEQ ID NO: 112) |
| **16** | KTLNDYKAIANKLSQ | (SEQ ID NO: 90) | MHELIHVLHGLYGMQ | (SEQ ID NO: 113) |
| **17** | TLNDYKAIANKLSQV | (SEQ ID NO: 91) | HELIHVLHGLYGMQV | (SEQ ID NO: 114) |
| **18** | LNDYKAIANKLSQVT | (SEQ ID NO: 92) | ELIHVLHGLYGMQVS | (SEQ ID NO: 115) |
| **19** | NDYKAIANKLSQVTS | (SEQ ID NO: 93) | LIHVLHGLYGMQVSS | (SEQ ID NO: 116) |
| **20** | DYKAIANKLSQVTSC | (SEQ ID NO: 94) | IHVLHGLYGMQVSSH | (SEQ ID NO: 117) |
| **21** | YKAIANKLSQVTSCN | (SEQ ID NO: 95) | HVLHGLYGMQVSSHE | (SEQ ID NO: 118) |
| **22** | KAIANKLSQVTSCND | (SEQ ID NO: 96) | VLHGLYGMQVSSHEI | (SEQ ID NO: 119) |
| **23** | AIANKLSQVTSCNDP | (SEQ ID NO: 97) | LHGLYGMQVSSHEII | (SEQ ID NO: 120) |

### Results

### Phage Display Selections from Naive Libraries and Subsequent Binding Screens and Sequencing

A total of 10 phage selection tracks were performed in parallel for the five peptides SM083P001-0005 using SciLifeLib 1 and 2. 188 clones were picked and analysed from each of the 10 tracks in a primary ELISA. Positive clones were identified for all peptides except for SM083P002. Subsequent sequencing of positive clones resulted in a total of 22 unique clones (Tables 8 and 9).

All sequence-unique clones were analysed in a secondary ELISA. Here, binding to peptides having a PEGylated spacer between the N-terminal part of the peptide and the biotin was assessed. As summarized in Table 8, only eight of the sequence-unique scFv passed this selection criterion. Also, to make sure that the now eight scFv did not rely on streptavidin for interaction with their cognate peptide, binding was assessed using neutravidin as capture surface for the biotinylated peptide. It could be concluded that all of the eight scFv bind their cognate peptide independent of capture surface (data not shown).

**Table 8**

| Peptide antigen | Nr of sequence-unique clones after primary ELISA | Nr of clones binding the peptide independent of capture surface or N-terminal modification |
|---|---|---|
| SM083P001 | 11 | 0 |
| SM083P002 | 0 | 0 |
| SM083P003 | 3 | 2 |
| SM083P004 | 8 | 6 |
| SM083P005 | 1 | 0 |

**Table 9**

| **scFv** | **Target Peptide** | **Sequence** |
|---|---|---|
| Y-SM083-p03-C06 | SM083P003 | SEQ ID NO: 150 |
| Y-SM083-p04-C04 | SM083P004 | SEQ ID NO: 151 |
| Y-SM083-p04-D04 | SM083P004 | SEQ ID NO: 152 |
| Y-SM083-p04-F04 | SM083P004 | SEQ ID NO: 153 |
| Y-SM083-p04-G04 | SM083P004 | SEQ ID NO: 154 |
| Y-SM083-p04-H04 | SM083P004 | SEQ ID NO: 155 |

### Affinity Measurements (SPR)

Kinetic parameters, including association rate constant (kd (M-1 s-1)), dissociation rate constant (kd (s-1)) and equilibrium of dissociation (K_{D} (M)), for binding to cognate peptide were calculated for a subset of scFv by surface plasmon resonance. The results are shown in Table 10.

**Table 10**

| **scFv** | **Target peptide** | **ka (M-1 s-1)** | **kd (s-1))** | **K_{D} (M)** |
|---|---|---|---|---|
| Y-SM083-p04-C04 | SM083P004 | 3.8x10+04 | 6.2x10-04 | 1.6x10-08 |
| Y-SM083-p04-D04 | SM083P004 | 1.3x10+05 | 4.3x10-04 | 3.3x10-09 |
| Y-SM083-p04-G04 | SM083P004 | 2.0x10+04 | 1.8x10-03 | 8.9x10-08 |
| Y-SM083-p04-H04 | SM083P004 | 3.8x10+04 | 4.5x10-04 | 1.2x10-08 |

### Epitope Mapping

By epitope mapping on an array of 15-mer peptides it was possible to map the epitopes of the scFv to 9-15 aa long windows (Table 11). Although the relative affinities to the cognate peptides and the shorter 15-mer peptides were not calculated, this indicates that peptide trimming is possible.

**Table 11**

| **scFv** | **Target peptide** | **Peptides positive in epitope mapping ELISA** | **Epitope** | |
|---|---|---|---|---|
| Y-SM083-p03-C06 | SM083P003 | 15, 16, 17, 18, 19 | NDYKAIANKLS | SEQ ID NO: 121 |
| Y-SM083-p04-C04 | SM083P004 | 13, 14, 15 | LMHELIHVLHGLY | SEQ ID NO: 122 |
| Y-SM083-p04-D04 | SM083P004 | 13, 14, 15 | LMHELIHVLHGLY | SEQ ID NO: 122 |
| Y-SM083-p04-F04 | SM083P004 | 15 | LMHELIHVLHGLYGM | SEQ ID NO: 123 |
| Y-SM083-p04-G04 | SM083P004 | 13, 14, 15, 16, 17, 18, 19 | LIHVLHGLY | SEQ ID NO: 124 |
| Y-SM083-p04-H04 | SM083P004 | 10, 11 | PALLLMHELIHVLH | SEQ ID NO: 125 |

### Example 9 - Effect of Bispecific Antibodies In Vivo

### Materials and Methods

8 week old male B6.Cg-Thy1a/Cy Tg(TcraTcrb)8Rest/J mice (herein called pmel mice) were used for the *in vivo* tumour experiment. Mice were bred in the animal facility at Uppsala Biomedical center and animal experiments were approved by Uppsala animal testing ethical committee, Uppsala, Sweden.

Mice were divided into two treatment groups (n=2 per group). Group-1 was vaccinated with 18.75 pmol peptide UU-30, while group-2 was vaccinated with a combination of 18.75 pmol UU-30 and 7.5 pmol bispecific antibody Bi-10. Vaccination was performed via intra-dermal injection into the hock in a prime-boost-boost fashion. For priming and first boost, 4 × 10⁵ tghCD40-expressing immature bone marrow derived dendritic cells (BMDC) were mixed with the vaccine and injected into the right side hock on day 0 and day 4 respectively. On day 8, the second boost was administered to the left side hock mixed with 4 × 10⁵tghCD40 immature BMDC. On day 5, 75,000 B16-F1 melanoma cells were inoculated into the right side flank. Tumour sizes were measured every other day to follow up tumour progression.

### Results

As shown in **Figure 14****,** by 20 days post tumour inoculation mice vaccinated with peptide UU-30 alone had developed tumours with an average volume of over 400 mm³. Mice vaccinated with peptide UU-30 in combination with Bi-10 had developed tumours with an average volume of only about 100 mm³. This shows that vaccination according to the invention is effective in stimulating an anti-tumour immune response.

### Example 10 - Generation and Binding Characterisation of anti-MTTE scFv by SPR

### Materials and Methods

### Small-Scale Production and Purification

The sequences of the VH and VL domains of the IBIIICI and 14GIIICII clones were obtained by sequencing hybridoma cells (performed by Absolute Antibody, Cleveland, UK).

By fusing the variable domain of the heavy chain to the variable domain of the light chain of the antibodies, via a glycine-serine linker ((Gly₄Ser)₄), the corresponding scFv constructs were generated (SEQ ID NO: 47 and 48). The scFv genes were sub-cloned into a screening vector, providing a signal for secretion of the scFv along with a triple-FLAG tag and a hexahistidine (His) tag at the C-terminus. Synthesis and sub-cloning of the scFv genes were out-sourced to GenScript (Piscataway, NJ, USA). The constructs were subsequently transformed into TOP10 *E.coli.* Bacterial supernatants of lysed cells were Ni-NTA-purified using immobilised metal affinity chromatography (IMAC) under gravity flow and buffer exchanged to PBS. Purified scFv fragments were analysed by gel electrophoresis under reducing conditions to determine purity and integrity.

### Surface Plasmon Resonance (SPR) Measurements

Affinity measurements of the scFv clones were performed by SPR using Biacore T200 (GE Healthcare). An α-FLAG M2 antibody was immobilised onto a CM5 S chip by primary amine coupling using NHS-EDC chemistry, allowing capture of the scFvs through their FLAG tags. A 5-fold dilution series comprised of five different concentrations (0.16 nM to 100 nM) of biotinylated MTTE-peptide (SEQ ID NO: 6) was sequentially injected over the flow cells, allowing binding to the captured scFv fragments. As a negative control, a scrambled MTTE sequence was similarly injected. Regeneration of the surface was accomplished under acidic conditions using 10 mM glycine-HCI at pH 2.2. Single cycle kinetic data obtained was fitted to a 1:1 Langmuir binding model and kinetic parameters, kₐ (1/Ms), k_{d} (1/s) and K_{D} (M) were retrieved using software BlAevaluation.

### Results

### Small-Scale Production and Purification

Gel electrophoresis of bacterially expressed and purified scFv fragments showed a high purity with one main protein band corresponding to the expected molecular weight of the scFv (data not shown).

### Surface Plasmon Resonance (SPR) Measurements

A single cycle kinetic approach was used to determine the kinetic parameters, including association rate constant (kₐ (M⁻¹ s⁻¹)), dissociation rate constant (k_{d} (s⁻¹)) and equilibrium of dissociation (K_{D} (M)), of IBIIICI-b and 14GIIICII-b binding to the MTTE peptide. The results are shown in Table 12. No binding could be detected to the scrambled negative control peptide (not shown).

**Table 12**

| **scFv** | **kₐ (M⁻¹ s⁻1)** | **k_{d} (S⁻¹)** | **K_{D} (M)** |
|---|---|---|---|
| IBIIICI-b | 9.2 × 10⁵ | 4.8 × 10⁻⁴ | 5.2 × 10⁻¹⁰ |
| 14GIIICII-b | 1.8 × 10⁵ | 3.2 × 10⁻⁴ | 1.8 × 10⁻⁹ |

## Claims

1. A conjugate comprising:
i) at least one first specific binding molecule which binds CD40, wherein said first specific binding molecule is an agonist of CD40, and wherein said first specific binding molecule induces CD40 internalisation upon binding to CD40; and
ii) at least one second specific binding molecule which binds a tag moiety, wherein said tag moiety is a peptide and not a cancer antigen, wherein said peptide consists of the amino acid sequence set forth in any one of SEQ ID NOs: 6 or 10-14, or a fragment of any one of SEQ ID NOs: 6 or 10-14 of at least 5 amino acids;
wherein said first specific binding molecule and second specific binding molecule are antigen-binding proteins comprising an antigen-binding domain of an antibody and are covalently linked

2. The conjugate of claim 1, wherein said first specific binding molecule:
(i) further comprises a Fc region of an antibody; and/or
(ii) is bivalent; and/or
(iii) is a monoclonal antibody, optionally of the IgG isotype; and/or
(iv) is a F(ab')₂ fragment of a monoclonal antibody; and/or
(v) binds human CD40, and is an agonist of human CD40.

3. The conjugate of claim 2, wherein said first specific binding molecule is an antigen-binding protein comprising at least one heavy chain variable region (VH) and at least one light chain variable region (VL), wherein:
(i)
(a) the VH region comprises VHCDRs 1, 2 and 3 which comprise the amino acid sequences set forth in SEQ ID NOs: 17, 18 and 19 respectively, and
(b) the VL region comprises VLCDRs 1, 2 and 3 which comprise the amino acid sequences set forth in SEQ ID NOs: 20, 21 and 22, respectively; or
(ii)
(a) the VH region comprises VHCDRs 1, 2 and 3 which comprise the amino acid sequences set forth in SEQ ID NOs: 31, 32 and 33, respectively; and
(b) the VL region comprises VLCDRs 1, 2 and 3 which comprise the amino acid sequences set forth in SEQ ID NOs: 34, 35 and 36, respectively; or
(iii)
(a) the VH region comprises VHCDRs 1, 2 and 3 which comprise the amino acid sequences set forth in SEQ ID NOs: 58, 59 and 60, respectively; and
(b) the VL region comprises VLCDRs 1, 2 and 3 which comprise the amino acid sequences set forth in SEQ ID NOs: 61, 62 and 63, respectively; or
(iv)
(a) the VH region comprises VHCDRs 1, 2 and 3 which comprise the amino acid sequences set forth in SEQ ID NOs: 64, 65 and 66, respectively, and
(b) the VL region comprises VLCDRs 1, 2 and 3 which comprise the amino acid sequences set forth in SEQ ID NOs: 67, 68 and 69, respectively; or
(v)
(a) the VH region comprises VHCDRs 1, 2 and 3 which comprise the amino acid sequences set forth in SEQ ID NOs: 138, 139 and 140, respectively, and
(b) the VL region comprises VLCDRs 1, 2 and 3 which comprise the amino acid sequences set forth in SEQ ID NOs: 141, 142 and 143, respectively; or
(vi)
(a) the VH region comprises VHCDRs 1, 2 and 3 which comprise the amino acid sequences set forth in SEQ ID NOs: 144, 145 and 146, respectively, and
(b) the VL region comprises VLCDRs 1, 2 and 3 which comprise the amino acid sequences set forth in SEQ ID NOs: 147, 148 and 149, respectively.

4. The conjugate of any one of claims 1 to 3, wherein said second specific binding molecule is an scFv, and/or wherein said second specific binding molecule is covalently linked to a light or heavy chain of said first specific binding molecule.

5. The conjugate of claim 4, wherein said conjugate comprises one first specific binding molecule, which is an antibody, and two second specific binding molecules, which are scFvs, and:
(i) one scFv is conjugated to the C_{H}3 domain of each heavy chain of said antibody; or
(ii) one scFv is conjugated to the C_{L} domain of each light chain of said antibody.

6. The conjugate of any one of claims 1 to 5, wherein said tag moiety comprises no universal human B-cell epitopes.

7. The conjugate of any one of claims 1 to 5 wherein said tag moiety comprises a B-cell epitope, wherein said B-cell epitope optionally is a non-human B-cell epitope.

8. A complex comprising a conjugate as defined in any one of claims 1 to 7 and a tag construct, said tag construct comprising a tag moiety covalently attached to an antigen, wherein the tag moiety of said tag construct is non-covalently bound to the second specific binding molecule and said tag moiety is as defined in any one of claims 1, 6 and 7.

9. The complex of claim 8, wherein said antigen is a cancer antigen or derived from a pathogen, optionally wherein said cancer antigen is a neoantigen, a tumour-associated antigen, or an antigen derived from an oncovirus.

10. A pharmaceutical composition comprising:
i) a conjugate as defined in any one of claims 1 to 7; or
iii) a complex as defined in any one of claims 8 to 9,
and at least one pharmaceutically-acceptable carrier or excipient.

11. A kit comprising a conjugate as defined in any one of claims 1 to 7 and a tag construct comprising:
i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
ii) an antigen, being a cancer antigen or an antigen derived from a pathogen;
wherein said antigen is a polypeptide and said tag moiety is covalently linked to said antigen.

12. A complex as defined in claim 8 or 9 or a kit as defined in claim 11 for use in therapy.

13. A product comprising a conjugate as defined in any one of claims 1 to 7 and a tag construct as a combined preparation for simultaneous or sequential use in therapy, wherein the tag construct comprises
i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
ii) an antigen, being a cancer antigen or an antigen derived from a pathogen;
wherein said antigen is a polypeptide and said tag moiety is covalently linked to said antigen.

14. A complex as defined in claim 8 or 9, a kit as defined in claim 11 or a product for use as defined in claim 13, for use in the treatment or prevention of cancer, or for use in the treatment or prevention of an infectious disease.

15. An *in vitro* or *ex vivo* method of activating a T-cell expressing a TCR which recognises an antigen, said method comprising contacting an antigen-presenting cell with:
(a) a conjugate as defined in any one of claims 1 to 7 and a tag construct, wherein said tag construct comprises the antigen recognised by said TCR and wherein the tag construct comprises
i) a peptide tag moiety which is not a cancer antigen and consists of the amino acid sequence set forth in any one of SEQ ID NOs: 6 or 10-14, or a fragment of any one of SEQ ID NOs: 6 or 10-14 of at least 5 amino acids, optionally wherein said tag moiety is as defined in any one of claims 6 to 7; and
ii) an antigen, being a cancer antigen or an antigen derived from a pathogen; wherein said antigen is a polypeptide and said tag moiety is covalently linked to said antigen.
; or
(b) a complex as defined in claim 8 or 9, wherein the tag construct of said complex comprises the antigen recognised by said TCR.

## Patentansprüche

1. Konjugat, umfassend:
i) wenigstens ein erstes spezifisches Bindemolekül, das CD40 bindet, wobei das erste spezifische Bindemolekül ein Agonist von CD40 ist und wobei das erste spezifische Bindemolekül bei Bindung an CD40 CD40-Internalisierung induziert; und
ii) mindestens ein zweites spezifisches Bindemolekül, das eine Tag-Gruppierung bindet, wobei es sich bei der Tag-Gruppierung um ein Peptid und nicht um ein Krebsantigen handelt, wobei das Peptid aus der Aminosäuresequenz unter einer von SEQ ID NO: 6 oder 10-14 oder einem Fragment von mindestens 5 Aminosäuren einer von SEQ ID NO: 6 oder 10-14 besteht;
wobei es sich bei dem ersten spezifischen Bindemolekül und dem zweiten spezifischen Bindemolekül um antigenbindende Proteine handelt, die eine antigenbindende Domäne eines Antikörpers umfassen und kovalent verknüpft sind.

2. Konjugat nach Anspruch 1, wobei das erste spezifische Bindemolekül:
(i) ferner eine Fe-Region eines Antikörpers umfasst; und/oder
(ii) bivalent ist; und/oder
(iii) ein monoklonaler Antikörper, gegebenenfalls vom IgG-Isotyp, ist; und/oder
(iv) ein F(ab')₂-Fragment eines monoklonalen Antikörpers ist; und/oder
(v) menschliches CD40 bindet und ein Agonist von menschlichem CD40 ist.

3. Konjugat nach Anspruch 2, wobei es sich bei dem ersten spezifischen Bindemolekül um ein antigenbindendes Protein handelt, das wenigstens eine variable Region der schweren Kette (VH) und wenigstens eine variable Region der leichten Kette (VL) umfasst, wobei:
(i)
(a) die VH-Region VHCDR 1, 2 und 3 umfasst, die die Aminosäuresequenzen unter SEQ ID NO: 17, 18 bzw. 19 umfassen, und
(b) die VL-Region VLCDR 1, 2 und 3 umfasst, die die Aminosäuresequenzen unter SEQ ID NO: 20, 21 bzw. 22 umfassen; oder
(ii)
(a) die VH-Region VHCDR 1, 2 und 3 umfasst, die die Aminosäuresequenzen unter SEQ ID NO: 31, 32 bzw. 33 umfassen; und
(b) die VL-Region VLCDR 1, 2 und 3 umfasst, die die Aminosäuresequenzen unter SEQ ID NO: 34, 35 bzw. 36 umfassen; oder
(iii)
(a) die VH-Region VHCDR 1, 2 und 3 umfasst, die die Aminosäuresequenzen unter SEQ ID NO: 58, 59 bzw. 60 umfassen; und
(b) die VL-Region VLCDR 1, 2 und 3 umfasst, die die Aminosäuresequenzen unter SEQ ID NO: 61, 62 bzw. 63 umfassen; oder
(iv)
(a) die VH-Region VHCDR 1, 2 und 3 umfasst, die die Aminosäuresequenzen unter SEQ ID NO: 64, 65 bzw. 66 umfassen, und
(b) die VL-Region VLCDR 1, 2 und 3 umfasst, die die Aminosäuresequenzen unter SEQ ID NO: 67, 68 bzw. 69 umfassen; oder
(v)
(a) die VH-Region VHCDR 1, 2 und 3 umfasst, die die Aminosäuresequenzen unter SEQ ID NO: 138, 139 bzw. 140 umfassen, und
(b) die VL-Region VLCDR 1, 2 und 3 umfasst, die die Aminosäuresequenzen unter SEQ ID NO: 141, 142 bzw. 143 umfassen; oder
(vi)
(a) die VH-Region VHCDR 1, 2 und 3 umfasst, die die Aminosäuresequenzen unter SEQ ID NO: 144, 145 bzw. 146 umfassen, und
(b) die VL-Region VLCDR 1, 2 und 3 umfasst, die die Aminosäuresequenzen unter SEQ ID NO: 147, 148 bzw. 149 umfassen.

4. Konjugat nach einem der Ansprüche 1 bis 3, wobei es sich bei dem zweiten spezifischen Bindemolekül um ein scFv handelt und/oder wobei das zweite spezifische Bindemolekül mit einer leichten oder schweren Kette des ersten spezifischen Bindemoleküls kovalent verknüpft ist.

5. Konjugat nach Anspruch 4, wobei das Konjugat ein erstes spezifisches Bindemolekül, bei dem es sich um einen Antikörper handelt, und zwei zweite spezifische Bindemoleküle, bei denen es sich jeweils um ein scFv handelt, umfasst und:
(i) ein scFv an die C_{H}3-Domäne jeder schweren Kette des Antikörpers konjugiert ist; oder
(ii) ein scFv an die C_{L-} Domäne jeder leichten Kette des Antikörpers konjugiert ist.

6. Konjugat nach einem der Ansprüche 1 bis 5, wobei die Tag-Gruppierung keine universalen menschlichen B-Zell-Epitope umfasst.

7. Konjugat nach einem der Ansprüche 1 bis 5, wobei die Tag-Gruppierung ein B-Zell-Epitop umfasst, wobei es sich bei dem B-Zell-Epitop gegebenenfalls um ein nichtmenschliches B-Zell-Epitop handelt.

8. Komplex, umfassend ein Konjugat gemäß einem der Ansprüche 1 bis 7 und ein Tag-Konstrukt, wobei das Tag-Konstrukt eine Tag-Gruppierung umfasst, die an ein Antigen kovalent gebunden ist, wobei die Tag-Gruppierung des Tag-Konstrukts an das zweite spezifische Bindemolekül nichtkovalent gebunden ist und die Tag-Gruppierung gemäß einem der Ansprüche 1, 6 und 7 vorliegt.

9. Komplex nach Anspruch 8, wobei das Antigen ein Krebsantigen ist oder von einem Krankheitserreger stammt, gegebenenfalls wobei es sich bei dem Krebsantigen um ein Neoantigen, ein tumorassoziiertes Antigen oder ein von einem Onkovirus stammendes Antigen handelt.

10. Pharmazeutische Zusammensetzung, umfassend: i) ein Konjugat gemäß einem der Ansprüche 1 bis 7 oder iii) einen Komplex gemäß einem der Ansprüche 8 bis 9 und wenigstens einen pharmazeutisch unbedenklichen Träger oder Exzipienten.

11. Kit, umfassend ein Konjugat gemäß einem der Ansprüche 1 bis 7 und ein Tag-Konstrukt, das Folgendes umfasst:
i) eine Peptid-Tag-Gruppierung, bei der es sich nicht um ein Krebsantigen handelt und die aus der Aminosäuresequenz unter einer von SEQ ID NO: 6 oder 10-14 oder einem Fragment von mindestens 5 Aminosäuren einer von SEQ ID NO: 6 oder 10-14 besteht, gegebenenfalls wobei die Tag-Gruppierung gemäß einem der Ansprüche 6 bis 7 vorliegt; und
ii) ein Antigen, bei dem es sich um ein Krebsantigen oder ein von einem Krankheitserreger stammendes Antigen handelt; wobei das Antigen ein Polypeptid ist und die Tag-Gruppierung mit dem Antigen kovalent verknüpft ist.

12. Komplex gemäß Anspruch 8 oder 9 oder Kit gemäß Anspruch 11 zur therapeutischen Verwendung.

13. Produkt, umfassend ein Konjugat gemäß einem der Ansprüche 1 bis 7 und ein Tag-Konstrukt als Kombinationspräparat zur gleichzeitigen oder nacheinander erfolgenden therapeutischen Verwendung, wobei das Tag-Konstrukt umfasst:
i) eine Peptid-Tag-Gruppierung, bei der es sich nicht um ein Krebsantigen handelt und die aus der Aminosäuresequenz unter einer von SEQ ID NO: 6 oder 10-14 oder einem Fragment von mindestens 5 Aminosäuren einer von SEQ ID NO: 6 oder 10-14 besteht, gegebenenfalls wobei die Tag-Gruppierung gemäß einem der Ansprüche 6 bis 7 vorliegt, und
ii) ein Antigen, bei dem es sich um ein Krebsantigen oder ein von einem Krankheitserreger stammendes Antigen handelt; wobei das Antigen ein Polypeptid ist und die Tag-Gruppierung mit dem Antigen kovalent verknüpft ist.

14. Komplex gemäß Anspruch 8 oder 9, Kit gemäß Anspruch 11 oder Produkt zur Verwendung gemäß Anspruch 13, zur Verwendung bei der Behandlung oder Vorbeugung von Krebs oder zur Verwendung bei der Behandlung oder Vorbeugung einer Infektionskrankheit.

15. *In-vitro-* oder *Ex*-*vivo*-Verfahren zur Aktivierung einer T-Zelle, die einen TCR exprimiert, der ein Antigen erkennt, wobei das Verfahren In-Kontakt-Bringen einer antigenpräsentierenden Zelle mit Folgendem umfasst:
(a) einem Konjugat gemäß einem der Ansprüche 1 bis 7 und ein Tag-Konstrukt, wobei das Tag-Konstrukt das von dem TCR erkannte Antigen umfasst und wobei das Tag-Konstrukt umfasst:
i) eine Peptid-Tag-Gruppierung, bei der es sich nicht um ein Krebsantigen handelt und die aus der Aminosäuresequenz unter einer von SEQ ID NO: 6 oder 10-14 oder einem Fragment von mindestens 5 Aminosäuren einer von SEQ ID NO: 6 oder 10-14 besteht, gegebenenfalls wobei die Tag-Gruppierung gemäß einem der Ansprüche 6 bis 7 vorliegt, und
ii) ein Antigen, bei dem es sich um ein Krebsantigen oder ein von einem Krankheitserreger stammendes Antigen handelt; wobei das Antigen ein Polypeptid ist und die Tag-Gruppierung mit dem Antigen kovalent verknüpft ist. ; oder
(b) einem Komplex gemäß Anspruch 8 oder 9 definiert, wobei das Tag-Konstrukt des Komplexes das von dem TCR erkannte Antigen umfasst.

## Revendications

1. Conjugué comprenant :
i) au moins une première molécule de liaison spécifique qui se lie à CD40, dans lequel ladite première molécule de liaison spécifique est un agoniste de CD40, et dans lequel ladite première molécule de liaison spécifique induit l'internalisation de CD40 lors de la liaison à CD40 ; et
ii) au moins une deuxième molécule de liaison spécifique qui se lie à un fragment marqueur, dans lequel ledit fragment marqueur est un peptide et non un antigène de cancer, dans lequel ledit peptide est constitué de la séquence d'acides aminés indiquée dans l'une quelconque des SEQ ID NO: 6 ou 10-14, ou un fragment de l'une quelconque des SEQ ID NO: 6 ou 10-14 d'au moins 5 acides aminés ;
dans lequel ladite première molécule de liaison spécifique et ladite deuxième molécule de liaison spécifique sont des protéines de liaison à un antigène comprenant un domaine de liaison à un antigène d'un anticorps et sont liées de manière covalente.

2. Conjugué selon la revendication 1, dans lequel ladite première molécule de liaison spécifique :
(i) comprend en outre une région Fc d'un anticorps ; et/ou
(ii) est bivalente ; et/ou
(iii) est un anticorps monoclonal, éventuellement de l'isotype IgG ; et/ou
(iv) est un fragment F(ab')₂ d'un anticorps monoclonal ; et/ou
(v) se lie à CD40 humaine et est un agoniste de CD40 humaine.

3. Conjugué selon la revendication 2, dans lequel ladite première molécule de liaison spécifique est une protéine de liaison à un antigène comprenant au moins une région variable de chaîne lourde (VH) et au moins une région variable de chaîne légère (VL), dans lequel :
(i)
(a) la région VH comprend les VHCDR 1, 2 et 3 qui comprennent les séquences d'acides aminés indiquées dans les SEQ ID NO: 17, 18 et 19 respectivement, et
(b) la région VL comprend des VLCDR 1, 2 et 3 qui comprennent les séquences d'acides aminés indiquées dans les SEQ ID NO: 20, 21 et 22, respectivement ; ou
(ii)
(a) la région VH comprend les VHCDR 1, 2 et 3 qui comprennent les séquences d'acides aminés indiquées dans les SEQ ID NO: 31, 32 et 33, respectivement ; et
(b) la région VL comprend des VLCDR 1, 2 et 3 qui comprennent les séquences d'acides aminés indiquées dans les SEQ ID NO: 34, 35 et 36, respectivement ; ou
(iii)
(a) la région VH comprend les VHCDR 1, 2 et 3 qui comprennent les séquences d'acides aminés indiquées dans les SEQ ID NO: 58, 59 et 60, respectivement ; et
(b) la région VL comprend des VLCDR 1, 2 et 3 qui comprennent les séquences d'acides aminés indiquées dans les SEQ ID NO: 61, 62 et 63, respectivement ; ou
(iv)
(a) la région VH comprend les VHCDR 1, 2 et 3 qui comprennent les séquences d'acides aminés indiquées dans les SEQ ID NO: 64, 65 et 66, respectivement, et
(b) la région VL comprend des VLCDR 1, 2 et 3 qui comprennent les séquences d'acides aminés indiquées dans les SEQ ID NO: 67, 68 et 69, respectivement ; ou
(v)
(a) la région VH comprend les VHCDR 1, 2 et 3 qui comprennent les séquences d'acides aminés indiquées dans les SEQ ID NO: 138, 139 et 140, respectivement, et
(b) la région VL comprend des VLCDR 1, 2 et 3 qui comprennent les séquences d'acides aminés indiquées dans les SEQ ID NO: 141, 142 et 143, respectivement ; ou
(vi)
(a) la région VH comprend les VHCDR 1, 2 et 3 qui comprennent les séquences d'acides aminés indiquées dans les SEQ ID NO: 144, 145 et 146, respectivement, et
(b) la région VL comprend des VLCDR 1, 2 et 3 qui comprennent les séquences d'acides aminés indiquées dans les SEQ ID NO: 147, 148 et 149, respectivement.

4. Conjugué selon l'une quelconque des revendications 1 à 3, dans lequel ladite deuxième molécule de liaison spécifique est un scFv, et/ou dans lequel ladite deuxième molécule de liaison spécifique est liée de manière covalente à une chaîne légère ou lourde de ladite première molécule de liaison spécifique.

5. Conjugué selon la revendication 4, dans lequel ledit conjugué comprend une première molécule de liaison spécifique, qui est un anticorps, et deux deuxièmes molécules de liaison spécifique, qui sont des scFv, et :
(i) un scFv est conjugué au domaine C_{H}3 de chaque chaîne lourde dudit anticorps ; ou
(ii) un scFv est conjugué au domaine C_{L} de chaque chaîne légère dudit anticorps.

6. Conjugué selon l'une quelconque des revendications 1 à 5, dans lequel ledit fragment marqueur ne comprend pas d'épitopes universels de cellules B humaines.

7. Conjugué selon l'une quelconque des revendications 1 à 5, dans lequel ledit fragment marqueur comprend un épitope de cellule B, dans lequel ledit épitope de cellule B est éventuellement un épitope de cellule B non humaines.

8. Complexe comprenant un conjugué tel que défini dans l'une quelconque des revendications 1 à 7 et une construction de marqueur, ladite construction de marqueur comprenant un fragment marqueur lié de manière covalente à un antigène, dans lequel le fragment marqueur de ladite construction de marqueur est lié de manière non covalente à la deuxième molécule de liaison spécifique et ledit fragment marqueur est tel que défini dans l'une quelconque des revendications 1, 6 et 7.

9. Complexe selon la revendication 8, dans lequel ledit antigène est un antigène de cancer ou dérivé d'un pathogène, éventuellement dans lequel ledit antigène de cancer est un néoantigène, un antigène associé à une tumeur ou un antigène dérivé d'un oncovirus.

10. Composition pharmaceutique comprenant :
i) un conjugué tel que défini dans l'une quelconque des revendications 1 à 7 ; ou
iii) un complexe tel que défini dans l'une quelconque des revendications 8 à 9,
et au moins un support ou excipient pharmaceutiquement acceptable.

11. Kit comprenant un conjugué tel que défini dans l'une quelconque des revendications 1 à 7 et une construction de marqueur comprenant :
i) un fragment marqueur peptidique qui n'est pas un antigène de cancer et qui est constitué de la séquence d'acides aminés indiquée dans l'une quelconque des SEQ ID NO: 6 ou 10-14, ou un fragment de l'une quelconque des SEQ ID NO: 6 ou 10-14 d'au moins 5 acides aminés, éventuellement dans lequel ledit fragment marqueur est tel que défini dans l'une quelconque des revendications 6 à 7 ; et
ii) un antigène, qui est un antigène de cancer ou un antigène dérivé d'un pathogène ; dans lequel ledit antigène est un polypeptide et ledit fragment marqueur est lié de manière covalente audit antigène.

12. Complexe tel que défini dans la revendication 8 ou 9 ou kit tel que défini dans la revendication 11 pour une utilisation en thérapie.

13. Produit comprenant un conjugué tel que défini dans l'une quelconque des revendications 1 à 7 et une construction de marqueur sous forme d'une préparation combinée pour une utilisation simultanée ou séquentielle en thérapie, dans lequel la construction de marqueur comprend
i) un fragment marqueur peptidique qui n'est pas un antigène de cancer et qui est constitué de la séquence d'acides aminés indiquée dans l'une quelconque des SEQ ID NO: 6 ou 10-14, ou un fragment de l'une quelconque des SEQ ID NO: 6 ou 10-14 d'au moins 5 acides aminés, éventuellement dans lequel ledit fragment marqueur est tel que défini dans l'une quelconque des revendications 6 à 7 ; et
ii) un antigène, qui est un antigène de cancer ou un antigène dérivé d'un pathogène ; dans lequel ledit antigène est un polypeptide et ledit fragment marqueur est lié de manière covalente audit antigène.

14. Complexe tel que défini dans la revendication 8 ou 9, kit tel que défini dans la revendication 11 ou produit pour une utilisation tel que défini dans la revendication 13, pour une utilisation dans le traitement ou la prévention d'un cancer, ou pour une utilisation dans le traitement ou la prévention d'une maladie infectieuse.

15. Procédé *in vitro* ou ex *vivo* d'activation d'une cellule T exprimant un TCR qui reconnaît un antigène, ledit procédé comprenant la mise en contact d'une cellule présentant un antigène avec :
(a) un conjugué tel que défini dans l'une quelconque des revendications 1 à 7 et une construction de marqueur, dans lequel ladite construction de marqueur comprend l'antigène reconnu par ledit TCR et dans lequel la construction de marqueur comprend
i) un fragment marqueur peptidique qui n'est pas un antigène de cancer et qui est constitué de la séquence d'acides aminés indiquée dans l'une quelconque des SEQ ID NO: 6 ou 10-14, ou un fragment de l'une quelconque des SEQ ID NO: 6 ou 10-14 d'au moins 5 acides aminés, éventuellement dans lequel ledit fragment marqueur est tel que défini dans l'une quelconque des revendications 6 à 7 ; et
ii) un antigène, qui est un antigène de cancer ou un antigène dérivé d'un pathogène ; dans lequel ledit antigène est un polypeptide et ledit fragment marqueur est lié de manière covalente audit antigène.
; ou
(b) un complexe tel que défini dans la revendication 8 ou 9, dans lequel la construction de marqueur dudit complexe comprend l'antigène reconnu par ledit TCR.
